# EUROPEAN PATENT APPLICATION

(11) **EP 3 772 542 A1**
(43) Date of publication of application: **10.02.2021**
(21) Application number: 19190509.0
(22) Date of filing: 07.08.2019
(51) Int. Cl.: C12N 15/82

(54) **MODIFYING GENETIC VARIATION IN CROPS BY MODULATING THE PACHYTENE CHECKPOINT PROTEIN 2**

(71) Applicant: KWS SAAT SE & Co. KGaA, 37574 Einbeck (DE); University of Leicester, Leicester LE1 7RH (GB)
(72) Inventor: Ogle, Daisy, Leicester, LE1 7RH (GB); Higgins, James, Leicester, LE1 7RH (GB); Kettles, Nicola, Thriplow, SG8 7RE (GB); Byrne, Edward, Thriplow, SG8 7RE (GB); Mittmann, Sybille, Thriplow, SG8 7RE (GB)

(57) **Abstract**

The present invention relates to method for altering genetic variability in plants or plant populations, comprising reducing the expression, activity, and/or stability of Pachytene checkpoint protein 2 homolog (Pch2), thereby altering linkage drag or linkage sweep, altering crossover frequency, distribution, and/or interference, generating a hyper-Rec phenotype, or altering recombination frequency, such as altered homologous and/or homoeologous recombination. The invention further relates to plants produced as such.

## Description

### FIELD OF THE INVENTION

The invention relates to modulation of genetic variation in crops, preferably cereals, in particular by altering linkage drag or selective sweep. The invention further relates to plants, in particular cereals such as wheat or barley exhibiting modified linkage drag or selective sweep.

### BACKGROUND OF THE INVENTION

Meiotic chromosomes are organized into linear looped chromatin arrays by a protein axis localized along the loop-bases. Programmed remodelling of the axis occurs during prophase I of meiosis. In meiosis, homologous recombination entails programmed DNA double-strand break (DSB) formation and synaptonemal complex (SC) assembly coupled with the DSB repair. Although SCs display extensive structural conservation among species, their components identified are poorly conserved at the sequence level.

Pachytene checkpoint protein 2 (Pch2), which is an AAA-ATPase, was first identified as a pachytene checkpoint factor in budding yeast (San-Segundo and Roeder, 1999). Subsequently, the essential role of Pch2 in pachytene checkpoint control was also uncovered in C. elegans and Drosophila (Bhalla and Dernburg, 2005; Joyce and McKim, 2009). In mice, the Pch2 ortholog THYROID RECEPTOR-INTERACTING PROTEIN13 (TRIP13) promotes the normal localization of RAD51 on meiotic chromosomes. In addition, the establishment of normal crossover distribution also requires Pch2/TRIP13 in budding yeast and mice (Roig et al., 2010). Furthermore, the establishment of normal crossover distribution also requires Pch2/TRIP13 in budding yeast and mice (Zanders and Alani, 2009; Roig et al., 2010). The downregulation of PCH2 ortholog in rice with RNAi has been reported. It revealed that the regulation is essential for meiotic DSB formation and crossovers/chiasmata did not form (Miao et al. 2013).

Orthologs of PCH2 have been identified in a variety of organisms. In mouse, analysis of a weak hypomorphic allele of TRIP13 (PCH2) indicated that the protein was required for the efficient repair of DSBs that enter the noncrossover (NCO) pathway but not crossover (CO) designated intermediates, which were processed normally. Despite the presence of unrepaired DSBs synapsis was normal in these mice (Li, X and Schimenti, J. C. 2007). Similar to Pch2 in budding yeast, TRIP13 is required for the depletion of the Hop1 orthologs HORMAD1 and HORMAD2 along synapsed regions of the chromosome axes (Wojtasz L. et al. 2009). In Caenorhabditis elegans it is reported to maintain the fidelity of recombination and synapsis during prophase I by acting to constrain these processes (Deshong A. J. et al. 2014).

Structured illumination microscopy (SIM) has revealed dynamic changes in the chromosome axis in Arabidopsis thaliana and Brassica oleracea. It has showed that the axis associated protein ASY1 is depleted during zygotene concomitant with synaptonemal complex (SC) formation. Study of an Atpch2 mutant demonstrates this requires the AAA+ ATPase, PCH2, which localizes to the sites of axis remodeling. Loss of PCH2 leads to a failure to deplete ASY1 from the axes and compromises SC polymerization. In another study, pch2 Knockout T-DNA insertion lines was obtained. A reduced CO frequency was observed, along with the presence of univalent, at a frequency of 10%, which caused the mis-segregation of chromosomes at the first meiotic division, resulting in unbalanced tetrads. However, Arabidopsis does not have low recombining regions of the genome (except centromeres), so a shift of crossovers from high to low recombining regions could not be assessed (Lambing et al. 2015).

Many crops such as barley and wheat show a skewed distribution of crossovers towards the end of the chromosome arms. This results in large chromosomal regions where little or no crossovers form, which is estimated to be about 48% of the genome in barley. These recombinationally 'cold' regions still contain about 30% of the total amount of genes, which are inaccessible by traditional breeding methods.

Further, the amount and location of crossovers can also detrimentally affect maintenance of desirable traits or phenotypes in traditional breeding programs.

It is an objective of the present invention to address one or more of the shortcomings of the prior art.

### SUMMARY OF THE INVENTION

The present invention relates to methods for modifying genetic variation, linkage drag, selective sweep, crossover distribution, crossover location, and/or crossover interference in crops by modulating the Pachytene checkpoint protein 2 (PCH2), in particular in cereals, such as barley and wheat. The present invention further relates to methods by using PCH2 to crossover sites in cereals. Further provided are methods for shifting of crossovers to area of low-/no recombination, in particular in barley, highly desirable in a breeding context, of which not many genes have shown to do this in a crop. There are further provided methods for shifting of crossovers to very distal positions, in particular in wheat, with a potential application in maintaining desirable genotypes.

The present inventors have surprisingly found that the barley mutant gene pachytene checkpoint protein 2 homolog (pch2) shifts recombination into the 'cold' low-recombining areas, making it a valuable breeders' tool to break linkage drag and create new allelic combinations that were previously unattainable.

Interestingly, the pch2 mutant in wheat has a different and opposing effect and shifts crossovers to extreme distal sites, which is useful for maintaining favourable genotypes, something that many breeders are very interested in, as no crossovers results in infertile plants.

The present invention is in particular captured by any one or any combination of one or more of the below numbered statements 1 to 43, with any other statement and/or embodiments.
1. A method for altering linkage drag or selective sweep in a plant, plant part, or plant population, comprising reducing or eliminating the expression, stability, and/or activity of Pachytene checkpoint protein 2 homolog (PCH2) (mRNA and/or protein) in a plant, plant part, or plant population.
2. The method according to statement 1, wherein crossover frequency, crossover interference and/or crossover distribution is altered or thereby altering crossover frequency, crossover interference and/or crossover distribution.
3. A method for altering crossover frequency, crossover interference, and/or crossover distribution in a plant, plant part, or plant population, comprising reducing or eliminating the expression, stability, and/or activity of PCH2 (mRNA and/or protein) in a plant, plant part, or plant population.
4. The method according to statement 3, wherein linkage drag or selective sweep is altered or thereby altering linkage drag or selective sweep.
5. A method for altering genetic variation in the genome of a plant, plant part, or plant population, comprising reducing or eliminating the expression, stability, and/or activity of Pachytene checkpoint protein 2 homolog (PCH2) (mRNA and/or protein) in a plant, plant part, or plant population.
6. The method according to statement 5, wherein crossover frequency, crossover interference and/or crossover distribution is altered or thereby altering crossover frequency, crossover interference and/or crossover distribution.
7. The method according to statement 5 or 6, wherein linkage drag or selective sweep is altered or thereby altering linkage drag or selective sweep.
8. A method for altering (the frequency of) (meiotic) interchromosomal recombination, preferably homeologous recombination, in a plant, plant part, or plant population, comprising reducing or eliminating the expression, stability, and/or activity of Pachytene checkpoint protein 2 homolog (PCH2) (mRNA and/or protein) in a plant, plant part, or plant population.
9. The method according to statement 8, wherein (the frequency of) interchromosomal recombination, preferably homeologous recombination, is increased.
10. A method for generating a plant, plant part, or plant population having a hyper-rec phenotype, comprising reducing or eliminating the expression, stability, and/or activity of Pachytene checkpoint protein 2 homolog (PCH2) (mRNA and/or protein) in a plant, plant part, or plant population.
11. The method according to any of statements 1 to 10, wherein said plant is from the family of Poaceae.
12. The method according to statement 11, wherein said plant is from the subfamily of Pooideae, preferably the tribe Triticeae, preferably the genera Hordeum or Triticum, preferably Hordeum vulgare or Triticum aestivum.
13. A method for generating a plant of the subfamily of pooideae, a plant part, or a plant population, comprising reducing or eliminating expression, stability, and/or activity of PCH2 (mRNA and/or protein), optionally wherein said plant, plant part, or plant population has a hyper-Rec phenotype.
14. The method according to any of statements 1 to 13, wherein linkage drag or selective sweep is reduced.
15. The method according to any of statements 1 to 14, wherein crossover frequency is increased.
16. The method according to any of statements 1 to 15, wherein crossover distribution is skewed towards the centromere.
17. The method according to any of statements 1 to 16, wherein the plant, plant part, or plant population having reduced or eliminated expression, stability, and/or activity of PCH2 exhibits more crossovers towards the centromere compared to a plant, plant part, or plant population not having reduced or eliminated expression, stability, and/or activity of PCH2.
18. The method according to any of statements 1 to 17, wherein crossover interference is reduced.
19. The method according to any of statements 1 to 18, wherein said plant, plant part, or plant population is from the genus Hordeum, preferably from the species Hordeum vulgare.
20. The method according to any of statements 1 to 13, wherein linkage drag or selective sweep is increased or maintained.
21. The method according to any of statements 1 to 13 or statement 20, wherein crossover frequency is decreased.
22. The method according to any of statements 1 to 13 or statement 20 to 21, wherein crossover distribution is skewed towards the telomers.
23. The method according to any of statements 1 to 13 or statements 20 to 22, wherein the plant, plant part, or plant population having reduced or eliminated expression, stability, and/or activity of PCH2 exhibits more crossovers towards the telomere compared to a plant, plant part, or plant population not having reduced or eliminated expression, stability, and/or activity of PCH2.
24. The method according to any of statements 1 to 13 or statements 20 to 23, wherein crossover interference is increased or maintained.
25. The method according to any of statements 1 to 13 or statements 20 to 24, wherein said plant, plant part, or plant population is from the genus Triticum, preferably from the species Triticum aestivum.
26. The method according to any of statements 1 to 25, wherein said reducing or eliminating comprises knocking out said Pch2 gene or knocking down said PCH2 protein.
27. The method according to any of statements 1 to 26, wherein reducing or eliminating comprises mutagenesis, RNAi, or gene editing.
28. The method according to any of statements 1 to 27, wherein the method comprises introducing or introgressing into the genome of a plant or plant part a nucleotide sequence of a gene encoding PCH2, having a mutation, preferably a mutation leading to reduced or eliminated expression of the mRNA of the gene and/or the PCH2 protein, a mutation leading to a PCH2 protein having reduced or eliminated activity upon translation, or a mutation leading to a PCH2 protein having reduced stability.
29. The method according to any of statements 1 to 28, wherein the method comprises
   (a) introducing or introgressing into a plant or plant part into a nucleotide sequence of a (endogenous (wild type)) gene encoding the PCH2 a mutation, preferably a mutation leading to reduced or eliminated expression of the (endogenous (full length)) mRNA of the gene and/or the (endogenous (full length)) PCH2 protein, a mutation leading to an PCH2 protein having reduced activity upon translation, or a mutation leading to an PCH2 protein having reduced stability;
   (b) introducing or introgressing into a plant or a plant part an RNAi molecule directed against, targeting, or hybridizing with a nucleotide sequence encoding the PCH2 protein, or a polynucleotide sequence encoding an RNAi molecule directed against, targeting, or hybridizing with a nucleotide sequence encoding the PCH2 protein, or
   (c) introducing or introgressing into a plant or a plant part an RNA-specific or DNA-specific CRISPR/Cas system directed against or targeting a nucleotide sequence encoding the PCH2 protein, or one or more polynucleotide sequence(s) encoding said RNA-specific CRISPR/Cas system, or
   (d) introducing or introgressing into a plant or a plant part a chemical compound or an antibody altering the activity of the PCH2 protein upon interaction with said PCH2.
   (e) optionally, regenerating a plant from the plant part of any of (a) to (d).
30. A plant, plant part, or plant population of the subfamily of pooideae having reduced or eliminated expression, stability, and/or activity of PCH2.
31. A plant, plant part, or plant population obtainable by the method according to any one of statement 13 to 30, or the progeny thereof having reducing or eliminated expression and/or activity of PCH2.
32. The plant, plant part, or plant population according to statement 30 or 31, which is selected from the genera Triticum or Hordeum, preferably Triticum aestivum or Hordeum vulgare.
33. The plant, plant part, or plant population according to any of statements 30 to 32, which is mutagenized.
34. The plant, plant part, or plant population according to any of statements 30 to 33, which is transgenic or gene-edited.
35. A plant part derived from the plant according to any of statements 30 to 34, having reduced or eliminated expression and/or activity of PCH2.
36. The plant part according to statement 35, which is a cell, tissue, or seed.
37. The method, plant, plant part, or plant population according to any of the preceding statements, wherein the wild type Pch2 gene encoding said PCH2 is selected from the group consisting of:
   (i) a nucleotide sequence comprising the sequence of SEQ ID NO: 31, 34, 37 or 40;
   (ii) a nucleotide sequence having the cDNA of SEQ ID NO: 32, 35, 38, or 41;
   (iii) a nucleotide sequence encoding for an amino acid sequence having the amino acid sequence of SEQ ID NO: 33, 36, 39, or 42;
   (iv) a nucleotide sequence having at least 60% identity to one of the sequences selected form the group consisting of SEQ ID NO: 31, 34, 37 or 40 or having a cDNA at least 60% identical to one of the sequences selected from the group consisting of SEQ ID NO: 32, 35, 38, or 41;
   (v) a nucleotide sequence encoding for a polypeptide having at least 60% identity to one of the sequences selected from the group consisting of SEQ ID NO: 33, 36, 39, or 42;
   (vi) a nucleotide sequence hybridizing with the reverse complement of a nucleotide sequence as defined in (i) to (v) under stringent hybridization conditions; and
   (vii) a nucleotide sequence encoding a protein derived from the amino acid sequence encoded by the nucleotide sequence of (i) to (vi) by way of substitution, deletion and/or addition of one or more amino acid(s).
38. Use of a plant, plant part, or plant population according to any of statements 30 to 37 for modifying genetic variation in a plant population, such as increasing genetic variation (for instance in barley) or decreasing genetic variation.
39. Use of a plant, plant part, or plant population according to any of statements 30 to 37 for modifying genetic variation in the genome of a plant (population), such as increasing genetic variation (for instance in barley) or decreasing genetic variation.
40. Use of a plant, plant part, or plant population according to any of statements 30 to 37 for modifying genetic variation (in the gene pool) of a plant (population), such as increasing genetic variation (for instance in barley) or decreasing genetic variation.
41. Use of a plant, plant part, or plant population according to any of statements 30 to 37 for modifying genetic fixation in a plant population, such as increasing genetic fixation (for instance in wheat) or decreasing genetic fixation.
42. Use of a plant, plant part, or plant population according to any of statements 30 to 37 for modifying genetic fixation in the genome of a plant (population), such as increasing genetic fixation (for instance in wheat) or decreasing genetic fixation.
43. Use of a plant, plant part, or plant population according to any of statements 30 to 37 for modifying genetic fixation (in the gene pool) of a plant (population), such as increasing genetic fixation (for instance in wheat) or decreasing genetic fixation.

### BRIEF DESCRIPTION OF THE FIGURES

**Figure 1****:** Typical WT Golden Promise metaphase I (MI) spreads, show ring-like structures, which typically have two chiasmata positioned at the ends of the chromosomes, which is visualised as a ring at metaphase I.
**Figure 2****:** Pch2 homozygous CRISPR/Cas9 mutant MI spreads (from CRISPR/Cas9 Line 1). One/two rods are present and the ring-like structures are different to WT, showing more interstitial chiasmata. An example of this is indicated by an arrow.

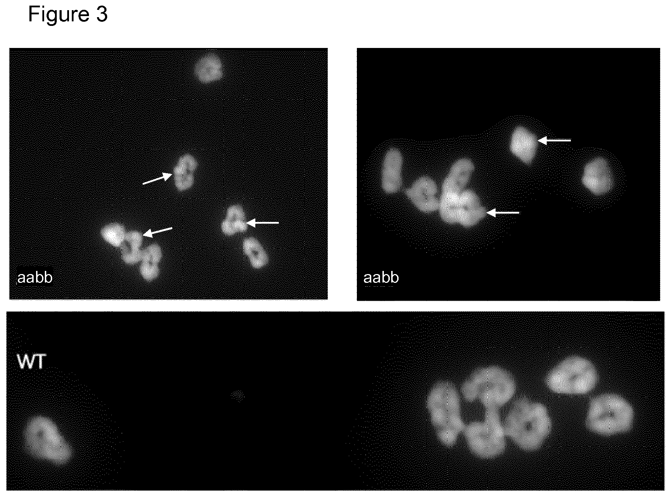

**Figure 3****:** aabb: pch2 homozygous CRISPR/Cas9 mutant MI spreads (from CRISPR/Cas9 Line 1). Ring-like structures are different to WT, showing more interstitial chiasmata. Examples of this are indicated by an arrow. WT: MI spread, showing ring-like structures with distal crossover positions.

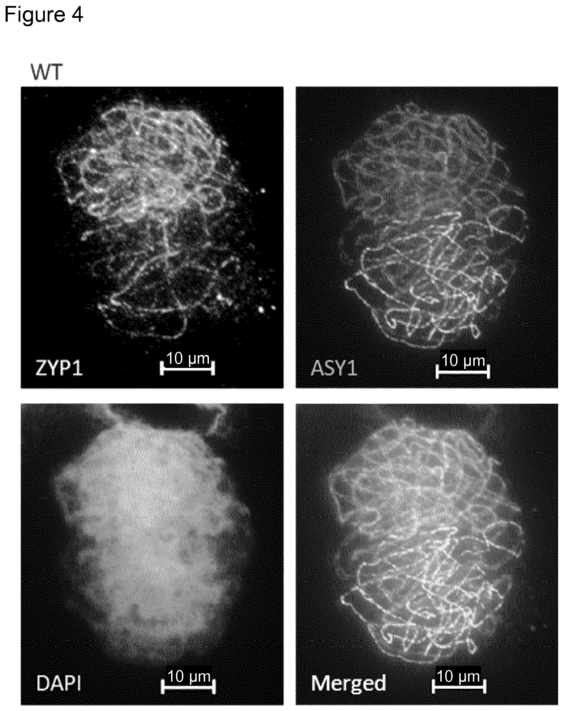

**Figure 4****:** Immunolocalisation of a meiotic nucleus, showing ASY1 and ZYP1 in a cell from the segregating WT Golden Promise CRISPR/Cas Line 1. Synapsis is normal, as indicated by ASY1 and ZYP1 proteins which show long linear signals. ASY1 is depleted from the axes by PCH2.

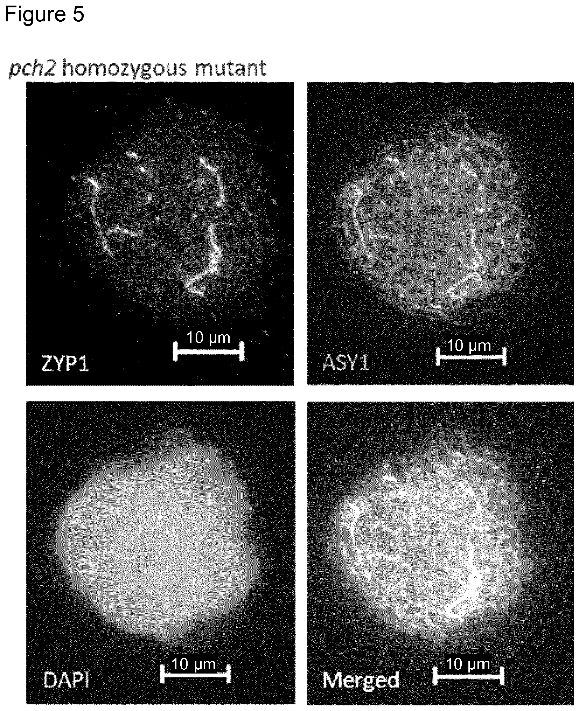

**Figure 5****:** Immunolocalisation of ASY1 and ZYP1 in a pch2 homozygous mutant, from the Golden Promise CRISPR/Cas Line 1. ASY1 is not depleted from the axes in the absence of PCH2 and ZYP1 is present in short stretches. These stretches are spread throughout the cell, indicating that chiasmata are not necessarily at a distal position.

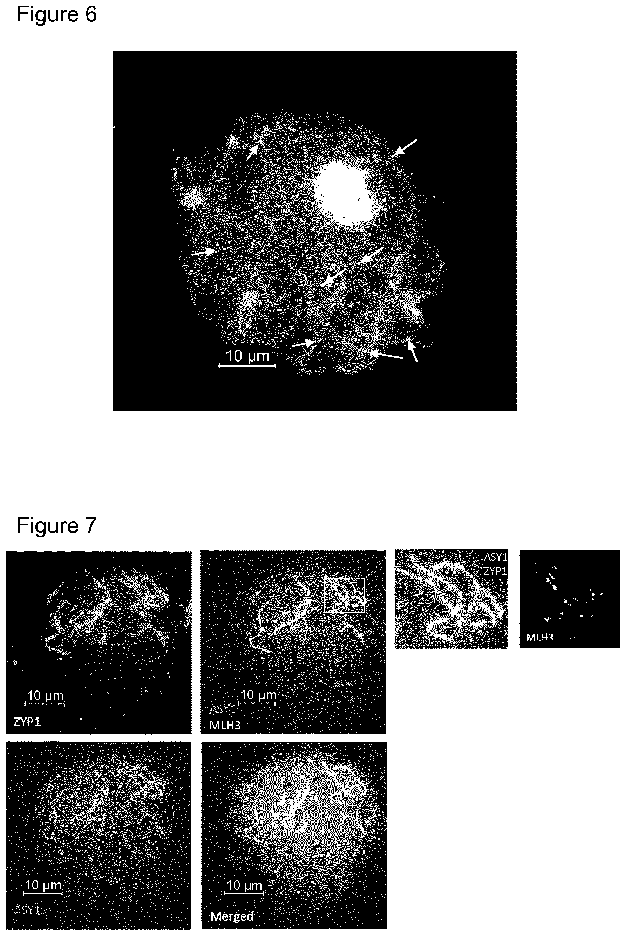

**Figure 6****:** Immunolocalisation of ZYP1 and MLH3 in WT barley. MLH3 is used in immunolocalisation studies as a marker of crossover sites. In WT barley, MLH3 foci are spread throughout the cell, indicating interference is present (hence the MLH3 are not nearby on the axes). MLH3 can be seen by the bright white foci (spots, exemplary indicated by arrows) on the image. ZYP1 can be seen in light grey as a synaptonemal complex marker.
**Figure 7****:** Immunolocalisation of ASY1, ZYP1 and MLH3 in a pch2 homozygous mutant, from the Golden Promise CRISPR/Cas Line 1. MLH3 is present along the short ZYP1 stretches, indicating crossovers occur on these short stretches. MLH3 foci are much closer together than expected (in WT these are spread out across the cell). Interference has been compromised in the absence of PCH2, meaning COs occur closer together and hence may become more interstitial.

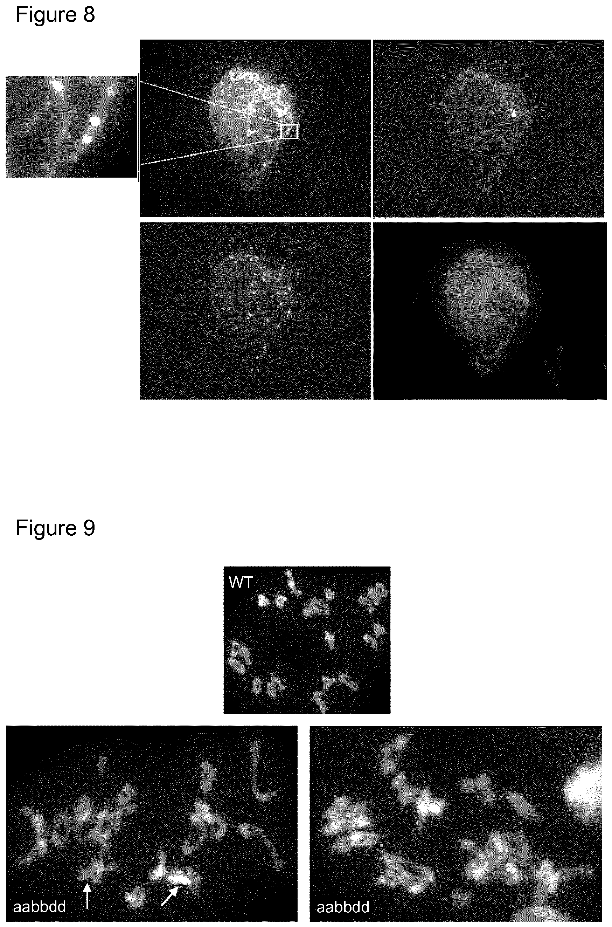

**Figure 8****:** Immunolocalisation of ZYP1 and HEI10 in pch2 CRISPR/Cas lines, HEI10 is present along the axes (marked by ZYP1). As seen with MLH3, HEI10 foci are much closer together than expected. Interference has been compromised in the absence of PCH2, meaning COs occur closer together and hence may become more interstitial. The discovery that HEI10 foci are appearing close together in the PCH2 CRISPR lines is highly important as it implicates a role for PCH2 regulating the interference mechanism.
**Figure 9****:** Cadenza TILLING line MI spreads, some apparently more interstitial COs are present in the pch2 mutant, indicated by arrows, compared to WT. There also appear to be some interchromosomal connections present, implicating a potential hyperrec phenotype.

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel et al. 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous.

In an aspect, the invention relates to a method for altering linkage drag or selective sweep in a plant, comprising reducing or eliminating the expression, stability, and/or activity of Pachytene checkpoint protein 2 homolog (PCH2) in a plant, preferably wherein interchromosomal recombination, preferably homologous or homeologous recombination or is altered or wherein crossover frequency, crossover interference and/or crossover distribution is altered.

In another aspect, the invention relates to a method for altering interchromosomal recombination, preferably homologous or homeologous recombination or for altering crossover frequency, crossover interference, and/or crossover distribution in a plant, comprising reducing or eliminating the expression, stability, and/or activity of PCH2 in a plant, preferably wherein linkage drag or selective sweep is altered.

In a further aspect, the invention relates to a method for generating a plant, preferably a plant having a hyper-Rec phonotype, preferably of the subfamily of pooideae, comprising reducing or eliminating expression, stability, and/or activity of PCH2.

In a further aspect, the invention relates to a plant or plant part thereof obtained by the methods of the invention as described herein, preferably of the subfamily of pooideae, such as a plant or plant part having reduced or eliminated expression, stability, and/or activity of PCH2, having altered linkage drag or linkage sweep, having altered crossover frequency, distribution, and/or interference, having a hyper-Rec phenotype, or having altered recombination frequency, such as altered homologous and/or homoeologous recombination, preferably wherein the plant or plant part is selected from the genera Triticum or Hordeum, preferably Triticum aestivum or Hordeum vulgare.

In further aspects, the invention relates to the use of such plants for modifying genetic variation in the genome of a plant (population), such as increasing genetic variation (for instance in barley) or decreasing genetic variation, for modifying genetic variation (in the gene pool) of a plant (population), such as increasing genetic variation (for instance in barley) or decreasing genetic variation, for modifying genetic fixation in a plant population, such as increasing genetic fixation (for instance in wheat) or decreasing genetic fixation, for modifying genetic fixation in the genome of a plant (population), such as increasing genetic fixation (for instance in wheat) or decreasing genetic fixation, or for modifying genetic fixation (in the gene pool) of a plant (population), such as increasing genetic fixation (for instance in wheat) or decreasing genetic fixation.

In further aspects, the invention relates to methods for modifying genetic variation in the genome of a plant (population), such as increasing genetic variation (for instance in barley) or decreasing genetic variation, for modifying genetic variation (in the gene pool) of a plant (population), such as increasing genetic variation (for instance in barley) or decreasing genetic variation, for modifying genetic fixation in a plant population, such as increasing genetic fixation (for instance in wheat) or decreasing genetic fixation, for modifying genetic fixation in the genome of a plant (population), such as increasing genetic fixation (for instance in wheat) or decreasing genetic fixation, or for modifying genetic fixation (in the gene pool) of a plant (population), such as increasing genetic fixation (for instance in wheat) or decreasing genetic fixation, comprising reducing or eliminating the expression, stability, and/or activity of Pachytene checkpoint protein 2 homolog (PCH2) in a plant (population).

The term "plant" includes whole plants, including descendants or progeny thereof. The term "plant part" includes any part or derivative of the plant, including particular plant tissues or structures, plant cells, plant protoplast, plant cell or tissue culture from which plants can be regenerated, plant calli, plant clumps and plant cells that are intact in plants or parts of plants, such as seeds, kernels, cobs, flowers, cotyledons, leaves, stems, buds, roots, root tips, stover, and the like. Plant parts may include processed plant parts or derivatives, including flower, oils, extracts etc. In certain embodiments, the plant part or derivative as referred to herein is a cell, tissue, or seed (or grain). In certain embodiments, the plant part or derivative as referred to herein is a seed (or grain).

In certain embodiments, the plant part or derivative is not (functional) propagation material, such as germplasm, a seed, or plant embryo or other material from which a plant can be regenerated. In certain embodiments, the plant part or derivative does not comprise (functional) male and female reproductive organs. In certain embodiments, the plant part or derivative is or comprises propagation material, but propagation material which does not or cannot be used (anymore) to produce or generate new plants, such as propagation material which have been chemically, mechanically or otherwise rendered non-functional, for instance by heat treatment, acid treatment, compaction, crushing, chopping, etc.

As used herein, the term plant population may be used interchangeably with population of plants. A plant population preferably comprises a multitude of individual plants, such as preferably at least 10, such as 20, 30, 40, 50, 60, 70, 80, or 90, more preferably at least 100, such as 200, 300, 400, 500, 600, 700, 800, or 900, even more preferably at least 1000, such as at least 10000 or at least 100000.

As used herein, the term Poaceae refers to the family of grasses, or Gramineae. Preferably, the Poaceae are cereals (or cereal grasses), which are in particular cultivated for the edible components of its grain.

As used herein, the term Pooideae refers to the subfamily of Poaceae in the Poaceae family. Preferably, the Pooideae are cereals (or cereal grasses), which are in particular cultivated for the edible components of its grain.

As used herein, the term Triticeae refers to the tribe of Triticeae in the ppoideae subfamily. Preferably, the Triticeae are cereals (or cereal grasses), which are in particular cultivated for the edible components of its grain. Non limiting genera in the tribe Triticaceae include Aegilops, Agropyron, Amblyopyrum, Anthosachne, Australopyrum, Cockaynea, Crithopsis, Dasypyrum, Elymus, Elytrigia, Eremium, Eremopyrum, Festucopsis, Haynaldia, Henrardia, Heteranthelium, Hordelymus, Hordeum, Hystrix, Kengyilia, Leymus, Lophopyrum, Malacurus, Pascopyrum, Peridictyon, Psathyrostachys, Pseudoroegneria, Secale, Sitanion, Stenostachys, Taeniatherum, Thinopyrum, , Triticum. Preferably, the Triticaceae genus is Triticum or Hordeum.

As used herein, the term Triticum refers to the genus Triticum in the Triticeae tribe. The term Triticum may be used herein interchangeably with wheat. Non limiting species in the genus Triticum include T. aestivum, T. aethiopicum, T. araraticum, T. boeoticum, T. carthlicum, T. compactum, T. dicoccoides, T. dicoccon, T. durum, T. ispahanicum, T. karamyschevii, T. macha, T. militinae, T. monococcum, T. polonicum, T. spelta, T. sphaerococcum, T. timopheevii, T. turanicum, T. turgidum, T. urartu, T. vavilovii, T. zhukovskyi; or any subspecies or hybrid thereof, including all ploidy levels, such as (allo)tetraploid and (allo)hexaploid. Preferably, the Triticum species is Triticum aestivum.

As used herein, the term Hordeum refers to the genus Hordeum in the Triticeae tribe. The term Hordeum may be used herein interchangeably with barley. Non limiting species in the genus Hordeum include H. aegiceras, H. arizonicum, H. bogdanii, H. brachyantherum, H. brachyatherum, H. brevisubulatum, H. bulbosum, H. californicum, H. capense H. chilense, H. comosum, H. cordobense, H. depressum, H. distichon, H. erectifolium, H. euclaston, H. flexuosum, H. fuegianum, H. guatemalense, H. halophilum, H. intercedens, H. jubatum, H. × lagunculciforme, H. lechleri, H. marinum, H. murinum, H. muticum, H. parodii, H. patagonicum, H. × pavisii, H. procerum, H. pubiflorum, H. pusillum, H. roshevitzii, H. secalinum, H. spontaneum, H. stenostachys, H. tetraploidum, H. vulgare; or any subspecies or hybrid thereof, including all ploidy levels, such as diploid.Preferably, the Hordeum species is Hordeum vulgaris.

As used herein, the terms "crossover", "chromosomal crossover", "crossing over" and the likes are used as known in the art. As further guidance, and without limitation, these terms may refer to the exchange of genetic material between two homologous (or homeologous) chromosomes non-sister chromatids that results in recombinant chromosomes during sexual reproduction. It is one of the final phases of genetic recombination, which occurs in the pachytene stage of prophase I of meiosis during a process called synapsis. Synapsis begins before the synaptonemal complex develops and is not completed until near the end of prophase I. Crossover usually occurs when matching regions on matching chromosomes break and then reconnect to the other chromosome.

As used herein, the term "crossover frequency" is used as known in the art. As further guidance, and without limitation, this term may refer to the (average) amount of crossovers (during meiosis) in a given plant or plant population. Crossover frequency may be expressed per chromosome or even chromosomal fragment, or may be expressed for the entire set of chromosomes. In certain embodiments, crossover frequency may be used interchangeably with recombination frequency, such homologous recombination frequency or homeologous recombination frequency, or both. In certain embodiments, crossover frequency may be used interchangeably with chiasma frequency. This is however not preferred, as a chiasma merely indicates the possibility of a crossover happening, however the result may equally be a noncrossover.

An altered of modified crossover frequency means that the (average) crossover frequency has been changed, compared to the expected (average) crossover frequency, either between homologous chromosomes, homeologous chromosomes, or both. For instance crossover frequency in a mutant plant, such as described herein elsewhere, may be altered compared to a wild type plant. In certain embodiments, (average) crossover frequency (for the total of chromosomes, for a single chromosome, or for a given chromosomal fragment) is altered by at least 10%, such as at least 15%, at least 20%, at least 25%, at least 30%, at least 40%, or at least 50%. Preferably, (average) crossover frequency is altered by at least 25%, more preferably at least 50%. In certain embodiments, (average) crossover frequency (for the total of chromosomes, for a single chromosome, or for a given chromosomal fragment) is increased by at least 10%, such as at least 15%, at least 20%, at least 25%, at least 30%, at least 40%, or at least 50%. Preferably, (average) crossover frequency is increased by at least 25%, more preferably at least 50%. In certain embodiments, (average) crossover frequency (for the total of chromosomes, for a single chromosome, or for a given chromosomal fragment) is decreased by at least 10%, such as at least 15%, at least 20%, at least 25%, at least 30%, at least 40%, or at least 50%. Preferably, (average) crossover frequency is decreased by at least 25%, more preferably at least 50%.

As used herein, the term "crossover distribution" is used as known in the art. As further guidance, and without limitation, this term may refer to the (average) "spread" of crossover events. In many organisms, crossovers are not distributed randomly. So-called cold regions exhibit a (relatively) low amount of crossovers, whereas so-called hot regions exhibit a (relatively) high amount of crossovers. Crossover distribution may be determined per chromosome or even chromosomal fragment, or may be determined for the entire set of chromosomes.

An altered of modified crossover distribution means that the (average) crossover distribution has been changed, compared to the expected (average) crossover distribution. An alteration of crossover distribution may indicate a (partial) shift of crossover distribution from hot regions to cold regions, or vice versa. For instance crossover distribution in a mutant plant, such as described herein elsewhere, may be altered compared to a wild type plant. In certain embodiments, (average) crossover distribution (for the total of chromosomes, for a single chromosome, or for a given chromosomal fragment) is altered by at least 10%, such as at least 15%, at least 20%, at least 25%, at least 30%, at least 40%, or at least 50%. This means that for instance at least 10% of crossovers has been shifted. Preferably, (average) crossover distribution is altered by at least 25%, more preferably at least 50%. In certain embodiments, (average) crossover distribution (for the total of chromosomes, for a single chromosome, or for a given chromosomal fragment) is increased by at least 10%, such as at least 15%, at least 20%, at least 25%, at least 30%, at least 40%, or at least 50%. Preferably, (average) crossover distribution is increased by at least 25%, more preferably at least 50%. In certain embodiments, (average) crossover distribution (for the total of chromosomes, for a single chromosome, or for a given chromosomal fragment) is decreased by at least 10%, such as at least 15%, at least 20%, at least 25%, at least 30%, at least 40%, or at least 50%. Preferably, (average) crossover distribution is decreased by at least 25%, more preferably at least 50%.

In certain embodiments, (average) crossover distribution (for the total of chromosomes, for a single chromosome, or for a given chromosomal fragment) is increased by at least 10%, such as at least 15%, at least 20%, at least 25%, at least 30%, at least 40%, or at least 50% in cold regions. Preferably, (average) crossover distribution is altered by at least 25%, more preferably at least 50% in cold regions. In certain embodiments, (average) crossover distribution (for the total of chromosomes, for a single chromosome, or for a given chromosomal fragment) is increased by at least 10%, such as at least 15%, at least 20%, at least 25%, at least 30%, at least 40%, or at least 50% in cold regions. Preferably, (average) crossover distribution is increased by at least 25%, more preferably at least 50% in cold regions. In certain embodiments, (average) crossover distribution (for the total of chromosomes, for a single chromosome, or for a given chromosomal fragment) is decreased by at least 10%, such as at least 15%, at least 20%, at least 25%, at least 30%, at least 40%, or at least 50% in cold regions. Preferably, (average) crossover distribution is decreased by at least 25%, more preferably at least 50% in cold regions.

In certain embodiments, (average) crossover distribution (for the total of chromosomes, for a single chromosome, or for a given chromosomal fragment) is increased by at least 10%, such as at least 15%, at least 20%, at least 25%, at least 30%, at least 40%, or at least 50% in hot regions. Preferably, (average) crossover distribution is altered by at least 25%, more preferably at least 50% in hot regions. In certain embodiments, (average) crossover distribution (for the total of chromosomes, for a single chromosome, or for a given chromosomal fragment) is increased by at least 10%, such as at least 15%, at least 20%, at least 25%, at least 30%, at least 40%, or at least 50% in hot regions. Preferably, (average) crossover distribution is increased by at least 25%, more preferably at least 50% in hot regions. In certain embodiments, (average) crossover distribution (for the total of chromosomes, for a single chromosome, or for a given chromosomal fragment) is decreased by at least 10%, such as at least 15%, at least 20%, at least 25%, at least 30%, at least 40%, or at least 50% in hot regions. Preferably, (average) crossover distribution is decreased by at least 25%, more preferably at least 50% in hot regions.

In certain embodiments, crossover distribution is altered by an increase or decrease in the (average) amount of crossovers in the (peri)centromeric region. In certain embodiments, crossover distribution is altered by an increase or decrease in the (average) amount of crossovers in the (sub)telomeric region. In certain embodiments, crossover distribution is altered by an increase or decrease in the (average) amount of crossovers towards the centromer. In certain embodiments, crossover distribution is altered by an increase or decrease in the (average) amount of crossovers towards the telomer.

In certain embodiments, crossover distribution is altered by an increase in the (average) amount of crossovers in the (peri)centromeric region and a decrease in the (average) amount of crossovers in the (sub)telomeric region. In certain embodiments, crossover distribution is altered by decrease in the (average) amount of crossovers in the (peri)centromeric region and an increase in the (average) amount of crossovers in the (sub)telomeric region. In certain embodiments, crossover distribution is altered by an increase in the (average) amount of crossovers towards the centromer and a decrease in the (average) amount of crossovers towards the telomer. In certain embodiments, crossover distribution is altered by decrease in the (average) amount of crossovers towards the centromer and an increase in the (average) amount of crossovers towards the telomer.

In certain embodiments, crossover distribution is altered by an increase in the (average) amount of crossovers in the (peri)centromeric region and an increase in the (average) amount of crossovers in the (sub)telomeric region. In certain embodiments, crossover distribution is altered by decrease in the (average) amount of crossovers in the (peri)centromeric region and a decrease in the (average) amount of crossovers in the (sub)telomeric region. In certain embodiments, crossover distribution is altered by an increase in the (average) amount of crossovers towards the centromer and an increase in the (average) amount of crossovers towards the telomer. In certain embodiments, crossover distribution is altered by decrease in the (average) amount of crossovers towards the centromer and a decrease in the (average) amount of crossovers towards the telomer.

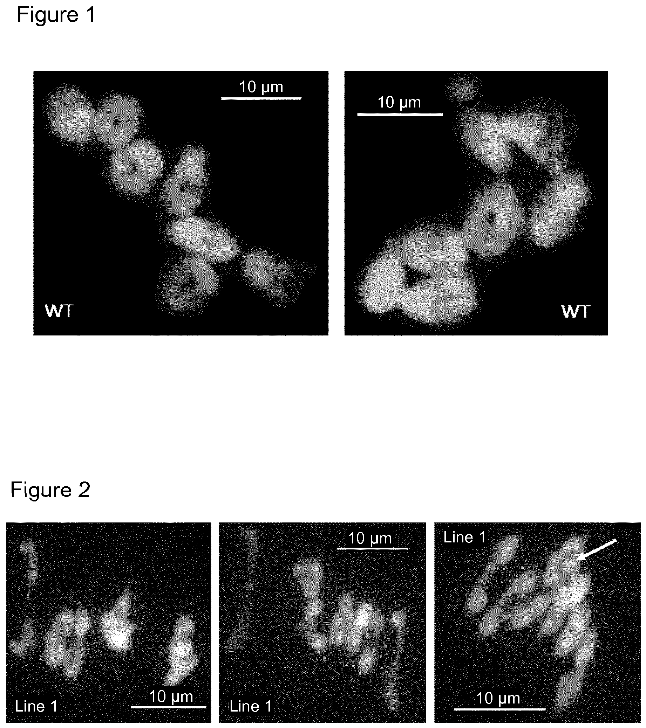

In certain embodiments, the (sub)telomeric region (preferably in barley) corresponds to zone 1 and the (peri)centromeric region corresponds to zone 3, as indicated in Figure 1 Mascher et al. (2017) Nature; 544:427-433 (doi:10.1038/nature22043). In certain embodiments, the (sub)telomeric region (preferably in wheat) corresponds to zone 1 and the (peri)centromeric region corresponds to zone 3, as indicated in Choulet, F. et al. (2014) Science 345:1249721 (doi: 10.1126/science.1249721).

In certain embodiments, the crossover distribution is skewed towards the telomere or (sub)telomeric region. In certain embodiments, the crossover distribution is skewed towards the centromere or (peri)centromeric region. As used herein, the term skewed may mean that a relative increase in the (average) amount of crossovers in the designated chromosomal region is observed, or that the overall (average) amount of crossovers in the designated chromosomal region is (relatively) increased or is (relatively) high.

As used herein, the term "crossover interference" is used as known in the art. As further guidance, and without limitation, this term may refer to the non-random placement of crossovers with respect to each other during meiosis. Meiotic crossovers (COs) appear to be regulated to ensure that COs on the same chromosome are distributed far apart (crossover interference). In other words, a crossover at a given position inhibits or results in inhibition of nearby crossovers (on the same chromosome). Crossover interference may be determined per chromosome or even chromosomal fragment, or may be determined for the entire set of chromosomes.

An altered of modified crossover interference means that the (average) crossover interference has been changed, compared to the expected (average) crossover interference. For instance crossover interference in a mutant plant, such as described herein elsewhere, may be altered compared to a wild type plant. In certain embodiments, (average) crossover interference (for the total of chromosomes, for a single chromosome, or for a given chromosomal fragment) is altered by at least 10%, such as at least 15%, at least 20%, at least 25%, at least 30%, at least 40%, or at least 50%. Preferably, (average) crossover interference is altered by at least 25%, more preferably at least 50%. In certain embodiments, (average) crossover interference (for the total of chromosomes, for a single chromosome, or for a given chromosomal fragment) is increased by at least 10%, such as at least 15%, at least 20%, at least 25%, at least 30%, at least 40%, or at least 50%. Preferably, (average) crossover interference is increased by at least 25%, more preferably at least 50%. In certain embodiments, (average) crossover interference (for the total of chromosomes, for a single chromosome, or for a given chromosomal fragment) is decreased by at least 10%, such as at least 15%, at least 20%, at least 25%, at least 30%, at least 40%, or at least 50%. Preferably, (average) crossover interference is decreased by at least 25%, more preferably at least 50%. In certain embodiments, an altered crossover interference (for the total of chromosomes, for a single chromosome, or for a given chromosomal fragment) refers to an altered (average) genetic distance between (neighbouring) chromosomal segments (for a given physical distance). In certain embodiments, the (average) genetic distance (for the total of chromosomes, for a single chromosome, or for a given chromosomal fragment) is altered by at least 10%, such as at least 15%, at least 20%, at least 25%, at least 30%, at least 40%, or at least 50%. Preferably, the (average) genetic distance is altered by at least 25%, more preferably at least 50%. In certain embodiments, (average) genetic distance (for the total of chromosomes, for a single chromosome, or for a given chromosomal fragment) is increased by at least 10%, such as at least 15%, at least 20%, at least 25%, at least 30%, at least 40%, or at least 50%. Preferably, (average) genetic distance is increased by at least 25%, more preferably at least 50%. In certain embodiments, (average) genetic distance interference (for the total of chromosomes, for a single chromosome, or for a given chromosomal fragment) is decreased by at least 10%, such as at least 15%, at least 20%, at least 25%, at least 30%, at least 40%, or at least 50%. Preferably, (average) genetic distance is decreased by at least 25%, more preferably at least 50%.

A used herein, a particular feature is maintained if such feature is not (substantially) changed, such as preferably by less than 10%, such as less than 5%. A used herein, crossover frequency, distribution, or interference is maintained if crossover frequency, distribution, or interference is not (substantially) changed, such as preferably by less than 10%, such as less than 5%. A used herein, linkage drag or selective sweep is maintained if linkage drag or selective sweep is not (substantially) changed, such as preferably by less than 10%, such as less than 5%.

As used herein, the term homologous recombination has its ordinary meaning in the art. By further guidance, and without limitation, this term refers to genetic recombination in which nucleotide sequences are exchanged between two similar or identical molecules of DNA. Homologous recombination produces new combinations of DNA sequences during meiosis. Homologous recombination may occur between homologous chromosomes (or homologs), which is the set of one (or more in case of (auto)polyploidy) paternal and one maternal (or more in case of (auto)polyploidy) chromosome. In contrast, homeologous recombination (or homoeologous recombination) may occur between homeologous chromosomes (or homeologs or homoeologs), which may result from inter-species hybridization and allopolyploidization with chromosomes derived from two or more diverged taxa.

As used herein, the term hyper-Rec (or hyper-rec or hyperrec) has its ordinary meaning in the art. By further guidance, and without limitation, this term refers to hyper-recombination, which is increased recombination, preferably increased homologous and/or homeologous recombination. Hyper-Rec phenotypes typically arise from mutations affecting recombination rates. A hyper-Rec phenotype refers to a plant or plant population exhibiting such hyper recombination. In certain embodiments, plants having a hyper-Rec phenotype exhibit an increase in (average) (homologous and/or homeologous) recombination of at least 10%, such as at least 15%, at least 20%, at least 25%, at least 30%, at least 40%, or at least 50%. Preferably, (average) recombination is increased by at least 25%, more preferably at least 50%.

As used herein, the term genetic variation is used as known in the art. As further guidance, and without limitation, genetic variability or genetic variation may refer to the presence or generation of genetic differences (between individuals or within a population) or the formation of individuals differing in genotype, or the presence of genotypically different individuals (in a population).

As used herein, the term gene pool is used as known in the art. As further guidance, and without limitation, gene pool may refer to the set of all genes, or genetic information or material, in a given population, in particular an interbreeding population, such as a taxon or (sub)species.

"Physical distance" between loci (e.g. between molecular markers and/or between phenotypic markers) on the same chromosome is the actually physical distance expressed in bases or base pairs (bp), kilo bases or kilo base pairs (kb) or megabases or mega base pairs (Mb).

"Genetic distance" between loci (e.g. between molecular markers and/or between phenotypic markers) on the same chromosome is measured by frequency of crossing-over, or recombination frequency (RF) and is indicated in centimorgans (cM). One cM corresponds to a recombination frequency of 1%. If no recombinants can be found, the RF is zero and the loci are either extremely close together physically or they are identical. The further apart two loci are, the higher the RF.

A "physical map" of the genome is a map showing the linear order of identifiable landmarks (including genes, markers, etc.) on chromosome DNA. However, in contrast to genetic maps, the distances between landmarks are absolute (for example, measured in base pairs or isolated and overlapping contiguous genetic fragments) and not based on genetic recombination (that can vary in different populations).

An allele "negatively" correlates with a trait when it is linked to it and when presence of the allele is an indicator that a desired trait or trait form will not occur in a plant comprising the allele. An allele "positively" correlates with a trait when it is linked to it and when presence of the allele is an indicator that the desired trait or trait form will occur in a plant comprising the allele.

A centimorgan ("cM") is a unit of measure of recombination frequency. One cM is equal to a 1 % chance that a marker at one genetic locus will be separated from a marker at a second locus due to crossing over in a single generation.

As used herein, the term "chromosomal interval" designates a contiguous linear span of genomic DNA that resides in planta on a single chromosome. The genetic elements or genes located on a single chromosomal interval are physically linked. The size of a chromosomal interval is not particularly limited. In some aspects, the genetic elements located within a single chromosomal interval are genetically linked, typically with a genetic recombination distance of, for example, less than or equal to 20 cM, or alternatively, less than or equal to 10 cM. That is, two genetic elements within a single chromosomal interval undergo recombination at a frequency of less than or equal to 20% or 10%.

The term "closely linked", in the present application, means that recombination between two linked loci occurs with a frequency of equal to or less than about 10% (i.e., are separated on a genetic map by not more than 10 cM). Put another way, the closely linked loci co-segregate at least 90% of the time. Marker loci are especially useful with respect to the subject matter of the current disclosure when they demonstrate a significant probability of co-segregation (linkage) with a desired trait (e.g., resistance to gray leaf spot). Closely linked loci such as a marker locus and a second locus can display an inter-locus recombination frequency of 10% or less, preferably about 9% or less, still more preferably about 8% or less, yet more preferably about 7% or less, still more preferably about 6% or less, yet more preferably about 5% or less, still more preferably about 4% or less, yet more preferably about 3% or less, and still more preferably about 2% or less. In highly preferred embodiments, the relevant loci display a recombination a frequency of about 1 % or less, e.g., about 0.75% or less, more preferably about 0.5% or less, or yet more preferably about 0.25% or less. Two loci that are localized to the same chromosome, and at such a distance that recombination between the two loci occurs at a frequency of less than 10% (e.g., about 9 %, 8%, 7%, 6%, 5%, 4%, 3%, 2%, 1 %, 0.75%, 0.5%, 0.25%, or less) are also said to be "proximal to" each other. In some cases, two different markers can have the same genetic map coordinates. In that case, the two markers are in such close proximity to each other that recombination occurs between them with such low frequency that it is undetectable.

"Linkage" refers to the tendency for alleles to segregate together more often than expected by chance if their transmission was independent. Typically, linkage refers to alleles on the same chromosome. Genetic recombination occurs with an assumed random frequency over the entire genome. Genetic maps are constructed by measuring the frequency of recombination between pairs of traits or markers. The closer the traits or markers are to each other on the chromosome, the lower the frequency of recombination, and the greater the degree of linkage. Traits or markers are considered herein to be linked if they generally co- segregate. A 1/100 probability of recombination per generation is defined as a genetic map distance of 1.0 centiMorgan (1.0 cM). The term "linkage disequilibrium" refers to a non-random segregation of genetic loci or traits (or both). In either case, linkage disequilibrium implies that the relevant loci are within sufficient physical proximity along a length of a chromosome so that they segregate together with greater than random (i.e., non-random) frequency. Markers that show linkage disequilibrium are considered linked. Linked loci co-segregate more than 50% of the time, e.g., from about 51 % to about 100% of the time. In other words, two markers that co-segregate have a recombination frequency of less than 50% (and by definition, are separated by less than 50 cM on the same linkage group.) As used herein, linkage can be between two markers, or alternatively between a marker and a locus affecting a phenotype. A marker locus can be "associated with" (linked to) a trait. The degree of linkage of a marker locus and a locus affecting a phenotypic trait is measured, e.g., as a statistical probability of co-segregation of that molecular marker with the phenotype (e.g., an F statistic or LOD score).

The genetic elements or genes located on a single chromosome segment are physically linked. In some embodiments, two loci are located in close proximity such that recombination between homologous chromosome pairs does not occur between the two loci during meiosis with high frequency, e.g., such that linked loci co-segregate at least about 90% of the time, e.g., 91%, 92%, 93%, 94%, 95%, 96%, 97%, 98%, 99%, 99.5%, 99.75%, or more of the time. The genetic elements located within a chromosomal segment are also "genetically linked", typically within a genetic recombination distance of less than or equal to 50cM, e.g., about 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0.75, 0.5, 0.25 cM or less. That is, two genetic elements within a single chromosomal segment undergo recombination during meiosis with each other at a frequency of less than or equal to about 50%, e.g., about 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.75%, 0.5%, 0.25% or less. "Closely linked" genetic elements display a cross over frequency the genetic elements of about 10% or less, e.g., 9%, 8%, 7%, 6%, 5%, 4%, 3%, 2%, 1%, 0.75%, 0.5%, 0.25% or less (a given genetic element is within about 10 cM of a closely linked genetic element, e.g., 9, 8, 7, 6, 5, 4, 3, 2, 1, 0.75, 0.5, 0.25 cM or less of a closely linked genetic element). Put another way, closely linked genetic elements co- segregate at least about 90% the time, e.g., 91%, 92%, 93%, 94%, 95%, 96%, 97%, 98%, 99%, 99.5%, 99.75%, or more of the time.

As used herein, the term "linkage drag" is used as known in the art. As further guidance, and without limitation, this term may refer to describe the phenotypical expression of (unwanted) donor genes which reside in the same genomic region as a desired gene (allele) or for instance a target QTL and thus are closely coupled with it. When a gene or allele is transfered from a donor parent, not just the gene is transferred. Rather upstream and downstream chromosomal regions are transferred as well, which is known as "linkage drag". This can be detrimental since undesirable DNA that can negatively affect crop performance may be linked to the target gene from the donor parent. As an example, this includes the observation that, by means of introgression of the chromosomal fragment which carries the desired gene (allele) or QTL, donor genes which have a negative effect are transferred into the introgression line, so that the introgression line then performs less well for specific agronomic traits than the original recipient line.

As used herein, the term "selective sweep" is used as known in the art. As further guidance, and without limitation, this term may refer to the reduction or elimination of variation among the nucleotides near a particular allele, such as a QTL or a particular mutation in DNA. It results from a beneficial allele having recently reached fixation due to strong positive natural selection. A selective sweep can occur when a rare or previously non-existing allele that increases the fitness of the carrier (relative to other members of the population) increases rapidly in frequency due to natural selection. As the prevalence of such a beneficial allele increases, genetic variants that happen to be present on the genomic background (the DNA neighbourhood) of the beneficial allele will also become more prevalent. This is called genetic hitchhiking. A selective sweep due to a strongly selected allele, which arose on a single genomic background therefore results in a region of the genome with a large reduction of genetic variation in that chromosome region.

Underlying linkage drag or selective sweep is the tendency of co-segregation of genetic elements, such as genes (alleles) or QTLs, i.e. crossover frequency between such genetic elements is low or (substantially) absent. Linkage drag or selective sweep typically reduce genetic variation of genetic variability or prevent the increase in genetic variation of genetic variability. In certain embodiments, the terms linkage drag and selective sweep may be used interchangeably.

As used herein, an altered or modified linkage drag or selective sweep may refer to an increase of decrease in linkage drag or selective sweep. An increased or decreased linkage drag or selective sweep respectively may result from a decreased or increased crossover frequency between genetic elements, such as at a given locus, or between (neighbouring) loci. An altered of modified linkage drag or selective sweep means that the (average) linkage drag or selective sweep has been changed, compared to the expected (average) linkage drag or selective sweep, and may ge determines for a particular chromosome, a chromosomal fragment, or the entirety of chromosomes. For instance linkage drag or selective sweep in a mutant plant, such as described herein elsewhere, may be altered compared to a wild type plant. In certain embodiments, (average) linkage drag or selective sweep (for the total of chromosomes, for a single chromosome, or for a given chromosomal fragment) is altered by at least 10%, such as at least 15%, at least 20%, at least 25%, at least 30%, at least 40%, or at least 50%. Preferably, (average) linkage drag or selective sweep is altered by at least 25%, more preferably at least 50%. In certain embodiments, (average) linkage drag or selective sweep (for the total of chromosomes, for a single chromosome, or for a given chromosomal fragment) is increased by at least 10%, such as at least 15%, at least 20%, at least 25%, at least 30%, at least 40%, or at least 50%. Preferably, (average) linkage drag or selective sweep is increased by at least 25%, more preferably at least 50%. In certain embodiments, (average) linkage drag or selective sweep (for the total of chromosomes, for a single chromosome, or for a given chromosomal fragment) is decreased by at least 10%, such as at least 15%, at least 20%, at least 25%, at least 30%, at least 40%, or at least 50%. Preferably, (average) linkage drag or selective sweep is decreased by at least 25%, more preferably at least 50%.

The term "locus" (loci plural) means a specific place or places or a site on a chromosome where for example a QTL, a gene or genetic marker is found. As used herein, the term "quantitative trait locus" or "QTL" has its ordinary meaning known in the art. By means of further guidance, and without limitation, a QTL may refer to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window. A QTL may encode for one or more alleles that affect the expressivity of a continuously distributed (quantitative) phenotype. In certain embodiments, the QTL as described herein may be homozygous. In certain embodiments, the QTL as described herein may be heterozygous.

As used herein, the term "allele" or "alleles" refers to one or more alternative forms, i.e. different nucleotide sequences, of a locus.

As used herein, the term "mutant alleles" or "mutation" of alleles include alleles having one or more mutations, such as insertions, deletions, stop codons, base changes (e.g. , transitions or transversions), or alterations in splice junctions, which may or may not give rise to altered gene products. Modifications in alleles may arise in coding or non-coding regions (e.g. promoter regions, exons, introns or splice junctions).

As used herein, a "mutation" refers to a modification at the DNA level, and includes changes in the genetics and/or epigenetics. An alteration in the genetics may include an insertion, a deletion, an introduction of a stop codon, a base change (e.g. transition or transversion), or an alteration in splice junctions. These alterations may arise in coding or non-coding regions (e.g. promoter regions, exons, introns or splice junctions) of the endogenous DNA sequence. For example, an alteration in the genetics may be the exchange of at least one nucleobase in the endogenous DNA sequence or in a regulatory sequence of the endogenous DNA sequence. If such a nucleobase exchange takes place in a promoter, for example, this may lead to an altered activity of the promoter, since, for example, cis-regulator elements are modified such that the affinity of a transcription factor to the mutated cis-regulatory elements is altered in comparison to the wild-type promoter, so that the activity of the promoter with the mutated cis-regulatory elements is increased or reduced, depending upon whether the transcription factor is a repressor or inductor, or whether the affinity of the transcription factor to the mutated cis-regulatory elements is intensified or weakened. If such a nucleobase exchange occurs, e.g., in an encoding region of the endogenous DNA sequence, this may lead to an amino acid exchange in the encoded protein, which may produce an alteration in the activity or stability of the protein, in comparison to the wild-type protein. An alteration in the epigenetics may take place via an altered methylation pattern of the DNA.

As used herein, the terms "introgression", "introgressed" and "introgressing" refer to both a natural and artificial process whereby chromosomal fragments or genes of one species, variety or cultivar are moved into the genome of another species, variety or cultivar, by crossing those species. The process may optionally be completed by backcrossing to the recurrent parent. For example, introgression of a desired allele at a specified locus can be transmitted to at least one progeny via a sexual cross between two parents of the same species, where at least one of the parents has the desired allele in its genome. Alternatively, for example, transmission of an allele can occur by recombination between two donor genomes, e.g., in a fused protoplast, where at least one of the donor protoplasts has the desired allele in its genome. The desired allele can be, e.g., detected by a marker that is associated with a phenotype, at a QTL, a transgene, or the like. In any case, offspring comprising the desired allele can be repeatedly backcrossed to a line having a desired genetic background and selected for the desired allele, to result in the allele becoming fixed in a selected genetic background. The process of "introgressing" is often referred to as "backcrossing" when the process is repeated two or more times. "Introgression fragment" or "introgression segment" or "introgression region" refers to a chromosome fragment (or chromosome part or region) which has been introduced into another plant of the same or related species either artificially or naturally such as by crossing or traditional breeding techniques, such as backcrossing, i.e. the introgressed fragment is the result of breeding methods referred to by the verb "to introgress" (such as backcrossing). It is understood that the term "introgression fragment" never includes a whole chromosome, but only a part of a chromosome. The introgression fragment can be large, e.g. even three quarter or half of a chromosome, but is preferably smaller, such as about 15 Mb or less, such as about 10 Mb or less, about 9 Mb or less, about 8 Mb or less, about 7 Mb or less, about 6 Mb or less, about 5 Mb or less, about 4 Mb or less, about 3 Mb or less, about 2.5 Mb or 2 Mb or less, about 1 Mb (equals 1,000,000 base pairs) or less, or about 0.5 Mb (equals 500,000 base pairs) or less, such as about 200,000 bp (equals 200 kilo base pairs) or less, about 100,000 bp (100 kb) or less, about 50,000 bp (50 kb) or less, about 25,000 bp (25 kb) or less.

A genetic element, an introgression fragment, or a gene or allele conferring a trait (such as modified crossover frequency, distribution, or interference or modified linkage drag or selective sweep) is said to be "obtainable from" or can be "obtained from" or "derivable from" or can be "derived from" or "as present in" or "as found in" a plant or plant part as described herein elsewhere if it can be transferred from the plant in which it is present into another plant in which it is not present (such as a line or variety) using traditional breeding techniques without resulting in a phenotypic change of the recipient plant apart from the addition of the trait conferred by the genetic element, locus, introgression fragment, gene or allele. The terms are used interchangeably and the genetic element, locus, introgression fragment, gene or allele can thus be transferred into any other genetic background lacking the trait. Not only pants comprising the genetic element, locus, introgression fragment, gene or allele can be used, but also progeny/descendants from such plants which have been selected to retain the genetic element, locus, introgression fragment, gene or allele, can be used and are encompassed herein. Whether a plant (or genomic DNA, cell or tissue of a plant) comprises the same genetic element, locus, introgression fragment, gene or allele as obtainable from such plant can be determined by the skilled person using one or more techniques known in the art, such as phenotypic assays, whole genome sequencing, molecular marker analysis, trait mapping, chromosome painting, allelism tests and the like, or combinations of techniques. It will be understood that transgenic plants may also be encompassed.

As used herein the terms "genetic engineering", "transformation" and "genetic modification" are all used herein as synonyms for the transfer of isolated and cloned genes into the DNA, usually the chromosomal DNA or genome, of another organism.

"Transgenic" or "genetically modified organisms" (GMOs) as used herein are organisms whose genetic material has been altered using techniques generally known as "recombinant DNA technology". Recombinant DNA technology encompasses the ability to combine DNA molecules from different sources into one molecule ex vivo (e.g. in a test tube). This terminology generally does not cover organisms whose genetic composition has been altered by conventional cross-breeding or by "mutagenesis" breeding, as these methods predate the discovery of recombinant DNA techniques. "Non-transgenic" as used herein refers to plants and food products derived from plants that are not "transgenic" or "genetically modified organisms" as defined above.

"Transgene" or "chimeric gene" refers to a genetic locus comprising a DNA sequence, such as a recombinant gene, which has been introduced into the genome of a plant by transformation, such as Agrobacterium mediated transformation. A plant comprising a transgene stably integrated into its genome is referred to as "transgenic plant".

"Gene editing" or "genome editing" refers to genetic engineering in which in which DNA or RNA is inserted, deleted, modified or replaced in the genome of a living organism. Gene editing may comprise targeted or non-targeted (random) mutagenesis. Targeted mutagenesis may be accomplished for instance with designer nucleases, such as for instance with meganucleases, zinc finger nucleases (ZFNs), transcription activator-like effector-based nucleases (TALEN), and the clustered regularly interspaced short palindromic repeats (CRISPR/Cas9) system. These nucleases create site-specific double-strand breaks (DSBs) at desired locations in the genome. The induced double-strand breaks are repaired through nonhomologous end-joining (NHEJ) or homologous recombination (HR), resulting in targeted mutations or nucleic acid modifications. The use of designer nucleases is particularly suitable for generating gene knockouts or knockdowns. In certain embodiments, designer nucleases are developed which specifically induce a mutation in the Pch2 gene, as described herein elsewhere, such as to generate a mutated Pch2 or a knockout of the Pch2 gene. In certain embodiments, designer nucleases, in particular RNA-specific CRISPR/Cas systems are developed which specifically target the Pch2 mRNA, such as to cleave the Pch2 mRNA and generate a knockdown of the Pch2 gene/mRNA/protein. Delivery and expression systems of designer nuclease systems are well known in the art.

In certain embodiments, the nuclease or targeted/site-specific/homing nuclease is, comprises, consists essentially of, or consists of a (modified) CRISPR/Cas system or complex, a (modified) Cas protein, a (modified) zinc finger, a (modified) zinc finger nuclease (ZFN), a (modified) transcription factor-like effector (TALE), a (modified) transcription factor-like effector nuclease (TALEN), or a (modified) meganuclease. In certain embodiments, said (modified) nuclease or targeted/site-specific/homing nuclease is, comprises, consists essentially of, or consists of a (modified) RNA-guided nuclease. It will be understood that in certain embodiments, the nucleases may be codon optimized for expression in plants. As used herein, the term "targeting" of a selected nucleic acid sequence means that a nuclease or nuclease complex is acting in a nucleotide sequence specific manner. For instance, in the context of the CRISPR/Cas system, the guide RNA is capable of hybridizing with a selected nucleic acid sequence. As uses herein, "hybridization" or "hybridizing" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PGR, or the cleavage of a polynucleotide by an enzyme. A sequence capable of hybridizing with a given sequence is referred to as the "complement" of the given sequence.

Gene editing may involve transient, inducible, or constitutive expression of the gene editing components or systems. Gene editing may involve genomic integration or episomal presence of the gene editing components or systems. Gene editing components or systems may be provided on vectors, such as plasmids, which may be delivered by appropriate delivery vehicles, as is known in the art. Preferred vectors are expression vectors.

Gene editing may comprise the provision of recombination templates, to effect homology directed repair (HDR). For instance a genetic element may be replaced by gene editing in which a recombination template is provided. The DNA may be cut upstream and downstream of a sequence which needs to be replaced. As such, the sequence to be replaced is excised from the DNA. Through HDR, the excised sequence is then replaced by the template. In certain embodiments, the QTL allele of the invention as described herein may be provided on/as a template. By designing the system such that double strand breaks are introduced upstream and downstream of the corresponding region in the genome of a plant not comprising the QTL allele, this region is excised and can be replaced with the template comprising the QTL allele of the invention. In this way, introduction of the QTL allele of the invention in a plant need not involve multiple backcrossing, in particular in a plant of specific genetic background. Similarly, the mutated PCH2 of the invention may be provided on/as a template. More advantageously however, the mutated PCH2 of the invention may be generated without the use of a recombination template, but solely through the endonuclease action leading to a double strand DNA break which is repaired by NHEJ, resulting in the generation of indels.

In certain embodiments, the nucleic acid modification or mutation is effected by a (modified) transcription activator-like effector nuclease (TALEN) system. Transcription activator-like effectors (TALEs) can be engineered to bind practically any desired DNA sequence. Exemplary methods of genome editing using the TALEN system can be found for example in Cermak T. Doyle EL. Christian M. Wang L. Zhang Y. Schmidt C, et al. Efficient design and assembly of custom TALEN and other TAL effector-based constructs for DNA targeting. Nucleic Acids Res. 2011;39:e82; Zhang F. Cong L. Lodato S. Kosuri S. Church GM. Arlotta P Efficient construction of sequence-specific TAL effectors for modulating mammalian transcription. Nat Biotechnol. 2011;29:149-153 and US Patent Nos. 8,450,471, 8,440,431 and 8,440,432, all of which are specifically incorporated by reference. By means of further guidance, and without limitation, naturally occurring TALEs or "wild type TALEs" are nucleic acid binding proteins secreted by numerous species of proteobacteria. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. In advantageous embodiments the nucleic acid is DNA. As used herein, the term "polypeptide monomers", or "TALE monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. As provided throughout the disclosure, the amino acid residues of the RVD are depicted using the IUPAC single letter code for amino acids. A general representation of a TALE monomer which is comprised within the DNA binding domain is X1-11-(X12X13)-X14-33 or 34 or 35, where the subscript indicates the amino acid position and X represents any amino acid. X12X13 indicate the RVDs. In some polypeptide monomers, the variable amino acid at position 13 is missing or absent and in such polypeptide monomers, the RVD consists of a single amino acid. In such cases the RVD may be alternatively represented as X*, where X represents X12 and (*) indicates that X13 is absent. The DNA binding domain comprises several repeats of TALE monomers and this may be represented as (X1-11-(X12X13)-X14-33 or 34 or 35)z, where in an advantageous embodiment, z is at least 5 to 40. In a further advantageous embodiment, z is at least 10 to 26. The TALE monomers have a nucleotide binding affinity that is determined by the identity of the amino acids in its RVD. For example, polypeptide monomers with an RVD of NI preferentially bind to adenine (A), polypeptide monomers with an RVD of NG preferentially bind to thymine (T), polypeptide monomers with an RVD of HD preferentially bind to cytosine (C) and polypeptide monomers with an RVD of NN preferentially bind to both adenine (A) and guanine (G). In yet another embodiment of the invention, polypeptide monomers with an RVD of IG preferentially bind to T. Thus, the number and order of the polypeptide monomer repeats in the nucleic acid binding domain of a TALE determines its nucleic acid target specificity. In still further embodiments of the invention, polypeptide monomers with an RVD of NS recognize all four base pairs and may bind to A, T, G or C. The structure and function of TALEs is further described in, for example, Moscou et al., Science 326:1501 (2009); Boch et al., Science 326:1509-1512 (2009); and Zhang et al., Nature Biotechnology 29:149-153 (2011), each of which is incorporated by reference in its entirety.

In certain embodiments, the nucleic acid modification or mutation is effected by a (modified) zinc-finger nuclease (ZFN) system. The ZFN system uses artificial restriction enzymes generated by fusing a zinc finger DNA-binding domain to a DNA-cleavage domain that can be engineered to target desired DNA sequences. Exemplary methods of genome editing using ZFNs can be found for example in U.S. Patent Nos. 6,534,261, 6,607,882, 6,746,838, 6,794,136, 6,824,978, 6,866,997, 6,933,113, 6,979,539, 7,013,219, 7,030,215, 7,220,719, 7,241,573, 7,241,574, 7,585,849, 7,595,376, 6,903,185, and 6,479,626, all of which are specifically incorporated by reference. By means of further guidance, and without limitation, artificial zinc-finger (ZF) technology involves arrays of ZF modules to target new DNA-binding sites in the genome. Each finger module in a ZF array targets three DNA bases. A customized array of individual zinc finger domains is assembled into a ZF protein (ZFP). ZFPs can comprise a functional domain. The first synthetic zinc finger nucleases (ZFNs) were developed by fusing a ZF protein to the catalytic domain of the Type IIS restriction enzyme Fokl. (Kim, Y. G. et al., 1994, Chimeric restriction endonuclease, Proc. Natl. Acad. Sci. U.S.A. 91, 883-887; Kim, Y. G. et al., 1996, Hybrid restriction enzymes: zinc finger fusions to Fok I cleavage domain. Proc. Natl. Acad. Sci. U.S.A. 93, 1156-1160). Increased cleavage specificity can be attained with decreased off target activity by use of paired ZFN heterodimers, each targeting different nucleotide sequences separated by a short spacer. (Doyon, Y. et al., 2011, Enhancing zinc-finger-nuclease activity with improved obligate heterodimeric architectures. Nat. Methods 8, 74-79). ZFPs can also be designed as transcription activators and repressors and have been used to target many genes in a wide variety of organisms.

In certain embodiments, the nucleic acid modification is effected by a (modified) meganuclease, which are endodeoxyribonucleases characterized by a large recognition site (double-stranded DNA sequences of 12 to 40 base pairs). Exemplary method for using meganucleases can be found in US Patent Nos: 8,163,514; 8,133,697; 8,021,867; 8,119,361; 8,119,381; 8,124,369; and 8,129,134, which are specifically incorporated by reference.

In certain embodiments, the nucleic acid modification is effected by a (modified) CRISPR/Cas complex or system. With respect to general information on CRISPR/Cas Systems, components thereof, and delivery of such components, including methods, materials, delivery vehicles, vectors, particles, and making and using thereof, including as to amounts and formulations, as well as Cas9CRISPR/Cas-expressing eukaryotic cells, Cas-9 CRISPR/Cas expressing eukaryotes, such as a mouse, reference is made to: US Patents Nos. 8,999,641, 8,993,233, 8,697,359, 8,771,945, 8,795,965, 8,865,406, 8,871,445, 8,889,356, 8,889,418, 8,895,308, 8,906,616, 8,932,814, 8,945,839, 8,993,233 and 8,999,641; US Patent Publications US 2014-0310830 (US App. Ser. No. 14/105,031), US 2014-0287938 A1 (U.S. App. Ser. No. 14/213,991), US 2014-0273234 A1 (U.S. App. Ser. No. 14/293,674), US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,017), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486), US 2014-0170753 (US App Ser No 14/183,429); US 2015-0184139 (U.S. App. Ser. No. 14/324,960); 14/054,414 European Patent Applications EP 2 771 468 (EP13818570.7), EP 2 764 103 (EP13824232.6), and EP 2 784 162 (EP14170383.5); and PCT Patent Publications WO 2014/093661 (PCT/US2013/074743), WO 2014/093694 (PCT/US2013/074790), WO 2014/093595 (PCT/US2013/074611), WO 2014/093718 (PCT/US2013/074825), WO 2014/093709 (PCT/US2013/074812), WO 2014/093622 (PCT/US2013/074667), WO 2014/093635 (PCT/US2013/074691), WO 2014/093655 (PCT/US2013/074736), WO 2014/093712 (PCT/US2013/074819), WO 2014/093701 (PCT/US2013/074800), WO 2014/018423 (PCT/US2013/051418), WO 2014/204723 (PCT/US2014/041790), WO 2014/204724 (PCT/US2014/041800), WO 2014/204725 (PCT/US2014/041803), WO 2014/204726 (PCT/US2014/041804), WO 2014/204727 (PCT/US2014/041806), WO 2014/204728 (PCT/US2014/041808), WO 2014/204729 (PCT/US2014/041809), WO 2015/089351 (PCT/US2014/069897), WO 2015/089354 (PCT/US2014/069902), WO 2015/089364 (PCT/US2014/069925), WO 2015/089427 (PCT/US2014/070068), WO 2015/089462 (PCT/US2014/070127), WO 2015/089419 (PCT/US2014/070057), WO 2015/089465 (PCT/US2014/070135), WO 2015/089486 (PCT/US2014/070175), PCT/US2015/051691, PCT/US2015/051830. Reference is also made to US provisional patent applications 61/758,468; 61/802,174; 61/806,375; 61/814,263; 61/819,803 and 61/828,130, filed on January 30, 2013; March 15, 2013; March 28, 2013; April 20, 2013; May 6, 2013 and May 28, 2013 respectively. Reference is also made to US provisional patent application 61/836,123, filed on June 17, 2013. Reference is additionally made to US provisional patent applications 61/835,931, 61/835,936, 61/835,973, 61/836,080, 61/836,101, and 61/836,127, each filed June 17, 2013. Further reference is made to US provisional patent applications 61/862,468 and 61/862,355 filed on August 5, 2013; 61/871,301 filed on August 28, 2013; 61/960,777 filed on September 25, 2013 and 61/961,980 filed on October 28, 2013. Reference is yet further made to: PCT/US2014/62558 filed October 28, 2014, and US Provisional Patent Applications Serial Nos.: 61/915,148, 61/915,150, 61/915,153, 61/915,203, 61/915,251, 61/915,301, 61/915,267, 61/915,260, and 61/915,397, each filed December 12, 2013; 61/757,972 and 61/768,959, filed on January 29, 2013 and February 25, 2013; 62/010,888 and 62/010,879, both filed June 11, 2014; 62/010,329, 62/010,439 and 62/010,441, each filed June 10, 2014; 61/939,228 and 61/939,242, each filed February 12, 2014; 61/980,012, filed April 15,2014; 62/038,358, filed August 17, 2014; 62/055,484, 62/055,460 and 62/055,487, each filed September 25, 2014; and 62/069,243, filed October 27, 2014. Reference is made to PCT application designating, inter alia, the United States, application No. PCT/US14/41806, filed June 10, 2014. Reference is made to US provisional patent application 61/930,214 filed on January 22, 2014. Reference is made to PCT application designating, inter alia, the United States, application No. PCT/US14/41806, filed June 10, 2014. Mention is also made of US application 62/180,709, 17-Jun-15, PROTECTED GUIDE RNAS (PGRNAS); US application 62/091,455, filed, 12-Dec-14, PROTECTED GUIDE RNAS (PGRNAS); US application 62/096,708, 24-Dec-14, PROTECTED GUIDE RNAS (PGRNAS); US applications 62/091,462, 12-Dec-14, 62/096,324, 23-Dec-14, 62/180,681, 17-Jun-2015, and 62/237,496, 5-Oct-2015, DEAD GUIDES FOR CRISPR TRANSCRIPTION FACTORS; US application 62/091,456, 12-Dec-14 and 62/180,692, 17-Jun-2015, ESCORTED AND FUNCTIONALIZED GUIDES FOR CRISPR-CAS SYSTEMS; US application 62/091,461, 12-Dec-14, DELIVERY, USE AND THERAPEUTIC APPLICATIONS OF THE CRISPR-CAS SYSTEMS AND COMPOSITIONS FOR GENOME EDITING AS TO HEMATOPOETIC STEM CELLS (HSCs); US application 62/094,903, 19-Dec-14, UNBIASED IDENTIFICATION OF DOUBLE-STRAND BREAKS AND GENOMIC REARRANGEMENT BY GENOME-WISE INSERT CAPTURE SEQUENCING; US application 62/096,761, 24-Dec-14, ENGINEERING OF SYSTEMS, METHODS AND OPTIMIZED ENZYME AND GUIDE SCAFFOLDS FOR SEQUENCE MANIPULATION; US application 62/098,059, 30-Dec-14, 62/181,641, 18-Jun-2015, and 62/181,667, 18-Jun-2015, RNA-TARGETING SYSTEM; US application 62/096,656, 24-Dec-14 and 62/181,151, 17-Jun-2015, CRISPR HAVING OR ASSOCIATED WITH DESTABILIZATION DOMAINS; US application 62/096,697, 24-Dec-14, CRISPR HAVING OR ASSOCIATED WITH AAV; US application 62/098,158, 30-Dec-14, ENGINEERED CRISPR COMPLEX INSERTIONAL TARGETING SYSTEMS; US application 62/151,052, 22-Apr-15, CELLULAR TARGETING FOR EXTRACELLULAR EXOSOMAL REPORTING; US application 62/054,490, 24-Sep-14, DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS; US application 61/939,154, 12-FEB-14, SYSTEMS, METHODS AND COMPOSITIONS FOR SEQUENCE MANIPULATION WITH OPTIMIZED FUNCTIONAL CRISPR-CAS SYSTEMS; US application 62/055,484, 25-Sep-14, SYSTEMS, METHODS AND COMPOSITIONS FOR SEQUENCE MANIPULATION WITH OPTIMIZED FUNCTIONAL CRISPR-CAS SYSTEMS; US application 62/087,537, 4-Dec-14, SYSTEMS, METHODS AND COMPOSITIONS FOR SEQUENCE MANIPULATION WITH OPTIMIZED FUNCTIONAL CRISPR-CAS SYSTEMS; US application 62/054,651, 24-Sep-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/067,886, 23-Oct-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US applications 62/054,675, 24-Sep-14 and 62/181,002, 17-Jun-2015, DELIVERY, USE AND THERAPEUTIC APPLICATIONS OF THE CRISPR-CAS SYSTEMS AND COMPOSITIONS IN NEURONAL CELLS/TISSUES; US application 62/054,528, 24-Sep-14, DELIVERY, USE AND THERAPEUTIC APPLICATIONS OF THE CRISPR-CAS SYSTEMS AND COMPOSITIONS IN IMMUNE DISEASES OR DISORDERS; US application 62/055,454, 25-Sep-14, DELIVERY, USE AND THERAPEUTIC APPLICATIONS OF THE CRISPR-CAS SYSTEMS AND COMPOSITIONS FOR TARGETING DISORDERS AND DISEASES USING CELL PENETRATION PEPTIDES (CPP); US application 62/055,460, 25-Sep-14, MULTIFUNCTIONAL-CRISPR COMPLEXES AND/OR OPTIMIZED ENZYME LINKED FUNCTIONAL-CRISPR COMPLEXES; US application 62/087,475, 4-Dec-14 and 62/181,690, 18-Jun-2015, FUNCTIONAL SCREENING WITH OPTIMIZED FUNCTIONAL CRISPR-CAS SYSTEMS; US application 62/055,487, 25-Sep-14, FUNCTIONAL SCREENING WITH OPTIMIZED FUNCTIONAL CRISPR-CAS SYSTEMS; US application 62/087,546, 4-Dec-14 and 62/181,687, 18-Jun-2015, MULTIFUNCTIONAL CRISPR COMPLEXES AND/OR OPTIMIZED ENZYME LINKED FUNCTIONAL-CRISPR COMPLEXES; and US application 62/098,285, 30-Dec-14, CRISPR MEDIATED IN VIVO MODELING AND GENETIC SCREENING OF TUMOR GROWTH AND METASTASIS. Mention is made of US applications 62/181,659, 18-Jun-2015 and 62/207,318, 19-Aug-2015, ENGINEERING AND OPTIMIZATION OF SYSTEMS, METHODS, ENZYME AND GUIDE SCAFFOLDS OF CAS9 ORTHOLOGS AND VARIANTS FOR SEQUENCE MANIPULATION. Mention is made of US applications 62/181,663, 18-Jun-2015 and 62/245,264, 22-Oct-2015, NOVEL CRISPR ENZYMES AND SYSTEMS, US applications 62/181,675, 18-Jun-2015, and Attorney Docket No. 46783.01.2128, filed 22-Oct-2015, NOVEL CRISPR ENZYMES AND SYSTEMS, US application 62/232,067, 24-Sep-2015, US application 62/205,733, 16-Aug-2015, US application 62/201,542, 5-Aug-2015, US application 62/193,507, 16-Jul-2015, and US application 62/181,739, 18-Jun-2015, each entitled NOVEL CRISPR ENZYMES AND SYSTEMS and of US application 62/245,270, 22-Oct-2015, NOVEL CRISPR ENZYMES AND SYSTEMS. Mention is also made of US application 61/939,256, 12-Feb-2014, and WO 2015/089473 (PCT/US2014/070152), 12-Dec-2014, each entitled ENGINEERING OF SYSTEMS, METHODS AND OPTIMIZED GUIDE COMPOSITIONS WITH NEW ARCHITECTURES FOR SEQUENCE MANIPULATION. Mention is also made of PCT/US2015/045504, 15-Aug-2015, US application 62/180,699, 17-Jun-2015, and US application 62/038,358, 17-Aug-2014, each entitled GENOME EDITING USING CAS9 NICKASES. European patent application EP3009511. Reference is further made to Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013); RNA-guided editing of bacterial genomes using CRISPR-Cas systems. Jiang W., Bikard D., Cox D., Zhang F, Marraffini LA. Nat Biotechnol Mar;31(3):233-9 (2013); One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013); Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23; Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013); DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013); Genome engineering using the CRISPR-Cas9 system. Ran, FA., Hsu, PD., Wright, J., Agarwala, V., Scott, DA., Zhang, F. Nature Protocols Nov;8(11):2281-308. (2013); Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print]; Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49; Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889; CRISPR-Cas9 Knockin Mice for Genome Editing and Cancer Modeling, Platt et al., Cell 159(2): 440-455 (2014) DOI: 10.1016/j.cell.2014.09.014; Development and Applications of CRISPR-Cas9 for Genome Engineering, Hsu et al, Cell 157, 1262-1278 (June 5, 2014) (Hsu 2014); Genetic screens in human cells using the CRISPR/Cas9 system, Wang et al., Science. 2014 January 3; 343(6166): 80-84. doi:10.1126/science.1246981; Rational design of highly active sgRNAs for CRISPR-Cas9-mediated gene inactivation, Doench et al., Nature Biotechnology 32(12):1262-7 (2014) published online 3 September 2014; doi:10.1038/nbt.3026, and In vivo interrogation of gene function in the mammalian brain using CRISPR-Cas9, Swiech et al, Nature Biotechnology 33, 102-106 (2015) published online 19 October 2014; doi:10.1038/nbt.3055, Cpf1 Is a Single RNA-Guided Endonuclease of a Class 2 CRISPR-Cas System, Zetsche et al., Cell 163, 1-13 (2015); Discovery and Functional Characterization of Diverse Class 2 CRISPR-Cas Systems, Shmakov et al., Mol Cell 60(3): 385-397 (2015); C2c2 is a single-component programmable RNA-guided RNA-targeting CRISPR effector, Abudayyeh et al, Science (2016) published online June 2, 2016 doi: 10.1126/science.aaf5573. Each of these publications, patents, patent publications, and applications, and all documents cited therein or during their prosecution ("appln cited documents") and all documents cited or referenced in the appln cited documents, together with any instructions, descriptions, product specifications, and product sheets for any products mentioned therein or in any document therein and incorporated by reference herein, are hereby incorporated herein by reference, and may be employed in the practice of the invention. All documents (e.g., these patents, patent publications and applications and the appln cited documents) are incorporated herein by reference to the same extent as if each individual document was specifically and individually indicated to be incorporated by reference.

In certain embodiments, the CRISPR/Cas system or complex is a class 2 CRISPR/Cas system. In certain embodiments, said CRISPR/Cas system or complex is a type II, type V, or type VI CRISPR/Cas system or complex. The CRISPR/Cas system does not require the generation of customized proteins to target specific sequences but rather a single Cas protein can be programmed by an RNA guide (gRNA) to recognize a specific nucleic acid target, in other words the Cas enzyme protein can be recruited to a specific nucleic acid target locus (which may comprise or consist of RNA and/or DNA) of interest using said short RNA guide.

In general, the CRISPR/Cas or CRISPR system is as used herein foregoing documents refers collectively to transcripts and other elements involved in the expression of or directing the activity of CRISPR-associated ("Cas") genes, including sequences encoding a Cas gene and one or more of, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g. CRISPR RNA and, where applicable, transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides.

In certain embodiments, the gRNA is a chimeric guide RNA or single guide RNA (sgRNA). In certain embodiments, the gRNA comprises a guide sequence and a tracr mate sequence (or direct repeat). In certain embodiments, the gRNA comprises a guide sequence, a tracr mate sequence (or direct repeat), and a tracr sequence. In certain embodiments, the CRISPR/Cas system or complex as described herein does not comprise and/or does not rely on the presence of a tracr sequence (e.g. if the Cas protein is Cpf1).

As used herein, the term "crRNA" or "guide RNA" or "single guide RNA" or "sgRNA" or "one or more nucleic acid components" of a CRISPR/Cas locus effector protein, as applicable, comprises any polynucleotide sequence having sufficient complementarity with a target nucleic acid sequence to hybridize with the target nucleic acid sequence and direct sequence-specific binding of a nucleic acid-targeting complex to the target nucleic acid sequence. In some embodiments, the degree of complementarity, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, nonlimiting example of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). The ability of a guide sequence (within a nucleic acid-targeting guide RNA) to direct sequence-specific binding of a nucleic acid -targeting complex to a target nucleic acid sequence may be assessed by any suitable assay.

A guide sequence, and hence a nucleic acid-targeting guide RNA may be selected to target any target nucleic acid sequence. The target sequence may be DNA. The target sequence may be genomic DNA. The target sequence may be mitochondrial DNA. The target sequence may be any RNA sequence. In some embodiments, the target sequence may be a sequence within a RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (IncRNA), and small cytoplasmatic RNA (scRNA). In some preferred embodiments, the target sequence may be a sequence within a RNA molecule selected from the group consisting of mRNA, pre-mRNA, and rRNA. In some preferred embodiments, the target sequence may be a sequence within a RNA molecule selected from the group consisting of ncRNA, and IncRNA. In some more preferred embodiments, the target sequence may be a sequence within an mRNA molecule or a pre-mRNA molecule.

In certain embodiments, the gRNA comprises a stem loop, preferably a single stem loop. In certain embodiments, the direct repeat sequence forms a stem loop, preferably a single stem loop. In certain embodiments, the spacer length of the guide RNA is from 15 to 35 nt. In certain embodiments, the spacer length of the guide RNA is at least 15 nucleotides. In certain embodiments, the spacer length is from 15 to 17 nt, e.g., 15, 16, or 17 nt, from 17 to 20 nt, e.g., 17, 18, 19, or 20 nt, from 20 to 24 nt, e.g., 20, 21, 22, 23, or 24 nt, from 23 to 25 nt, e.g., 23, 24, or 25 nt, from 24 to 27 nt, e.g., 24, 25, 26, or 27 nt, from 27-30 nt, e.g., 27, 28, 29, or 30 nt, from 30-35 nt, e.g., 30, 31, 32, 33, 34, or 35 nt, or 35 nt or longer. In particular embodiments, the CRISPR/Cas system requires a tracrRNA. The "tracrRNA" sequence or analogous terms includes any polynucleotide sequence that has sufficient complementarity with a crRNA sequence to hybridize. In some embodiments, the degree of complementarity between the tracrRNA sequence and crRNA sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and gRNA sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop may correspond to the tracr mate sequence, and the portion of the sequence 3' of the loop then corresponds to the tracr sequence. In a hairpin structure the portion of the sequence 5' of the final "N" and upstream of the loop may alternatively correspond to the tracr sequence, and the portion of the sequence 3' of the loop corresponds to the tracr mate sequence. In alternative embodiments, the CRISPR/Cas system does not require a tracrRNA, as is known by the skilled person.

In certain embodiments, the guide RNA (capable of guiding Cas to a target locus) may comprise (1) a guide sequence capable of hybridizing to a target locus and (2) a tracr mate or direct repeat sequence (in 5' to 3' orientation, or alternatively in 3' to 5' orientation, depending on the type of Cas protein, as is known by the skilled person). In particular embodiments, the CRISPR/Cas protein is characterized in that it makes use of a guide RNA comprising a guide sequence capable of hybridizing to a target locus and a direct repeat sequence, and does not require a tracrRNA. In particular embodiments, where the CRISPR/Cas protein is characterized in that it makes use of a tracrRNA, the guide sequence, tracr mate, and tracr sequence may reside in a single RNA, i.e. an sgRNA (arranged in a 5' to 3' orientation or alternatively arranged in a 3' to 5' orientation), or the tracr RNA may be a different RNA than the RNA containing the guide and tracr mate sequence. In these embodiments, the tracr hybridizes to the tracr mate sequence and directs the CRISPR/Cas complex to the target sequence.

Typically, in the context of an endogenous nucleic acid-targeting system, formation of a nucleic acid-targeting complex (comprising a guide RNA hybridized to a target sequence and complexed with one or more nucleic acid-targeting effector proteins) results in modification (such as cleavage) of one or both DNA or RNA strands in or near (e.g., within 1, 2, 3, 4, 5, 6, 7, 8, 9, 10, 20, 50, or more base pairs from) the target sequence. As used herein the term "sequence(s) associated with a target locus of interest" refers to sequences near the vicinity of the target sequence (e.g. within 1, 2, 3, 4, 5, 6, 7, 8, 9, 10, 20, 50, or more base pairs from the target sequence, wherein the target sequence is comprised within a target locus of interest). The skilled person will be aware of specific cut sites for selected CRISPR/Cas systems, relative to the target sequence, which as is known in the art may be within the target sequence or alternatively 3' or 5' of the target sequence.

In some embodiments, the unmodified nucleic acid-targeting effector protein may have nucleic acid cleavage activity. In some embodiments, the nuclease as described herein may direct cleavage of one or both nucleic acid (DNA, RNA, or hybrids, which may be single or double stranded) strands at the location of or near a target sequence, such as within the target sequence and/or within the complement of the target sequence or at sequences associated with the target sequence. In some embodiments, the nucleic acid-targeting effector protein may direct cleavage of one or both DNA or RNA strands within about 1, 2, 3, 4, 5, 6, 7, 8, 9, 10, 15, 20, 25, 50, 100, 200, 500, or more base pairs from the first or last nucleotide of a target sequence. In some embodiments, the cleavage may be blunt (e.g. for Cas9, such as SaCas9 or SpCas9). In some embodiments, the cleavage may be staggered (e.g. for Cpf1), i.e. generating sticky ends. In some embodiments, the cleavage is a staggered cut with a 5' overhang. In some embodiments, the cleavage is a staggered cut with a 5' overhang of 1 to 5 nucleotides, preferably of 4 or 5 nucleotides. In some embodiments, the cleavage site is upstream of the PAM. In some embodiments, the cleavage site is downstream of the PAM. In some embodiments, the nucleic acid-targeting effector protein that may be mutated with respect to a corresponding wild-type enzyme such that the mutated nucleic acid-targeting effector protein lacks the ability to cleave one or both DNA or RNA strands of a target polynucleotide containing a target sequence. As a further example, two or more catalytic domains of a Cas protein (e.g. RuvC I, RuvC II, and RuvC III or the HNH domain of a Cas9 protein) may be mutated to produce a mutated Cas protein substantially lacking all DNA cleavage activity. In some embodiments, a nucleic acid-targeting effector protein may be considered to substantially lack all DNA and/or RNA cleavage activity when the cleavage activity of the mutated enzyme is about no more than 25%, 10%, 5%, 1%, 0.1%, 0.01%, or less of the nucleic acid cleavage activity of the non-mutated form of the enzyme; an example can be when the nucleic acid cleavage activity of the mutated form is nil or negligible as compared with the non-mutated form. As used herein, the term "modified" Cas generally refers to a Cas protein having one or more modifications or mutations (including point mutations, truncations, insertions, deletions, chimeras, fusion proteins, etc.) compared to the wild type Cas protein from which it is derived. By derived is meant that the derived enzyme is largely based, in the sense of having a high degree of sequence homology with, a wildtype enzyme, but that it has been mutated (modified) in some way as known in the art or as described herein.

In certain embodiments, the target sequence should be associated with a PAM (protospacer adjacent motif) or PFS (protospacer flanking sequence or site); that is, a short sequence recognized by the CRISPR complex. The precise sequence and length requirements for the PAM differ depending on the CRISPR enzyme used, but PAMs are typically 2-5 base pair sequences adjacent the protospacer (that is, the target sequence). Examples of PAM sequences are given in the examples section below, and the skilled person will be able to identify further PAM sequences for use with a given CRISPR enzyme. Further, engineering of the PAM Interacting (PI) domain may allow programing of PAM specificity, improve target site recognition fidelity, and increase the versatility of the Cas, e.g. Cas9, genome engineering platform. Cas proteins, such as Cas9 proteins may be engineered to alter their PAM specificity, for example as described in Kleinstiver BP et al. Engineered CRISPR-Cas9 nucleases with altered PAM specificities. Nature. 2015 Jul 23;523(7561):481-5. doi: 10.1038/nature14592. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. The skilled person will understand that other Cas proteins may be modified analogously.

The Cas protein as referred to herein, such as without limitation Cas9, Cpf1 (Cas12a), C2c1 (Cas12b), C2c2 (Cas13a), C2c3, Cas13b protein, may originate from any suitable source, and hence may include different orthologues, originating from a variety of (prokaryotic) organisms, as is well documented in the art. In certain embodiments, the Cas protein is (modified) Cas9, preferably (modified) Staphylococcus aureus Cas9 (SaCas9) or (modified) Streptococcus pyogenes Cas9 (SpCas9). In certain embodiments, the Cas protein is (modified) Cpf1, preferably Acidaminococcus sp., such as Acidaminococcus sp. BV3L6 Cpf1 (AsCpf1) or Lachnospiraceae bacterium Cpf1, such as Lachnospiraceae bacterium MA2020 or Lachnospiraceae bacterium MD2006 (LbCpf1). In certain embodiments, the Cas protein is (modified) C2c2, preferably Leptotrichia wadei C2c2 (LwC2c2) or Listeria newyorkensis FSL M6-0635 C2c2 (LbFSLC2c2). In certain embodiments, the (modified) Cas protein is C2c1. In certain embodiments, the (modified) Cas protein is C2c3. In certain embodiments, the (modified) Cas protein is Cas13b.

In certain embodiments, the nucleic acid modification is effected by random mutagenesis. Cells or organisms may be exposed to mutagens such as UV radiation or mutagenic chemicals (such as for instance such as ethyl methanesulfonate (EMS)), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nucleases. The products are then separated by size, such as by HPLC. See also McCallum et al. "Targeted screening for induced mutations"; Nat Biotechnol. 2000 Apr;18(4):455-7 and McCallum et al. "Targeting induced local lesions IN genomes (TILLING) for plant functional genomics"; Plant Physiol. 2000 Jun;123(2):439-42.

RNA interference (RNAi) is a biological process in which RNA molecules inhibit gene expression or translation, by neutralizing targeted mRNA molecules. Two types of small ribonucleic acid (RNA) molecules - microRNA (miRNA) and small interfering RNA (siRNA) - are central to RNA interference. RNAs are the direct products of genes, and these small RNAs can bind to other specific messenger RNA (mRNA) molecules and either increase or decrease their activity, for example by preventing an mRNA from being translated into a protein. The RNAi pathway is found in many eukaryotes, including animals, and is initiated by the enzyme Dicer, which cleaves long double-stranded RNA (dsRNA) molecules into short double-stranded fragments of about 21 nucleotide siRNAs (small interfering RNAs). Each siRNA is unwound into two single-stranded RNAs (ssRNAs), the passenger strand and the guide strand. The passenger strand is degraded and the guide strand is incorporated into the RNA-induced silencing complex (RISC). Mature miRNAs are structurally similar to siRNAs produced from exogenous dsRNA, but before reaching maturity, miRNAs must first undergo extensive post-transcriptional modification. A miRNA is expressed from a much longer RNA-coding gene as a primary transcript known as a pri-miRNA which is processed, in the cell nucleus, to a 70-nucleotide stem-loop structure called a pre-miRNA by the microprocessor complex. This complex consists of an RNase III enzyme called Drosha and a dsRNA-binding protein DGCR8. The dsRNA portion of this pre-miRNA is bound and cleaved by Dicer to produce the mature miRNA molecule that can be integrated into the RISC complex; thus, miRNA and siRNA share the same downstream cellular machinery. A short hairpin RNA or small hairpin RNA (shRNA/Hairpin Vector) is an artificial RNA molecule with a tight hairpin turn that can be used to silence target gene expression via RNA interference. The most well-studied outcome is post-transcriptional gene silencing, which occurs when the guide strand pairs with a complementary sequence in a messenger RNA molecule and induces cleavage by Argonaute 2 (Ago2), the catalytic component of the RISC. As used herein, an RNAi molecule may be an siRNA, shRNA, or a miRNA. In will be understood that the RNAi molecules can be applied as such to/in the plant, or can be encoded by appropriate vectors, from which the RNAi molecule is expressed. Delivery and expression systems of RNAi molecules, such as siRNAs, shRNAs or miRNAs are well known in the art.

As used herein, Pch2 refers to the pachytene checkpoint protein 2 (also called pachytene checkpoint protein 2 homolog), which is an AAA-ATPase first identified as a pachytene checkpoint factor in budding yeast (San-Segundo and Roeder, 1999). Pch2 is present in most eukaryotes, including plants. Non limiting (wild type) representative genomic sequences of Pch2 as used herein are provided in SEQ ID NOs: 31, 34, 37, and 40. Non limiting (wild type) representative cDNA sequences of Pch2 as used herein are provided in SEQ ID NOs: 32, 35, 38, and 41. Non limiting (wild type) representative protein sequences of PCH2 as used herein are provided in SEQ ID NOs: 33, 36, 39, and 42.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

Amino acid substitutions encompass amino acid alterations in which an amino acid is replaced with a different naturally-occurring amino acid residue. Such substitutions may be classified as "conservative<1>, in which an amino acid residue contained in the wild-type protein is replaced with another naturally-occurring amino acid of similar character, for example Gly<→Ala, Val<→Ile<→Leu, Asp<→Glu, Lys<→Arg, Asn<→ Gln or Phe<→Trp<→Tyr. Substitutions encompassed by the present invention may also be "non-conservative", in which an amino acid residue which is present in the wild-type protein is substituted with an amino acid with different properties, such as a naturally-occurring amino acid from a different group (e.g. substituting a charged or hydrophobic amino acid with alanine. "Similar amino acids", as used herein, refers to amino acids that have similar amino acid side chains, i.e. amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

A used herein, the term "endogenous" refers to a gene or allele which is present in its natural genomic location. The term "endogenous" can be used interchangeably with "native". This does not however exclude the presence of one or more nucleic acid differences with the wild-type allele. In particular embodiments, the difference with a wild-type allele can be limited to less than 9 preferably less than 6, more particularly less than 3 nucleotide differences. More particularly, the difference with the wildtype sequence can be in only one nucleotide. Preferably, the endogenous allele encodes a modified protein having less than 9, preferably less than 6, more particularly less than 3 and even more preferably only one amino acid difference with the wild-type protein. In certain embodiments, the endogenous gene or allele is the wild type gene or allele. In certain embodiments, the endogenous gene is mutagenized, as described herein elsewhere. In certain embodiments, expression, activity, and/or stability of the endogenous gene is modified, as described herein elsewhere.

As used herein, the term "homozygote" refers to an individual cell or plant having the same alleles at one or more or all loci. When the term is used with reference to a specific locus or gene, it means at least that locus or gene has the same alleles. As used herein, the term "homozygous" means a genetic condition existing when identical alleles reside at corresponding loci on homologous chromosomes. As used herein, the term "heterozygote" refers to an individual cell or plant having different alleles at one or more or all loci. When the term is used with reference to a specific locus or gene, it means at least that locus or gene has different alleles. As used herein, the term "heterozygous" means a genetic condition existing when different alleles reside at corresponding loci on homologous chromosomes. In certain embodiments, the Pch2 gene as described herein is homozygous (e.g. mutated Pch2 on all homologous or homeologous chromosomes). In certain embodiments, the Pch2 gene as described herein is heterozygous (e.g. (at least) one mutated Pch2 and (at least) one wild type Pch2 on homologous or homeologous chromosomes).

A "polymorphism" is a variation in the DNA between two or more individuals within a population. A polymorphism preferably has a frequency of at least 1 % in a population. A useful polymorphism can include a single nucleotide polymorphism (SNP), a simple sequence repeat (SSR), or an insertion/deletion polymorphism, also referred to herein as an "indel". The term "indel" refers to an insertion or deletion, wherein one line may be referred to as having an inserted nucleotide or piece of DNA relative to a second line, or the second line may be referred to as having a deleted nucleotide or piece of DNA relative to the first line.

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used. The terms "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (Bl2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the Bl2seq program can be found in the readme file accompanying BLASTZ. Bl2seq performs a comparison between two sequences using either the BLASTN or BLASTP algorithm.

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g. , C:\seq I .txt); -j is set to a file containing the second nucleic acid sequence to be compared (e.g. , C:\seq2.txt); -p is set to blastn; -o is set to any desired file name (e.g. , C :\output.txt); -q is set to - 1 ; -r is set to 2; and all other options are left at their default setting. The following command will generate an output file containing a comparison between two sequences: C:\B12seq -i c:\seql .txt -j c:\seq2.txt -p blastn -o c:\output.txt -q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the Bl2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1 , while 78.15, 78.16, 78.17, 78.18, and 78.19 are rounded up to 78.2. It also is noted that the length value will always be an integer.

An "isolated nucleic acid" is understood to be a nucleic acid isolated from its natural or original environment. The term also includes a synthetic manufactured nucleic acid. An "isolated nucleic acid sequence" or "isolated DNA" refers to a nucleic acid sequence which is no longer in the natural environment from which it was isolated, e.g. the nucleic acid sequence in a bacterial host cell or in the plant nuclear or plastid genome. When referring to a "sequence" herein, it is understood that the molecule having such a sequence is referred to, e.g. the nucleic acid molecule. A "host cell" or a "recombinant host cell" or "transformed cell" are terms referring to a new individual cell (or organism) arising as a result of at least one nucleic acid molecule, having been introduced into said cell. The host cell is preferably a plant cell or a bacterial cell. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

When reference is made to a nucleic acid sequence (e.g. DNA or genomic DNA) having "substantial sequence identity to" a reference sequence or having a sequence identity of at least 80%>, e.g. at least 85%, 90%, 95%, 98%> or 99%> nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

In certain embodiments, if the (protein and/or mRNA) expression level of the PCH2, in particular the wild type or native PCH2, is reduced or expression is (substantially) absent or eliminated, then the plant or plant part has altered crossover frequency, crossover distribution, crossover interference, linkage drag, selective sweep, and/or genetic variation. In certain embodiments, if the (protein and/or mRNA) expression level of the PCH2, in particular the wild type or native PCH2, is reduced or expression is (substantially) absent or eliminated compared to a reference expression level, then the plant or plant part has altered crossover frequency, crossover distribution, crossover interference, linkage drag, selective sweep, and/or genetic variation. In certain embodiments, if the (protein and/or mRNA) expression level of the PCH2, in particular the wild type or native PCH2, is reduced or expression is (substantially) absent or eliminated compared to the reference expression level in a reference plant or plant part, then the plant or plant part has altered crossover frequency, crossover distribution, crossover interference, linkage drag, selective sweep, and/or genetic variation.

As used herein, reduced (protein and/or mRNA) expression levels may refer to decreased expression levels of about at least 10%, preferably at least 30%, more preferably at least 50%, such as at least 20%, 40%, 60%, 80% or more, such as at least 85%, at least 90%, at least 95%, or more. Expression is (substantially) absent or eliminated if expression levels are reduced at least 80%, preferably at least 90%, more preferably at least 95%. In certain embodiments, expression is (substantially) absent, if no protein and/or mRNA, in particular the wild type or native protein and/or mRNA, can be detected. Expression levels can be determined by any means known in the art, such as by standard detection methods, including for instance (quantitative) PCR, northern blot, western blot, ELISA, etc.

Reduced expression levels may result from increased turnover of mRNA or protein, such as increased breakdown or decreased stability. Reduced expression levels may result from reduced expression rate or reduced transcription rate. Reduced expression levels may result from reduced copy number (e.g. heterozygous wild type and mutant Pch2).

As used herein, reduced expression rate may refer to a reduction in the expression rate of a nucleotide sequence by more than 10%, 15%, 20%, 25% or 30%, preferably by more than 40%, 45%, 50%, 55%, 60% or 65%, more preferably by more than 70%, 75%, 80%, 85%, 90%, 92%, 94%, 96% or 98% in comparison to the specified reference, such as a plant not comprising the genetic or otherwise modifications according to the invention as described herein elsewhere, or a reference plant, such as a wild type plant. However, it may also mean that the expression rate of a nucleotide sequence is reduced by 100%. The reduction in the expression rate preferably leads to a change of the phenotype of a plant in which the expression rate is reduced.

As used herein, reduced transcription rate may refer to a reduction in the transcription rate of a nucleotide sequence by more than 10%, 15%, 20%, 25% or 30%, preferably by more than 40%, 45%, 50%, 55%, 60% or 65%, more preferably by more than 70%, 75%, 80%, 85%, 90%, 92%, 94%, 96% or 98% in comparison to the specified reference, such as a plant not comprising the genetic or otherwise modifications according to the invention as described herein elsewhere, or a reference plant, such as a wild type plant. However, it may also mean that the transcription rate of a nucleotide sequence is reduced by 100%. The reduction in the transcription rate preferably leads to a change of the phenotype of a plant in which the transcription rate is reduced.

As used herein, reduced (protein) activity may refer to decreased activity of about at least 10%, preferably at least 30%, more preferably at least 50%, such as at least 20%, 40%, 60%, 80% or more, such as at least 85%, at least 90%, at least 95%, or more. Activity is (substantially) absent or eliminated if activity is reduced at least 80%, preferably at least 90%, more preferably at least 95%. In certain embodiments, activity is (substantially) absent, if no activity, in particular the wild type or native protein activity, can be detected. (Protein) activity levels can be determined by any means known in the art, depending on the type of protein, such as by standard detection methods, including for instance enzymatic assays (for enzymes), transcription assays (for transcription factors), assays to analyse a phenotypic output, etc.

As used herein, reduced stability may refer to reduced protein stability or reduced RNA, such as mRNA stability. Stability of proteins or RNA can be determined by means known in the art, such as determination of protein/RNA half-life. Reduced protein or RNA stability in certain embodiments means a reduction of stability of about at least 10%, preferably at least 30%, more preferably at least 50%, such as at least 20%, 40%, 60%, 80% or more, such as at least 85%, at least 90%, at least 95. Reduced protein or RNA stability in certain embodiments means a reduction in half-life of about at least 10%, preferably at least 30%, more preferably at least 50%, such as at least 20%, 40%, 60%, 80% or more, such as at least 85%, at least 90%, at least 95%, such as a twofold, three-fold, four-fold, 5-fold or more decrease in half-life. Stability may be compared to a reference as defined above.

Expression levels, stability, or activity may be compared between different plants (or plant parts), such as a plant (part) having reduced Pch2 expression, stability, or activity according to the invention and a reference plant (part), such as a wild type plant (part). Expression levels, stability, or activity may be compared between different conditions. Expression levels, stability, or activity may be compared with a predetermined threshold. Such predetermined threshold may for instance correspond to expression levels, stability, or activity in a particular genotype or under particular conditions.

Reduction of expression, activity, and/or stability as described herein may be constitutive or conditional, such as inducible and/or tissue-specific (e.g. in reproductive organs). Means for conditional, such as inducible or tissue-specific manipulation are well known in the art.

In an aspect, the invention relates to identification of a plant according to the invention, as described herein elsewhere, such as a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination. Such method may comprise detecting the expression level, stability, and/or activity of Pch2 in a plant or plant part, and may include isolating genetic material from at least one cell of the plant or plant part.

Methods for screening for the presence of a Pch2 gene having reduced expression, activity, or stability as described herein are known in the art. Without limitation, screening may encompass or comprise sequencing, hybridization based methods (such as (dynamic) allele-specific hybridization, molecular beacons, SNP microarrays), enzyme based methods (such as PCR, KASP (Kompetitive Allele Specific PCR), RFLP, ALFP, RAPD, Flap endonuclease, primer extension, 5'-nuclease, oligonucleotide ligation assay), post-amplification methods based on physical properties of DNA (such as single strand conformation polymorphism, temperature gradient gel electrophoresis, denaturing high performance liquid chromatography, high-resolution melting of the entire amplicon, use of DNA mismatch-binding proteins, SNPlex, surveyor nuclease assay), etc.

In an aspect, the invention relates to a method, such as a method for generating a plant having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination; or a method for reducing Pch2 expression, stability, and/or activity, altering linkage drag or linkage sweep, altering crossover frequency, distribution, and/or interference, generating a hyper-Rec phenotype, or altering recombination frequency, such as altered homologous and/or homoeologous recombination; comprising introducing into the genome of a plant a Pch2 having a mutation.

In an aspect, the invention relates to a method, such as a method for generating a plant having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination; or a method for reducing Pch2 expression, stability, and/or activity, altering linkage drag or linkage sweep, altering crossover frequency, distribution, and/or interference, generating a hyper-Rec phenotype, or altering recombination frequency, such as altered homologous and/or homoeologous recombination; comprising introducing into the genome of a plant a mutation in a Pch2.

In an aspect, the invention relates to a method, such as a method for generating a plant having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination; or a method for reducing Pch2 expression, stability, and/or activity, altering linkage drag or linkage sweep, altering crossover frequency, distribution, and/or interference, generating a hyper-Rec phenotype, or altering recombination frequency, such as altered homologous and/or homoeologous recombination; comprising knocking out a Pch2.

In an aspect, the invention relates to a method, such as a method for generating a plant having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination; or a method for reducing Pch2 expression, stability, and/or activity, altering linkage drag or linkage sweep, altering crossover frequency, distribution, and/or interference, generating a hyper-Rec phenotype, or altering recombination frequency, such as altered homologous and/or homoeologous recombination; comprising knocking down protein and/or mRNA expression of a Pch2.

In an aspect, the invention relates to a method, such as a method for generating a plant having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination; or a method for reducing Pch2 expression, stability, and/or activity, altering linkage drag or linkage sweep, altering crossover frequency, distribution, and/or interference, generating a hyper-Rec phenotype, or altering recombination frequency, such as altered homologous and/or homoeologous recombination; comprising eliminating or reducing protein and/or mRNA expression of a Pch2.

In an aspect, the invention relates to a plant or plant part comprising a nucleotide sequence of a gene encoding a Pachytene checkpoint protein 2 homolog (PCH2) having a mutation.

Knockdown or knockout of PCH2 may be effected by any of the mutagenesis methods described herein.

In an aspect, the invention relates to a plant or plant part comprising a gene encoding a Pch2 having a mutation or other genetic event, preferably a mutation or other genetic event leading to a knock-out or knock-down of said gene, having reduced or eliminated mRNA and/or protein expression of an Pch2 gene, having reduced or eliminated protein activity of a Pch2 gene and/or having reduced stability of a PCH2 protein.

In certain embodiments, the plant is not a plant variety.

In an aspect, the invention relates to a method for obtaining or generating a plant or plant part, such as a wheat or barley plant or plant part, comprising (a) providing a first plant having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination, as described herein elsewhere, (b) crossing said first plant with a second plant, such as a second plant not having reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination, (c) selecting progeny plants having said reduced Pch2 expression, activity, and/or stability or generating a plant having reduced Pch2 expression, stability, and/or activity, a plant having altered linkage drag or linkage sweep, a plant having altered crossover frequency, distribution, and/or interference, a plant having a hyper-Rec phenotype, or a plant having altered recombination frequency, such as altered homologous and/or homoeologous recombination, and optionally (d) harvesting said plant part from said progeny.

In an aspect, the invention relates to a method for obtaining or generating a plant or plant part, such as a wheat or barley plant or plant part, comprising (a) providing a first plant having a mutated Pch2 gene or a first plant in which mRNA and/or protein expression, activity, and/or stability of a Pch2 gene is reduced or (substantially) eliminated or absent, such as described herein elsewhere, (b) crossing said first plant with a second plant, such as a second plant not having said mutated Pch2 gene, (c) selecting progeny plants having said mutated Pch2 gene or in which mRNA and/or protein expression, activity, and/or stability of a Pch2 gene is reduced or (substantially) eliminated or absent, and optionally (d) harvesting said plant part from said progeny.

In certain embodiments, Pch2 mutation (or reduced expression, activity, or stability) in the first plant is present in a homozygous state. In certain embodiments the Pch2 mutation (or reduced expression, activity, or stability) in the first plant is present in a heterozygous state. In certain embodiments, the Pch2 mutation (or reduced expression, activity, or stability) in the second plant is present in a heterozygous state. In certain embodiments the Pch2 mutation (or reduced expression, activity, or stability) in the second plant is not present.

In certain embodiments, the progeny is selected in which the mutated Pch2 (or Pch2 having reduced expression, activity, or stability) is present in a homozygous state. In certain embodiments, the progeny is selected in which the mutated Pch2 (or Pch2 having reduced expression, activity, or stability) is present in a heterozygous state.

In certain embodiments, the plant is or plant part is from wheat.

In certain embodiments, the plant is or the plant part is from barley.

In certain embodiments, the methods for obtaining plants or plant parts as described herein according to the invention, such as the methods for obtaining plants or plant parts having altered linkage drag, selective sweep, crossover frequency, crossover distribution, and/or crossover interference, involve or comprise transgenesis and/or gene editing, such as including CRISPR/Cas, TALEN, ZFN, meganucleases; (induced) mutagenesis, which may or may not be random mutagenesis, such as TILLING. In certain embodiments, the methods for obtaining plants or plant parts as described herein according to the invention, such as the methods for obtaining plants or plant parts having altered linkage drag, selective sweep, crossover frequency, crossover distribution, and/or crossover interference, involve or comprise RNAi applications, which may or may not be, comprise, or involve transgenic applications. By means of example, non-transgenic applications may for instance involve applying RNAi components such as double stranded siRNAs to plants or plant surfaces, such as for instance as a spray. Stable integration into the plant genome is not required.

In certain embodiments, the methods for obtaining plants or plant parts as described herein according to the invention, such as the methods for obtaining plants or plant parts having altered linkage drag, selective sweep, crossover frequency, crossover distribution, and/or crossover interference, do not involve or comprise transgenesis, gene editing, and/or mutagenesis.

In certain embodiments, the methods for obtaining plants or plant parts as described herein according to the invention, such as the methods for obtaining plants or plant parts having altered linkage drag, selective sweep, crossover frequency, crossover distribution, and/or crossover interference, involve, comprise or consist of breeding and selection.

In certain embodiments, the methods for obtaining plants or plant parts as described herein according to the invention, such as the methods for obtaining plants or plant parts having altered linkage drag, selective sweep, crossover frequency, crossover distribution, and/or crossover interference, do not involve, comprise or consist of breeding and selection.

In an aspect, the invention relates to a plant or plant part obtained or obtainable by the methods of the invention as described herein, such as the methods for obtaining plants or plant parts having altered linkage drag, selective sweep, crossover frequency, crossover distribution, and/or crossover interference.

In certain embodiments, the wild type or unmutated Pch2 gene comprises
(i) a nucleotide sequence comprising the sequence of SEQ ID NO: 31, 34, 37 or 40;
(ii) a nucleotide sequence having the cDNA or coding sequence of SEQ ID NO: 32, 35, 38, or 41;
(iii) a nucleotide sequence encoding for a polypeptide having the amino acid sequence of SEQ ID NO: 33, 36, 39, or 42;
(iv) a nucleotide sequence having at least 60% identity to the sequence of SEQ ID NO: 31, 34, 37 or 40, such as at least 65%, 70%, 75%, 80%, 85%, 90%, 95% or more sequence identity, preferably at least 85% sequence identity, more preferably at least 90% sequence identity or at least 95% sequence identity; or having a cDNA at least 60% identical to one of the sequences selected from the group consisting of SEQ ID NO: 32, 35, 38, or 41, such as at least 65%, 70%, 75%, 80%, 85%, 90%, 95% or more sequence identity, preferably at least 85% sequence identity, more preferably at least 90% sequence identity or at least 95% sequence identity;
(v) a nucleotide sequence encoding for a polypeptide having at least 60% identity to the sequence of SEQ ID NO: 33, 36, 39, or 42; such as at least 65%, 70%, 75%, 80%, 85%, 90%, 95% or more sequence identity, preferably at least 85% sequence identity, more preferably at least 90% sequence identity or at least 95% sequence identity;
(vi) a nucleotide sequence hybridizing with the reverse complement of a nucleotide sequence as defined in (i), (ii) or (iii) under stringent hybridization conditions; and
(vii) a nucleotide sequence encoding a protein derived from the polypeptide encoded by the nucleotide sequence of any of (i) to (vi) by way of substitution, deletion and/or addition of one or more amino acid(s).

In certain embodiments, the wild type or unmutated Pch2 gene comprises
(i) a nucleotide sequence comprising the sequence of SEQ ID NO: 31, 34, 37 or 40;
(ii) a nucleotide sequence having the cDNA or coding sequence of SEQ ID NO: 32, 35, 38, or 41;
(iii) a nucleotide sequence encoding for a polypeptide having the amino acid sequence of SEQ ID NO: 33, 36, 39, or 42;

In certain embodiments, the wild type or unmutated Pch2 gene comprises
(i) a nucleotide sequence having at least 60% identity to the sequence of SEQ ID NO: 31, 32, 34, 35, 37, 38, 40, or 41; such as at least 65%, 70%, 75%, 80%, 85%, 90%, 95% or more sequence identity, preferably at least 85% sequence identity, more preferably at least 90% sequence identity or at least 95% sequence identity; or
(ii) a nucleotide sequence encoding for a polypeptide having at least 60% identity to the sequence of SEQ ID NO: 33, 36, 39, or 42; such as at least 65%, 70%, 75%, 80%, 85%, 90%, 95% or more sequence identity, preferably at least 85% sequence identity, more preferably at least 90% sequence identity or at least 95% sequence identity.

In certain embodiments, the wild type or unmutated Pch2 gene comprises
(i) a nucleotide sequence having at least 60% identity to the sequence of SEQ ID NO: 32, 35, 38, or 41; such as at least 65%, 70%, 75%, 80%, 85%, 90%, 95% or more sequence identity, preferably at least 85% sequence identity, more preferably at least 90% sequence identity or at least 95% sequence identity; or
(ii) a nucleotide sequence encoding for a polypeptide having at least 60% identity to the sequence of SEQ ID NO: 33, 36, 39, or 42; such as at least 65%, 70%, 75%, 80%, 85%, 90%, 95% or more sequence identity, preferably at least 85% sequence identity, more preferably at least 90% sequence identity or at least 95% sequence identity.

In certain embodiments, the wild type or unmutated Pch2 gene comprises
(i) a nucleotide sequence comprising the sequence of SEQ ID NO: 31, 34, 37 or 40;
(ii) a nucleotide sequence having the cDNA or coding sequence of SEQ ID NO: 32, 35, 38, or 41;
(iii) a nucleotide sequence encoding for a polypeptide having the amino acid sequence of SEQ ID NO: 33, 36, 39, or 42;
(iv) a nucleotide sequence having at least 60% identity to the sequence of SEQ ID NO: 31, 34, 37 or 40, such as at least 65%, 70%, 75%, 80%, 85%, 90%, 95% or more sequence identity, preferably at least 85% sequence identity, more preferably at least 90% sequence identity or at least 95% sequence identity; or having a cDNA at least 60% identical to one of the sequences selected from the group consisting of SEQ ID NO: 32, 35, 38, or 41, such as at least 65%, 70%, 75%, 80%, 85%, 90%, 95% or more sequence identity, preferably at least 85% sequence identity, more preferably at least 90% sequence identity or at least 95% sequence identity;
(v) a nucleotide sequence encoding for a polypeptide having at least 60% identity to the sequence of SEQ ID NO: 33, 36, 39, or 42; such as at least 65%, 70%, 75%, 80%, 85%, 90%, 95% or more sequence identity, preferably at least 85% sequence identity, more preferably at least 90% sequence identity or at least 95% sequence identity.

In certain embodiments, the wild type or Pch2 gene comprises
(i) a nucleotide sequence hybridizing with the reverse complement of a nucleotide sequence of any of SEQ ID NO: 31, 34, 37 or 40 or SEQ ID NO: 32, 35, 38, or 41 under stringent hybridization conditions.

In certain embodiments, the wild type or unmutated Pch2 gene comprises
(i) a nucleotide sequence encoding a protein derived from the polypeptide encoded by the nucleotide sequence of any of SEQ ID NO: 31, 34, 37 or 40 or SEQ ID NO: 32, 35, 38, or 41 by way of substitution, deletion and/or addition of one or more amino acid(s)

The skilled person will understand that the wild type or unmutated Pch2 gene product is a functional gene product having enzymatic activity, as defined herein elsewhere. The skilled person will further understand that sequence variations described above for the wild type Pch2 do not include frame shift or nonsense mutations.

As used herein, the mutated Pch2 or the mutation in the Pch2 may comprise or may refer to any type of Pch2 mutation. In certain embodiments the mutation alters expression of the wild type or native PCH2 protein and/or mRNA. In certain embodiments the mutation reduces or eliminates expression of the (wild type or native) PCH2 protein and/or mRNA, as described herein elsewhere. Mutations may affect transcription and/or translation. Mutations may occur in exons or introns. Mutations may occur in regulatory elements, such as promotors, enhancers, terminators, insulators, etc. Mutations may occur in coding sequences. Mutations may occur in splicing signal sites, such as splice donor or splice acceptor sites. Mutations may be frame shift mutations. Mutations may be nonsense mutations. Mutations may be point mutations. Mutations may be insertion or deletion of one or more nucleotides, internally and/or terminally. Mutations may be non-conservative mutations (in which one or more wild type amino acids are replaced with one or more non-wild type amino acids). Mutations may result in a truncated protein. Mutations may affect or alter the function of the PCH2 protein, such as enzymatic activity. Mutations may reduce or (substantially) eliminate the function of the PCH2 protein, such as enzymatic activity. Reduced function, such as reduced enzymatic activity, may refer to a reduction of about at least 10%, preferably at least 30%, more preferably at least 50%, such as at least 20%, 40%, 60%, 80% or more, such as at least 85%, at least 90%, at least 95%, or more. (Substantially) eliminated function, such as (substantially) eliminated enzymatic activity, may refer to a reduction of at least 80%, preferably at least 90%, more preferably at least 95%. Mutations may be dominant negative mutations.

As used herein, the term "Pch2 activity" may refer to the enzymatic activity of Pch2 or non-enzymatic activity of Pch2. The term "having reduced Pch2 activity" in the context of variant Pch2 as described above in certain preferred embodiments refers to a Pch2 of which the enzymatic or non-enzymatic activity is affected, in particular reduced compared to wild type or native Pch2. In certain embodiments, the (enzymatic) activity is at most 50% of the wild type Pch2 activity, preferably at most 40%, more preferably at most 30%, even more preferably at most 20%, most preferably at most 10%, such as at most 5%. (Enzymatic) activity can be measured by means known in the art.

In certain embodiments, the Pch2 mutation is an insertion of one or more nucleotides in the coding sequence. In certain embodiments, the Pch2 mutation is a nonsense mutation. In certain embodiments, the Pch2 mutation results in reduced expression of the Pch2 gene. In certain embodiments, the Pch2 mutation results in knockout of the Pch2 gene or knockdown of the Pch2 mRNA and/or protein. In certain embodiments, the mutation results in a frame shift of the coding sequence of Pch2. In certain embodiments, the mutation results in an altered protein sequence encoded by the Pch2 gene.

In certain embodiments, the Pch2 mutation is an insertion, preferably in an exon, preferably an insertion in the first exon, of one or more nucleotides, preferably a frame shift insertion.

Pch2 mRNA and/or protein expression may be reduced or eliminated by mutating the Pch2 gene itself (including coding, non-coding, and regulatory element). Methods for introducing mutations are described herein elsewhere. Alternatively, Pch2 mRNA and/or protein expression may be reduced or eliminated by (specifically) interfering with transcription and/or translation, such as to decrease or eliminate mRNA and/or protein transcription or translation. Alternatively, Pch2 mRNA and/or protein expression may be reduced or eliminated by (specifically) interfering with mRNA and/or protein stability, such as to reduce mRNA and/or protein stability. By means of example, mRNA (stability) may be reduced by means of RNAi, as described herein elsewhere. Also miRNA can be used to affect mRNA (stability). In certain embodiments, a reduced Pch2 expression which is achieved by reducing mRNA or protein stability is also encompassed by the term "mutated" Pch2. In certain embodiments, a reduced Pch2 expression which is achieved by reducing mRNA or protein stability is not encompassed by the term "mutated" Pch2.

In an aspect, the invention relates to a (isolated) polynucleic acid comprising a mutated Pch2 genomic, mRNA, or cDNA sequence or fragment thereof of the invention, or the complement or the reverse complement of mutated Pch2 of the invention. In certain embodiments, the invention relates to a polynucleic acid comprising at least 10 contiguous nucleotides, preferably at least 15 contiguous nucleotides or at least 20 contiguous nucleotides of a mutated Pch2 of the invention, or the complement or the reverse complement of a (molecular) marker allele of the invention. In certain embodiments, the invention relates to a polynucleic acid comprising at least 10 contiguous nucleotides, preferably at least 15 contiguous nucleotides or at least 20 contiguous nucleotides of any of SEQ ID NOs: 14, 15, 16, 17, 18, or 19, or the complement or the reverse complement of any of SEQ ID NOs: 14, 15, 16, 17, 18, or 19,. In certain embodiments, the polynucleic acid is capable of discriminating between a mutated Pch2 and a wild type Pch2, such as to specifically hybridise with a mutated Pch2 of the invention. In certain embodiments, the polynucleic acid is capable of hybridising with a unique nucleotide fragment or section of any of SEQ ID NOs: 14, 15, 16, 17, 18, or 19, or the complement or the reverse complement of any of SEQ ID NOs: 14, 15, 16, 17, 18, or 19.

In certain embodiments, the invention relates to a plant (part) comprising a polynucleic acid selected form SEQ ID NOs: 14, 15, 16, 17, 18, or 19. Preferably the plant is a barley plant (part).

In an aspect, the invention relates to a polynucleic acid capable of specifically hybridizing with or specifically detecting a mutated Pch2 of the invention, or the complement thereof, or the reverse complement thereof.

In certain embodiments, the invention relates to a polynucleic acid specifically hybridising with any of the sequences of SEQ ID NOs: 14, 15, 16, 17, 18, or 19, or the complement or the reverse complement thereof.

In certain embodiments, the polynucleic acid is a primer. In certain embodiments, the polynucleic acid is a probe.

In certain embodiments, the polynucleic acid is an allele specific polynucleic acid, such as an allele specific primer or probe.

In certain embodiments, the polynucleic acid comprises at least 15 nucleotides, such as 16, 17, 18, 19, 20, 21, 22, 23, 24, or 25 nucleotides, such as at least 30, 35, 40, 45, or 50 nucleotides, such as at least 100, 200, 300, or 500 nucleotides.

When reference is made to a nucleic acid sequence (e.g. DNA or genomic DNA) having "substantial sequence identity to" a reference sequence or having a sequence identity of at least 80%>, e.g. at least 85%, 90%, 95%, 98%> or 99%> nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

The term "hybridizing" or "hybridization" means a process in which a single-stranded nucleic acid molecule attaches itself to a complementary nucleic acid strand, i.e. agrees with this base pairing. Standard procedures for hybridization are described, for example, in Sambrook et al. (Molecular Cloning. A Laboratory Manual, Cold Spring Harbor Laboratory Press, 3rd edition 2001). Preferably this will be understood to mean an at least 50%, more preferably at least 55%, 60%, 65%, 70%, 75%, 80% or 85%, more preferably 90%, 91%, 92%, 93%, 94%, 95%, 96%, 97%, 98% or 99% of the bases of the nucleic acid strand form base pairs with the complementary nucleic acid strand. The possibility of such binding depends on the stringency of the hybridization conditions. The term "stringency" refers to hybridization conditions. High stringency is if base pairing is more difficult, low stringency, when a base-pairing is facilitated. The stringency of hybridization conditions depends for example on the salt concentration or ionic strength and temperature. Generally, the stringency can be increased by increasing the temperature and / or decreasing salinity. "Stringent hybridization conditions" are defined as conditions in which hybridization occurs predominantly only between homologous nucleic acid molecules. The term "hybridization conditions" refers not only to the actual binding of the nucleic acids at the prevailing conditions, but also in the subsequent washing steps prevailing conditions. Stringent hybridization conditions are, for example, conditions under which predominantly only those nucleic acid molecules having at least 70%, preferably at least 75%, at least 80%, at least 85%, at least 90% or at least 95% sequence identity hybridize. Less stringent hybridization conditions include: hybridization in 4 x SSC at 37 ° C, followed by repeated washing in 1 x SSC at room temperature. Stringent hybridization conditions include: hybridization in 4 x SSC at 65 ° C, followed by repeated washing in 0.1 x SSC at 65 ° C for a total of about 1 hour.

It will be understood that "specifically hybridizing" means that the polynucleic acid hybridises with the (molecular) marker allele (such as under stringent hybridisation conditions, as defined herein elsewhere), but does not (substantially) hybridise with a polynucleic acid not comprising the marker allele or is (substantially) incapable of being used as a PCR primer. By means of example, in a suitable readout, the hybridization signal with the marker allele or PCR amplification of the marker allele is at least 5 times, preferably at least 10 times stronger or more than the hybridisation signal with a non-marker allele, or any other sequence.

In an aspect, the invention relates to a kit comprising such polynucleic acids, such as primers (comprising forward and/or reverse primers) and/or probes. The kit may further comprise instructions for use.

In will be understood that in embodiments relating to a set of forward and reverse primers, only one of both primers (forward or reverse) may need to be capable of discriminating between a (molecular) marker allele of the invention and a non-marker allele, and hence may be unique. The other primer may or may not be capable of discriminating between a (molecular) marker allele of the invention and a non-marker allele, and hence may be unique.

The aspects and embodiments of the invention are further supported by the following non-limiting examples. The following examples, including the experiments conducted and the results achieved, are provided for illustrative purposes only and are not constructed as limiting the present invention.

### EXAMPLES

### EXAMPLE 1: Golden Promise CRISPR/ Cas9 barley plants

### Materials and methods

### Genomic DNA (gDNA) extraction

gDNA was extracted from leaf material, using the following protocol:
Extraction Buffer: 100mM Tris pH 9.5, 10mM EDTA, 250mM KCI
Dilution Buffer: 3% Bovine Serum Albumin (diluted in sterile water)

Leaf discs were collected in 1.5ml microfuge tubes and samples were immediately placed on ice. 100µl of Extraction Buffer was added to the sample and then a sterile pellet pestle was used to grind up tissue. The sample was heated to 95oC for 10 minutes, then returned to ice for two minutes. 100µl Dilution Buffer was added to each tube, briefly mixed and then 1 µl was directly used in the PCR reaction.

### PCR- based methods

Standard Taq polymerase reactions were used for PCRs (using MyTaq Red Mix (Bioline)).

### Cytological methods

To prepare samples for both immunolocalisation and meiotic metaphase I spreads, the protocols described by Higgins (Plant Meiosis, Methods and Protocols, 2013, eds. Pawlowski et al.) were followed.

As this work was done in the barley cv. Golden Promise, it was necessary to obtain PCH2 sequence data through cloning and sequencing as the genomic sequence for Golden Promise had not yet been published. Genomic DNA was extracted from leaf material and primer pairs (SEQ ID NOs: 4 and 5) (including nested primers (SEQ ID NOs: 6-9)) were used to amplify PCH2, based on known cDNA sequence information from the barley cv. Optic (SEQ ID NO: 1).

Initial primers used to obtain gDNA PCH2 sequence (designed from cv. Optic)
D15 forward: CCATGGAGATCTCCTTCGACC (SEQ ID NO: 4)
D16 reverse: CAGCCGCGTGATTCCAAGAGC (SEQ ID NO: 5)

These primers amplify entire PCH2 gene but nested primers were required to gain accurate sequencing information.

Nested primers used (reverse primers used with D15 forward):
D18 reverse: CAGCAACCTCTGCTTGAGACC (SEQ ID NO: 6)
D30 reverse: GGTCTGTATCAGCGTGTGC (SEQ ID NO: 7)
D32 reverse: GTCACATATTTGGATCCT (SEQ ID NO: 8)

D31 forward also used (present in an intron hence not shown on below cDNA): ACAGCTATAAGCCAATC (SEQ ID NO: 9)

This allowed the overlay of sequencing results to identify the genomic sequence for ATG -2→2045bps of PCH2 in Golden Promise (SEQ ID NO: 3). The intron-exon boundaries were then identified using known sequence information from cv. Optic PCH2 cDNA (SEQ ID NO: 1). From this sequence information, four guide RNAs (two pairs) were designed to target exon 1 (SEQ ID NOs: 10-13), for knocking out PCH2 function in barley. While this sequence did not contain the conserved Walker A and B domains, nor the AAA+ ATPase region, a single bp change causing a frameshift would occur upstream of the conserved domains and hence result in a loss of PCH2 protein function. Sequences for two gRNA pairs were used for CRISPR/Cas9 transformations (sequences shown below). Mutant lines have been produced and used for genotyping and phenotypic analysis.

### Guide RNA sequences

1967: TGGAGATCTCCTTCGACCCC (SEQ ID NO: 10) and GCCCACCCCGCCAGACGACA (SEQ ID NO: 11)
1968: TCTCATCCCGCCCTGCCCGC (SEQ ID NO: 12) and GCTGCTGCACGCCTCGAGTA (SEQ ID NO: 13)

The 1967 guides were used to generate mutant lines 1, 2 and 3.

A 500bp amplicon spanning all four of the guide RNAs was established as a reliable PCR screening method (primers: SEQ ID NO: 24 and 25), to give clear chromatograms of DNA sequences over the target region when the primary transgenic plants were screened for mutations.

Primers used for genotyping (F2, R3) from genomic DNA
F2: TTCCATTCCGCTGCCTCG (SEQ ID NO: 24)
R3: GCATCCACGTGTCATCAAG (SEQ ID NO: 25)

While the majority of plants were found to be identical to the wild-type (WT) sequence, four mutant lines were established:
Line 1 (1967-08) was found to have a 152bp deletion between the two gRNAs in one copy of PCH2, along with a 1bp (G) insertion between the cut sites. This resulted in an overall 152bp deletion, causing a frameshift which introduced a premature stop codon towards the N-terminus of the protein (upstream of the functional domains) (SEQ ID NOs: 14 and 15).

Line 1 (1967-08) showing 152bp deletion (nucleotides from position 17 to 168 of SEQ ID NO: 1). Sequence fragment of PCH2 with the deletion:

TTATTCCGCTGCCTCGCGGAGCCTGGCCACCATGAGCGCCCCCATGGACAAGG
TCCTCGTGTCAGGTAGGCGCCGCGACGCGCACGCGTTTCCGGCCTCTTCCTAGC
GCCGCATGACTAAACCGTGCTCTGCGAATGCCCGATTTGGTTTCGCCGGGGTTGA
TTTTTTCCTTTTGTGCTTGCAGTTGAGGTGCTGCTGCACGCCTCGAGTACGGCTC
GCGCCGAGA (SEQ ID NO: 14)

Cut between the two gRNAs has occurred (TGG from forward (italic; (nucleotides 1-3 of SEQ ID NO: 10)) and GACA from reverse (bold; (nucleotides 17-20 of SEQ ID NO: 11)) remain but the rest of the gRNAs and all sequence between has been removed.

The other copy of PCH2 had two 3bp deletions and one base pair change, however this was deemed to have no effect on the function of the protein due to the changes being present upstream of the functional domains and also not resulting in a frameshift. These heterozygous pch2 plants were selfed and seeds were sown for genotyping.

Line 2 (1967-04) also has a 153bp deletion between the two gRNAs, along with a 1bp (A) insertion. This also causes a frameshift resulting in a premature stop codon, and as the bp insertions are different between the two lines, the amino acid sequences become different.

Line 2 (1967-04) and Line 3 (1967-05) both contain point mutations which result in a premature stop codon (seen in sequences section) (Line 2= SEQ ID NOs: 16 and 17; Line 3= SEQ ID NOs: 18 and 19).

Line 1 has been used for the main phenotypic analysis and Lines 2 and 3 to confirm this phenotype. A PCR- screen was set up to genotype plants from the next generation of the heterozygous plant (from Line 1). This screen was used to successfully distinguish between WT, heterozygous and mutant plants (by observing different band sizes on an agarose gel following PCR amplification). The heterozygous plants have been maintained as a seed stock and WT and homozygous mutant plants have been used in phenotypic analysis.

Primers used for genotyping (mutant and WT primer pairs; Line 2)
Common sequencing forward primer used: AGTTCAATCCGAACAGCACCTAC (SEQ ID NO: 26)
WT reverse primer: TGAGCGCCCCCATGGAGA (SEQ ID NO: 27)
Line 2 mutant reverse primer: ATGAGGGCCCCCAGGGAA (SEQ ID NO: 28)

An additional PCR-screen was also set up to identify plants which were free of the Cas9 T-DNA. Cas9-free plants would not create any further potential mutations or introduce off-target effects. Primers were designed against the Cas9 plasmid to identify a presence or absence of the Cas9 in the genomic DNA (SEQ ID NOs: 20-23). Only T-DNA free plants have been used in phenotypic analysis.

Primers used for identifying presence of Cas9
HvCas9F1: GCCGTCATTACGGACGAGTAC (SEQ ID NO: 20)
HvCas9R1: AGTCAGCCTTATCAGTACTGTCTAC (SEQ ID NO: 21)
HvCas9F2: GTATCTGGCCAGCCACTATGA (SEQ ID NO: 22)
HvCas9R2: CCGTAATTGACTGATGAATCAGTG (SEQ ID NO: 23)

### Results

Metaphase I (MI) spreads were made from WT and homozygous mutants. In cv. Golden Promise, WT MI chromosomes typically form ring-like structures, containing a minimum of two COs which tend to be distal. In the mutant plants, the occasional rod was present (one CO) and the position of the COs looked to be slightly more interstitial on some chromosomes. To further investigate this observation, quantification of MI chromosomes will be employed using 45S and 5S rDNA FISH probes to distinguish individual chromosomes.

Immunolocalisation has been performed on individuals from Line 1 (WT and pch2 homozygous mutant) to identify whether there is any effect on other protein localisation in the pch2 mutant. ASY1 and ZYP1 antibodies were used to identify any synapsis defects in the mutant. ASY1 is not depleted from the axes in the absence of PCH2. ZYP1 is present in short stretches, but these are spread throughout the cell rather than localised to one area. An MLH3 antibody (that marks CO sites) has been used to investigate CO numbers and positions. In WT cells, MLH3 foci are distributed at distal points on the axes and exhibit strong interference, with foci never positioned closely together. In the pch2 mutant, MLH3 foci have been seen in higher numbers along ZYP1 stretches and are positioned closely together. This indicates that interference has been compromised.

Further immunolocalisation studies revealed that there is a significant delay in synapsis in the pch2 mutants, but it is also seen that cells do eventually achieve full synapsis. The number of double-strand breaks seems to be the same in WT and pch2 mutant cells (quantified by cout yH2AX foci). However, as prophase begins, synapsis takes much longer to progress compared to WT. ZYP1 accumulations begin to form throughout the cell as synapsis progresses. This may suggest that without PCH2, ZYP1 becomes less organised on the axes, hence full synapsis is significantly delayed in the mutant. PCH2 therefore may be required for organising ZYP1 on the chromosome axes, and in the absence of PCH2, disorganised ZYP1 aggregates are seen and synapsis is delayed.

In the pch2 homozygous mutant plants, fertility has not been affected, with plants producing the same number of seeds as WT.

### Conclusion

The cytological analysis of the barley Golden Promise CRISPR/Cas9 lines has indicated that by removing PCH2, there is the potential for increasing chiasmata numbers and moving the position of chiasmata to new interstitial regions. This is something of great interest to plant breeders, as it has the potential to access previously inaccessible regions of the barley genome.

### EXAMPLE 2: Wheat Tetraploid Kronos TILLING lines

### Materials and methods

Targeted Induced Local Lesions In Genomes (TILLING) is a gene interference method that was first developed in Arabidopsis thaliana. This is a reverse genetics technique that uses ethyl methane sulfonate (EMS), a chemical mutagen, to generate mutations at random in the genome. EMS mutagenesis causes a high frequency of point mutations and typically produces C/G to T/A mutations. EMS mutations such as missense and the introduction of stop codons causing truncations are considered non-transgenic. Compared to insertional mutagenesis which only results in gene knockouts, TILLING also allows for an array of lines with different mutational severities. Once the mutations have been introduced, DNA screening is then used to identify the positions of these mutations and to predict the effect they will have on protein function. One recently developed method of screening is exome capture, which sequences the exome and compares the sequencing data against a sample of individuals to identify variation. This was used in the generation of the Wheat TILLING Database, which collated data from over 1500 EMS mutagenized individuals, from the tetraploid wheat line Triticum turgidum cv. Kronos (Krasileva et al., 2017). PCH2 mutants in this project are derived from the tetraploid Kronos TILLING mutant database, obtained from the John Innes Centre (JIC).

The Kronos TILLING line crosses that have been used in this analysis are as follows:
PCH2-A K0685 X PCH2-B K1344 (W15-204)
PCH2-A K0685 X PCH2-B K2734 (W15-216)

### PCH2-A K0685

Genome: A genome
Codon change: TGG → TGA
Amino acid change: W → stop
cDNA position: 549
Consequence: stop gained

### PCH2-B K1344

Genome: B genome
Codon change: TGG → TAG
Amino acid change: W → stop
cDNA position: 656
Consequence: stop gained

### PCH2-B K2734

Genome: B genome
Codon change: CAG → TAG
Amino acid change: Q → stop
cDNA position: 295
Consequence: stop gained

Each of these mutants causes a premature stop codon resulting in a non-functional truncated PCH2 protein. Plants were crossed and genotyped at the JIC and sent to Leicester for phenotypic analysis. WT (cDNA= SEQ ID NO: 29; amino acid sequence= SEQ ID NO: 30) and double homozygous pch2 mutant plants were grown and immature spikes were harvested at the boot stage for phenotypic analysis. Again, the same protocol was followed for immunolocalisation and metaphase I chromosome spreads (Higgins, 2013).

### Results

MI spreads reveal a dramatic increase in rod bivalents in the pch2 mutant, compared to the segregating Kronos WT. A decrease in ring bivalents is also observed as well as the presence of univalents in some cells (maximum of three univalents seen in a single cell). Over 80% of cells have at least one univalent present (n=23). Chiasmata have become even more distal in the pch2 mutant, occurring at the very ends of the chromosomes. As well as this distal shift in recombination, interchromosomal connections are seen in the pch2 mutant, indicating a "hyper-rec" phenotype (which has been described to show a high increase in homologous recombination, compared to the expected recombination in WT). This indicates that in the absence of pch2, recombination increases compared to WT, but because of the large number of chromosomes present in tetraploid wheat, homeologous recombination occurs, resulting in the observation of multivalents and chromosome mis-segregation.

pch2 mutants which have one remaining PCH2 functioning B copy (aaBb) have been observed at metaphase I. These mutants show an increase in interstitial/ proximal recombination. This demonstrates shifting recombination positions in wheat.

Immunolocalisation has been performed in the pch2 Kronos TILLING lines. A PCH2 antibody has been generated, showing no PCH2 protein localising to the chromosome axes in the mutants, compared to the WT. This confirms that PCH2 is completely absent in the homozygous mutant lines. In the pch2 mutant cells, ASY1 is present throughout prophase I. This indicates that PCH2 is indeed required to remove ASY1 from the axes and hence in the absence of PCH2, ASY1 is not depleted from the axes during zygotene. ZYP1 is only present in short stretches, which are only present in a specific area of the cell. Full synapsis is not reached in the pch2 mutants.

### Conclusion

The tetraploid Kronos TILLING lines have indicated that in the absence of PCH2 in wheat, chiasmata numbers are greatly decreased and their position moves to extremely distal regions on the chromosome. This can be used to maintain desirable gene blocks in elite wheat varieties. A desirable "hyper-rec" phenotype has been observed in the Kronos TILLING pch2 mutants, which could shift recombination positions in wheat, perhaps with a partial pch2 knockout mutant.

### EXAMPLE 3: Wheat Cadenza Hexaploid TILLING lines

### Materials and methods

The same method as the Kronos Tetraploid TILLING line generation (as described above in Example 2) was employed to generate TILLING lines in the hexaploid bread wheat Cadenza.

The Cadenza TILLING line crosses that have been used in this analysis are as follows:
PCH2-A Cadenza0169 X PCH2-B Cadenza0685 X PCH2-D Cadenza1198 (Lola_005)
PCH2-A Cadenza0169 X PCH2-B Cadenza0202 X PCH2-D Cadenza0156 X PCH2-D Cadenza1198 (Lola_024)
PCH2-A Cadenza0169 X PCH2-B Cadenza0685 X PCH2-D Cadenza1806 (Lola_087)
PCH2-A Cadenza0169 X PCH2-B Cadenza0202 X PCH2-D Cadenza1198 X PCH2-D Cadenza0156 (Lola_042)
PCH2-A Cadenza1436 X PCH2-B Cadenza1522 X PCH2-D Cadenza0676 (Lola_061)
PCH2-A Cadenza0282 X PCH2-B Cadenza0685 X PCH2-D Cadenza1806 (Lola_108)

The mutations used for aforementioned crossings are as follows:

### PCH2-A Cadenza0169

Genome: A genome
Codon change: CAG → TAG
Amino acid change: Q → stop
cDNA position: 448
Consequence: stop gained

### PCH2-A Cadenza 1436

Genome: A genome
Codon change: CTT → TTT
Amino acid change: L → F
cDNA position: 667
Consequence: missense variant

### PCH2-A Cadenza0282

Genome: A genome
Codon change: GGA → AGA
Amino acid change: G → R
cDNA position: 691
Consequence: missense variant

### PCH2-B Cadenza0685

Genome: B genome
Codon change: TGG → TGA
Amino acid change: W → stop
cDNA position: 552
Consequence: stop gained

### PCH2-B Cadenza0202

Genome: B genome
Codon change: TCG → TTG
Amino acid change: S → L
cDNA position: 116
Consequence: missense variant

### PCH2-B Cadenza 1522

Genome: B genome
Codon change: GTT → ATT
Amino acid change: V → I
cDNA position: 902
Consequence: missense variant

### PCH2-D Cadenza1198

Genome: D genome

### PCH2-D Cadenza0156

Genome: D genome

### PCH2-D Cadenza 1806

Genome: D genome
Codon change: ACA → ATA
Amino acid change: T → I
cDNA position: 497
Consequence: missense variant

### PCH2-D Cadenza0676

Genome: D genome
Codon change: CTT → TTT
Amino acid change: L → F
cDNA position: 475
Consequence: missense variant

### Results

MI analysis has been performed on line Lola_087, which are pch2 aaBbdd mutants (contain one functioning B copy of PCH2). Bivalents show interchromosomal connections (which is another example of the introduction of a hyperrec phenotype). While there are some distally positioned rods present and univalents are consistently observed, there are also some seemingly proximal and interstitial COs observed. This mutant shows that the presence of one functioning PCH2 copy is not enough to restore WT function, indicating a dosage effect.

Immunolocalisation has been performed on Cadenza TILLING line Lola_005, which has been shown by KASP genotyping to be complete pch2 knockouts (aabbdd).

Experiments have shown that there is a significant delay in synapsis is the pch2 mutants, which takes much longer than WT to complete prophase I. Initial analysis indicates that full synapsis is not quite achieved (although cells become close to full synapsis), which could cause interchromosomal connections.

### REFERENCES

Bhalla, N. and Dernburg, A.F.. A conserved checkpoint monitors meiotic chromosome synapsis in Caenorhabditis elegans. Science 2005, 310: 1683-1686.
Deshong A.J. et al. A Quality Control Mechanism Coordinates Meiotic Prophase Events to Promote Crossover Assurance. Plos Genet. 2014, 10.
Joyce, E.F. and McKim, K.S. Drosophila PCH2 is required for a pachytene checkpoint that monitors double-strand-break-independent events leading to meiotic crossover formation. Genet. 2009, 181: 39-51.
Lambing, Christophe et al. Arabidopsis PCH2 Mediates Meiotic Chromosome Remodeling and Maturation of Crossovers, PLoS Genet. 2015, 11(7): e1005372.
Li, X. and Schimenti, J. C. Mouse pachytene checkpoint 2 (Trip13) is required for completing meiotic recombination but not Synapsis. Plos Genet. 2007, 3: 1365-1376
Miao, Chunbo et al. CENTRAL REGION COMPONENT1, a Novel Synaptonemal Complex Component, Is Essential for Meiotic Recombination Initiation in Rice, The Plant Cell, August 2013, Vol. 25: 2998-3009.
Roig, I. et al. Mouse TRIP13/PCH2 is required for recombination and normal higher-order chromosome structure during meiosis. PLoS Genet. 2010, 6: e1001062.
San-Segundo, P.A., and Roeder, G.S. Pch2 links chromatin silencing to meiotic checkpoint control. Cell, 1999, 97: 313-324.
Wojtasz L. et al. Mouse HORMAD1 and HORMAD2, Two Conserved Meiotic Chromosomal Proteins, Are Depleted from Synapsed Chromosome Axes with the Help of TRIP13 AAA-ATPase. Plos Genet. 2009, 5.
Zanders, S., and Alani, E. The pch2D mutation in baker's yeast alters meiotic crossover levels and confers a defect in crossover interference. PLoS Genet. 2009, 5: e1000571.

## Claims

1. A method for altering linkage drag or selective sweep in a plant, comprising reducing or eliminating the expression, stability, and/or activity of Pachytene checkpoint protein 2 homolog (PCH2) in a plant, preferably wherein interchromosomal recombination, preferably homologous or homeologous recombination or is altered or wherein crossover frequency, crossover interference and/or crossover distribution is altered.

2. A method for altering interchromosomal recombination, preferably homologous or homeologous recombination or for altering crossover frequency, crossover interference, and/or crossover distribution in a plant, comprising reducing or eliminating the expression, stability, and/or activity of PCH2 in a plant, preferably wherein linkage drag or selective sweep is altered.

3. The method according to any of claims 1 to 4, wherein said plant is from the family of poaceae, preferably wherein said plant is from the subfamily of pooideae.

4. A method for generating a plant, preferably a plant having a hyper-Rec phonotype, of the subfamily of pooideae comprising reducing or eliminating expression, stability, and/or activity of PCH2.

5. The method according to any of claims 1 to 4, wherein
- linkage drag or selective sweep is reduced;
- crossover frequency is increased;
- crossover distribution is skewed towards the centromere; and/or
- the plant having reduced or eliminated expression, stability, and/or activity of PCH2 exhibits more crossovers towards the centromere compared to a plant not having reduced or eliminated expression, stability, and/or activity of PCH2; and/or
- crossover interference is reduced;
preferably wherein said plant is from the genus Hordeum, preferably from the species Hordeum vulgare.

6. The method according to any of claims 1 to 4, wherein
- linkage drag or selective sweep is increased or maintained;
- crossover frequency is decreased;
- crossover distribution is skewed towards the telomers;
- the plant having reduced or eliminated expression, stability, and/or activity of PCH2 exhibits more crossovers towards the telomere compared to a plant not having reduced or eliminated expression, stability, and/or activity of PCH2; and/or
- crossover interference is increased or maintained;
preferably wherein said plant is from the genus Triticum, preferably from the species Triticum aestivum.

7. The method according to any of claims 1 to 6, wherein said reducing or eliminating comprises knocking out said Pch2 gene or knocking down said PCH2 protein, preferably wherein reducing or eliminating comprises mutagenesis, RNAi, or gene editing.

8. The method according to any of claims 1 to 7, wherein the method comprises introducing or introgressing into the genome of a plant or plant part a nucleotide sequence of a gene encoding PCH2, having a mutation, preferably a mutation leading to reduced or eliminated expression of the mRNA of the gene and/or the PCH2 protein, a mutation leading to a PCH2 protein having reduced or eliminated activity upon translation, or a mutation leading to a PCH2 protein having reduced stability.

9. The method according to any of claims 1 to 8, wherein the method comprises
(a) introducing or introgressing into a plant or plant part into a nucleotide sequence of an endogenous (wild type) gene encoding the PCH2 a mutation, preferably a mutation leading to reduced or eliminated expression of the (endogenous full length) mRNA of the gene and/or the (endogenous full length) PCH2 protein, a mutation leading to an PCH2 protein having reduced activity upon translation, or a mutation leading to an PCH2 protein having reduced stability;
(b) introducing or introgressing into a plant or a plant part an RNAi molecule directed against, targeting, or hybridizing with a nucleotide sequence encoding the PCH2 protein, or a polynucleotide sequence encoding an RNAi molecule directed against, targeting, or hybridizing with a nucleotide sequence encoding the PCH2 protein, or
(c) introducing or introgressing into a plant or a plant part an RNA-specific or DNA-specific CRISPR/Cas system directed against or targeting a nucleotide sequence encoding the PCH2 protein, or one or more polynucleotide sequence(s) encoding said RNA-specific CRISPR/Cas system, or
(d) introducing or introgressing into a plant or a plant part a chemical compound or an antibody altering the activity of the PCH2 protein upon interaction with said PCH2.
(e) optionally, regenerating a plant from the plant part of any of (a) to (d).

10. A plant or plant part thereof of the subfamily of pooideae having reduced or eliminated expression, stability, and/or activity of PCH2, preferably which is selected from the genera Triticum or Hordeum, preferably Triticum aestivum or Hordeum vulgare.

11. A plant or plant part thereof obtainable by the method according to any one of claim 7 to 23, or the progeny thereof having reducing or eliminated expression and/or activity of PCH2, preferably which is selected from the genera Triticum or Hordeum, preferably Triticum aestivum or Hordeum vulgare.

12. The plant or plant part thereof according to claims 10 or 11, which is mutagenized, transgenic or gene-edited.

13. The plant part according to claim 11 or 12, which is a cell, tissue, or seed.

14. The method, plant, or plant part according to any of the preceding claims, wherein the wild type Pch2 gene encoding said PCH2 is selected from the group consisting of:
(i) a nucleotide sequence comprising the sequence of SEQ ID NO: 31, 34, 37 or 40;
(ii) a nucleotide sequence having the cDNA of SEQ ID NO: 32, 35, 38, or 41;
(iii) a nucleotide sequence encoding for an amino acid sequence having the amino acid sequence of SEQ ID NO: 33, 36, 39, or 42;
(iv) a nucleotide sequence having at least 60% identity to one of the sequences selected form the group consisting of SEQ ID NO: 31, 34, 37 or 40 or having a cDNA at least 60% identical to one of the sequences selected from the group consisting of SEQ ID NO: 32, 35, 38, or 41;
(v) a nucleotide sequence encoding for a polypeptide having at least 60% identity to one of the sequences selected from the group consisting of SEQ ID NO: 33, 36, 39, or 42;
(vi) a nucleotide sequence hybridizing with the reverse complement of a nucleotide sequence as defined in (i) to (v) under stringent hybridization conditions; and
(vii) a nucleotide sequence encoding a protein derived from the amino acid sequence encoded by the nucleotide sequence of (i) to (vi) by way of substitution, deletion and/or addition of one or more amino acid(s).

15. Use of a plant according to claim 10 or 11 for modifying genetic variation in a plant population.


SEQUENCE LISTING

<110>  KWS SAAT SE & Co. KGaA

<120>  Modifying Genetic Variation In Crops By Modulating The Pachytene
       Checkpoint Protein 2

<130>  KWS0324EP

<160>  42

<170>  PatentIn version 3.5

<210>  1
<211>  1428
<212>  DNA
<213>  Artificial Sequence

<220>
<223>  cDNA of pch2 derived from Hordeum vulgare cv. Optic

<400>  1
atgagcgccc ccatggagat ctccttcgac ccccaccctt ctcatcccgc cctgcccgcc      60

ttcccatcca gcccacccct ggagcctgca gccgccgcca cccctcccgc ggccccgtcc     120

tcgtcgcagc cgacggcgga ggccgtatct gcgcccaccc cgccagacga caaggtcctc     180

gtgtcagttg aggtgctgct gcacgcctcg agtacggctc gcgccgagga cgtccgagca     240

gcggtggaga ggatgttaga atctcgaagt ttaagctatg ttgatggtcc tgtccagata     300

cctcctgaca attcatttct tgtcgaacat gttaaaagga tccaaatatg tgacactgat     360

gagctgattg agaaccacaa aatcctgttg ttttggcaag tcaaacctga cgttcatgtg     420

ttccggttga atgaagatgg tccaggtgaa gaaccaagtg aagatgacac tctctctagc     480

ttcgatgagt gggctctacc tgccaaagaa tttgatggat tatgggaaag cttactatat     540

gaggtgggtc tcaagcagag gttgctgaga tacgctgcta gtgctttgct atttactgaa     600

agaggtgtag acccatgcct tgtttcatgg aacaggattg tacttttgca tggtccacct     660

ggaacaggaa agacttcatt gtgtaaagca ctagctcaga agctttctat tcgtttcaat     720

tcaaggtatt ctatgtgcca actaattgaa gtcaatgccc attcattgtt cagcaagtgg     780

ttttctgaaa gtgggaaact ggtagccaaa cttttccaga agattcagga gatggtggag     840

gaagaaagca acctggtatt tgtattgatc gatgaagttg aaagccttgc tgctgccagg     900

caggctgcca tatctggctc tgagccttca gactccatta gggtggtaaa tgccttgcta     960

acgcaaatgg acaaactgaa ggcttggcca aatgttatca ttttgacaac atcaaatatc    1020

actacagcta tcgatattgc atttgttgac agggcagata tcaaggcata cgtcggcccc    1080

cctactcttc aagcacgcta tgagattttg aggtcgtgca tagatgaact cttccgagta    1140

ggaattctta aatatcctca gggtggtgat ttgccttgta ttctgagtta ttcaaccctg    1200

aaggagaagt tgcactgtcc tgaggctaca gaaccccata cacttcatct ctccaaacta    1260

ctgcatgaag gtgcagaact gtgtgacgga ttgagcggaa gatcattgag aaaactccct 1320

ttcctggccc acgcttccgt tcccaaccct tcatgctgtg atgctacttc tttcatgcac 1380

acgctgatac agaccgcgcg gagggagctc ttggaatcac gcggctga 1428

<210> 2
<211> 475
<212> PRT
<213> Hordeum vulgare

<400> 2

Met Ser Ala Pro Met Glu Ile Ser Phe Asp Pro His Pro Ser His Pro
1               5                   10                  15

Ala Leu Pro Ala Phe Pro Ser Ser Pro Pro Leu Glu Pro Ala Ala Ala
            20                  25                  30

Ala Thr Pro Pro Ala Ala Pro Ser Ser Ser Gln Pro Thr Ala Glu Ala
        35                  40                  45

Val Ser Ala Pro Thr Pro Pro Asp Asp Lys Val Leu Val Ser Val Glu
    50                  55                  60

Val Leu Leu His Ala Ser Ser Thr Ala Arg Ala Glu Asp Val Arg Ala
65                  70                  75                  80

Ala Val Glu Arg Met Leu Glu Ser Arg Ser Leu Ser Tyr Val Asp Gly
                85                  90                  95

Pro Val Gln Ile Pro Pro Asp Asn Ser Phe Leu Val Glu His Val Lys
            100                 105                 110

Arg Ile Gln Ile Cys Asp Thr Asp Glu Leu Ile Glu Asn His Lys Ile
        115                 120                 125

Leu Leu Phe Trp Gln Val Lys Pro Asp Val His Val Phe Arg Leu Asn
    130                 135                 140

Glu Asp Gly Pro Gly Glu Glu Pro Ser Glu Asp Asp Thr Leu Ser Ser
145                 150                 155                 160

Phe Asp Glu Trp Ala Leu Pro Ala Lys Glu Phe Asp Gly Leu Trp Glu
                165                 170                 175

Ser Leu Leu Tyr Glu Val Gly Leu Lys Gln Arg Leu Leu Arg Tyr Ala
            180                 185                 190

Ala Ser Ala Leu Leu Phe Thr Glu Arg Gly Val Asp Pro Cys Leu Val
        195                 200                 205

Ser Trp Asn Arg Ile Val Leu Leu His Gly Pro Pro Gly Thr Gly Lys
    210                 215                 220

Thr Ser Leu Cys Lys Ala Leu Ala Gln Lys Leu Ser Ile Arg Phe Asn
225                 230                 235                 240

Ser Arg Tyr Ser Met Cys Gln Leu Ile Glu Val Asn Ala His Ser Leu
                245                 250                 255

Phe Ser Lys Trp Phe Ser Glu Ser Gly Lys Leu Val Ala Lys Leu Phe
            260                 265                 270

Gln Lys Ile Gln Glu Met Val Glu Glu Glu Ser Asn Leu Val Phe Val
        275                 280                 285

Leu Ile Asp Glu Val Glu Ser Leu Ala Ala Ala Arg Gln Ala Ala Ile
    290                 295                 300

Ser Gly Ser Glu Pro Ser Asp Ser Ile Arg Val Val Asn Ala Leu Leu
305                 310                 315                 320

Thr Gln Met Asp Lys Leu Lys Ala Trp Pro Asn Val Ile Ile Leu Thr
                325                 330                 335

Thr Ser Asn Ile Thr Thr Ala Ile Asp Ile Ala Phe Val Asp Arg Ala
            340                 345                 350

Asp Ile Lys Ala Tyr Val Gly Pro Pro Thr Leu Gln Ala Arg Tyr Glu
        355                 360                 365

Ile Leu Arg Ser Cys Ile Asp Glu Leu Phe Arg Val Gly Ile Leu Lys
    370                 375                 380

Tyr Pro Gln Gly Gly Asp Leu Pro Cys Ile Leu Ser Tyr Ser Thr Leu
385                 390                 395                 400

Lys Glu Lys Leu His Cys Pro Glu Ala Thr Glu Pro His Thr Leu His
                405                 410                 415

Leu Ser Lys Leu Leu His Glu Gly Ala Glu Leu Cys Asp Gly Leu Ser
            420                 425                 430

Gly Arg Ser Leu Arg Lys Leu Pro Phe Leu Ala His Ala Ser Val Pro
        435                 440                 445

Asn Pro Ser Cys Cys Asp Ala Thr Ser Phe Met His Thr Leu Ile Gln
    450                 455                 460

Thr Ala Arg Arg Glu Leu Leu Glu Ser Arg Gly
465                 470                 475

<210>  3
<211>  2045
<212>  DNA
<213>  Hordeum vulgare

<220>
<221>  misc_feature
<222>  (1409)..(1409)
<223>  n is a, c, g, or t

<400>  3
ccatggagat ctccttcgac ccccaccctt ctcatcccgc cctgcccgcc ttcccctcca      60

gcccacccct ggagcctgca gccgccgcca cccctcccgc ggccccgtcc tcgtcgcagc     120

cgacggcgga ggccgtagct gcgcccaccc cgccagacga caaggtcctc gtgtcaggta     180

ggcgccgcga cgcgcacgcg tttccggcct cttcctagcg ccgcatgact aaaccgtgct     240

ctgcgaatgc ccgatttggt ttcgccgggg ttgatttttt ccttttgtgc ttgcagttga     300

ggtgctgctg cacgcctcga gtacggctcg cgccgaggac gtccgagcag cggtggagag     360

gtaggtgctg ttcggattga actttgtttc gggcttattt tactgttatt tggatgcttg     420

atgacacgtg gatgcacgag ctgctaaatg cgggttgggt cagatatagt ttggcaccgg     480

aaaaatggaa atgtgccatg gttctaggga acttggtcaa caatcttcct gtgatttagc     540

agtggagttt gctgggtatg ttttaatagg aattaacagg catttttgtt cactagtcgc     600

tcatgatgca gcatattcga gatcgacgtg gcaactagta aagttaggag tgctaaattg     660

tttgtaataa gtaattaggc attgcagggt gattagcatt ttagagaaaa acatcagcta     720

gtgttataac agattagcaa ctaggtgggg atttctgccg tgcaacatgg atataagctg     780

atcatttcaa aacttactgc gtccatcatt tgcttgtata caaaggatgg agaaagcagt     840

aaatgaagtt aaaaaatcgt atcatctgta gtgttctgtt cagaaaagat taccggaaac     900

agctataagc caatcattta aaaaaaatat cacgttcaac tgttcatcat tgcttattga     960

taggctggat aactcaccaa agcaatgcca gaaatcttat cacgcttctt tcatattaaa    1020

gtggtaatca gatctgaggc catggaattg acatagtttc ttgtagacag cttctgggtt    1080

taagctcaca gcatacatat tgaatttgcg cagatatctt cttgtgcagg acattaatgt    1140

ttgttattgc tcatcaacat ttacaagtta ttttcttatc aggatgttag aatctcgaag    1200

tttaagctat gttgatggtc ctgtccagat acctcctgac aattcatttc ttgtcgaaca    1260

tgttaaaagg atccaaatat gtgacactgg tacgatgttt cacttaggct ggaaagtgtg 1320

ctttcttgat ctcagttatc tatgtacctc ttcctgaatg cctgcttttc atgtagatga 1380

gctgattgag aaccacaaaa tcctgttgnt ttggcaagtc aaacctgacg ttcatgtgtt 1440

ccgggtatgc atttaaatgt tttctgccta aatcacttca atctttgctg cttaaatctc 1500

ctctagttcc ttttaaaaca cagaaagtct ttgggtatgt ttacatgcta ttgccggcta 1560

tctttcttct ttaatcagtt gcatgttgaa atcgtttcat cttcacatca taagatatct 1620

tcgtaatgct gctgaggtgc tggtgttttg ttgttttcac ttaaaatttc acacttgttt 1680

tctcttgaaa caaccggtat tttctaaatt agtctactga cctctttcca agagatagat 1740

tgctttatgt catgtattct agtgaagatc tggttcaaat ttatcgtaca cctcaaggct 1800

caagctgatc tggttatctt gcagttgaat gaagatggtc caggtgaaga accaagtgaa 1860

gatgacactc tctctagctt cgatgagtgg gctctacctg ccaaagaatt tgatggatta 1920

tgggaaaggt ttggtttgat ctcagtatcc ttgaccagta caatttctgt gttttttatt 1980

ctgttgatga ttgcgtatgt gcttgcagct tactatatga ggtgggtctc aagcagaggt 2040

tgctg 2045

<210> 4
<211> 21
<212> DNA
<213> Artificial Sequence

<220>
<223> forward primer D15

<400> 4
ccatggagat ctccttcgac c 21

<210> 5
<211> 21
<212> DNA
<213> Artificial Sequence

<220>
<223> reverse primer D16

<400> 5
cagccgcgtg attccaagag c 21

<210> 6
<211> 21
<212> DNA
<213> Artificial Sequence

<220>
<223> reverse primer D18

<400> 6
cagcaacctc tgcttgagac c 21

<210>  7
<211>  19
<212>  DNA
<213>  Artificial Sequence

<220>
<223>  reverse primer D30

<400>  7
ggtctgtatc agcgtgtgc                                                    19


<210>  8
<211>  18
<212>  DNA
<213>  Artificial Sequence

<220>
<223>  reverse primer D32

<400>  8
gtcacatatt tggatcct                                                     18


<210>  9
<211>  17
<212>  DNA
<213>  Artificial Sequence

<220>
<223>  forward primer D31

<400>  9
acagctataa gccaatc                                                      17


<210>  10
<211>  20
<212>  DNA
<213>  Hordeum vulgare

<400>  10
tggagatctc cttcgacccc                                                   20


<210>  11
<211>  20
<212>  DNA
<213>  Hordeum vulgare

<400>  11
gcccaccccg ccagacgaca                                                   20


<210>  12
<211>  20
<212>  DNA
<213>  Hordeum vulgare

<400>  12
tctcatcccg ccctgcccgc                                                   20

<210>  13
<211>  20
<212>  DNA
<213>  Hordeum vulgare

<400>  13
gctgctgcac gcctcgagta                                                    20


<210>  14
<211>  227
<212>  DNA
<213>  Artificial Sequence

<220>
<223>  152bp deletion of line 1 (1967-08)

<400>  14
ttattccgct gcctcgcgga gcctggccac catgagcgcc cccatgggac aaggtcctcg        60

tgtcaggtag gcgccgcgac gcgcacgcgt ttccggcctc ttcctagcgc cgcatgacta       120

aaccgtgctc tgcgaatgcc cgatttggtt tcgccggggt tgattttttc cttttgtgct         180

tgcagttgag gtgctgctgc acgcctcgag tacggctcgc gccgaga                     227


<210>  15
<211>  12
<212>  PRT
<213>  Artificial Sequence

<220>
<223>  truncated translation product of pch2 of mutant line 1 (1967-08)

<400>  15

Met Ser Ala Pro Met Gly Gln Gly Pro Arg Val Arg
1               5                   10


<210>  16
<211>  399
<212>  DNA
<213>  Artificial Sequence

<220>
<223>  point mutation of line 2 (1967-04)

<400>  16
atttaattcc gtgccttcgg ggacccggcc ccccagaagg gcccccagga atctccttcg        60

acccccaccc ttctcatccc gccctgcccg ccttcccctc cagcccaccc ctggagcctg       120

cagccgccgc cacccctccc gcggccccgt cctcgtcgca gccgacggcg gaggccgtag       180

ctgcgcccac cccgccagac gacaaggtcc tcgtgtcagg taggcgccgc gacgcgcacg       240

cgtttccggc ctcttcctag cgccgcatga ctaaaccgtg ctctgcgaat gcccgatttg       300

gtttcgccgg ggttgatttt ttccttttgt gcttgcagtt gaggtgctgc tgcacgcctc        360

gagtacggct cgcgccgagg acgtcccgag caggcagta                                399

<210>  17
<211>  17
<212>  PRT
<213>  Artificial Sequence

<220>
<223>  truncated translation product of pch2 of mutant line 2 (1967-04)

<400>  17

Met Pro Asp Leu Val Ser Pro Gly Leu Ile Phe Ser Phe Cys Ala Cys
1               5                   10                  15

Ser

<210>  18
<211>  336
<212>  DNA
<213>  Artificial Sequence

<220>
<223>  point mutation of line 3 (1967-05)

<400>  18
cccccagaga tctccttcga cccccaccct tctcatcccg ccctgcccgc cttcccctcc        60

agcccacccc tggagcctgc agccgccgcc acccctcccg cggccccgtc ctcgtcgcag       120

ccgacggcgg aggccgtagc tgcgcccacc ccgccagacg acaaggtcct cgtgtcaggt       180

aggcgccgcg acgcgcacgc gtttccggcc tcttccagcg ccgcatgact aaaccgtgct       240

ctgcgaatgc ccgatttggt ttcgccgggg ttgatttttt ccttttgtgc ttgcagttga       300

ggtgctgctg cacgcctcga gtacggctcg cgtcga                                 336

<210>  19
<211>  17
<212>  PRT
<213>  Artificial Sequence

<220>
<223>  truncated translation product of pch2 of mutant line 2 (1967-05)

<400>  19

Met Pro Asp Leu Val Ser Pro Gly Leu Ile Phe Ser Phe Cys Ala Cys
1               5                   10                  15

Ser

<210>  20

<211>  21
<212>  DNA
<213>  Artificial Sequence

<220>
<223>  forward primer HvCas9F1

<400>  20
gccgtcatta cggacgagta c                                                21


<210>  21
<211>  25
<212>  DNA
<213>  Artificial Sequence

<220>
<223>  reverse primer HvCas9R1

<400>  21
agtcagcctt atcagtactg tctac                                            25


<210>  22
<211>  21
<212>  DNA
<213>  Artificial Sequence

<220>
<223>  forward primer HvCas9F2

<400>  22
gtatctggcc agccactatg a                                                21


<210>  23
<211>  24
<212>  DNA
<213>  Artificial Sequence

<220>
<223>  reverse primer HvCas9R2

<400>  23
ccgtaattga ctgatgaatc agtg                                             24


<210>  24
<211>  18
<212>  DNA
<213>  Artificial Sequence

<220>
<223>  forward primer F2

<400>  24
ttccattccg ctgcctcg                                                    18


<210>  25
<211>  19
<212>  DNA
<213>  Artificial Sequence

<220>
<223>  reverse primer 3

<400>  25
gcatccacgt gtcatcaag                                                    19


<210>  26
<211>  23
<212>  DNA
<213>  Artificial Sequence

<220>
<223>  Common sequencing forward primer

<400>  26
agttcaatcc gaacagcacc tac                                               23


<210>  27
<211>  18
<212>  DNA
<213>  Artificial Sequence

<220>
<223>  WT reverse primer

<400>  27
tgagcgcccc catggaga                                                     18


<210>  28
<211>  18
<212>  DNA
<213>  Artificial Sequence

<220>
<223>  Line 2 mutant reverse primer

<400>  28
atgagggccc ccagggaa                                                     18


<210>  29

<400>  29
000

<210>  30

<400>  30
000

<210>  31
<211>  7505
<212>  DNA
<213>  Hordeum vulgare


<220>
<221>  misc_feature
<222>  (1)..(61)

<223>  n is a, c, g, or t

<220>
<221>  misc_feature
<222>  (477)..(594)
<223>  n is a, c, g, or t

<400>  31
nnnnnnnnnn nnnnnnnnnn nnnnnnnnnn nnnnnnnnnn nnnnnnnnnn nnnnnnnnnn      60

ntatatatat atatatatat atatattgca gcgggcgccg aataggggtt tagctatatt     120

taccgcagag agcgttaagt agcaaagatc acagacgcac gctgggtcga aaatgggttt     180

cctattgagc ccatccaggt ctctaggccc acctagagct tacagagaga gcccatctcc     240

tgaccctaaa gtacctcaag cgcgggaaac ccaaatcctc cctcctcctt cccccaaatc     300

ccccattcct ccacggctcc gcctcccctc ccctctccaa acccaaaacc ctaggacctt     360

ttccattccg ctgcctcgcg gagcctggcc accatgagcg cccccatgga gatctccttc     420

gacccccacc cttctcatcc cgccctgccc gccttcccct ccagcccacc cctggannnn     480

nnnnnnnnnn nnnnnnnnnn nnnnnnnnnn nnnnnnnnnn nnnnnnnnnn nnnnnnnnnn     540

nnnnnnnnnn nnnnnnnnnn nnnnnnnnnn nnnnnnnnnn nnnnnnnnnn nnnnccaccc     600

ctcccgcggc cccgtcctcg tcgcagccga cggcggaggc cgtagctgcg cccaccccgc     660

cagacgacaa ggtcctcgtg tcaggtaggc gccgcgacgc gcacgcgttt ccggcctctt     720

cctagcgccg catgactaaa ccgtgctctg cgaatgcccg atttggtttc gccggggttg     780

attttttcct tttgtgcttg cagttgaggt gctgctgcac gcctcgagta cggctcgcgc     840

cgaggacgtc cgagcagcgg tggagaggta ggtgctgttc ggattgaact ttgtttcggg     900

cttattttac tgttatttgg atgcttgatg acacgtggat gcacgagctg ctaaatgcgg     960

gttgggtcag atatagtttg gcaccggaaa aatggaaatg tgccatggtt ctagggaact    1020

tggtcaacaa tcttcctgtg atttagcagt ggagtttgct gggtatgttt taataggaat    1080

taacaggcat ttttgttcac tagtcgctca tgatgcagca tattcgagat cgacgtggca    1140

actagtaaag ttaggagtgc taaattgttt gtaataagta attaggcatt gcagggtgat    1200

tagcatttta gagaaaaaca tcagctagtg ttataacaga ttagcaacta ggtggggatt    1260

tctgccgtgc aacatggata taagctgatc atttcaaaac ttactgcgtc catcatttgc    1320

ttgtatacaa aggatggaga aagcagtaaa tgaagttaaa aaatcgtatc atctgtagtg    1380

ttctgttcag aaaagattac cggaaacagc tataagccaa tcatttaaaa aaaatatcac    1440

gttcaactgt tcatcattgc ttattgatag gctggataac tcaccaaagc aatgccagaa    1500

atcttatcac gcttctttca tattaaagtg gtaatcagat ctgaggccat ggaattgaca    1560

tagtttcttg tagacagctt ctgggtttaa gctcacagca tacatattga atttgcgcag    1620

atatcttctt gtgcaggaca ttaatgtttg ttattgctca tcaacattta caagttattt    1680

tcttatcagg atgttagaat ctcgaagttt aagctatgtt gatggtcctg tccagatacc 1740

tcctgacaat tcatttcttg tcgaacatgt taaaaggatc caaatatgtg acactggtac 1800

gatgtttcac ttaggctgga aagtgtgctt tcttgatctc agttatctat gtacctcttc 1860

ctgaatgcct gcttttcatg tagatgagct gattgagaac cacaaaatcc tgttgttttg 1920

gcaagtcaaa cctgacgttc atgtgttccg ggtatgcatt taaatgtttt ctgcctaaat 1980

cacttcaatc tttgctgctt aaatctcctc tagttccttt taaaacacag aaagtctttg 2040

ggtatgttta catgctattg ccggctatct ttcttcttta atcagttgca tgttgaaatc 2100

gtttcatctt cacatcataa gatatcttcg taatgctgct gaggtgctgg tgttttgttg 2160

ttttcactta aaatttcaca cttgttttct cttgaaacaa ccggtatttt ctaaattagt 2220

ctactgacct ctttccaaga gatagattgc tttatgtcat gtattctagt gaagatctgg 2280

ttcaaattta tcgtacacct caaggctcaa gctgatctgg ttatcttgca gttgaatgaa 2340

gatggtccag gtgaagaacc aagtgaagat gacactctct ctagcttcga tgagtgggct 2400

ctacctgcca aagaatttga tggattatgg gaaaggtttg gtttgatctc agtatccttg 2460

accagtacaa tttctgtgtt ttttattctg ttgatgattg cgtatgtgct tgcagcttac 2520

tatatgaggt gggtctcaag cagaggttgc tgagatacgc tgctagtgct ttgctattta 2580

ctgaaagagg tgtagaccca tgccttgttt catggaacag gtaagcatct ttcacattgt 2640

taatcacttg gaggcttcac atgccatcag aagtattacc ttcgttatga actataagat 2700

gttttggata tttcaatatg gactacatac ggactgaaat tagtgaacag acacactaaa 2760

acagtaaaac acgtctataa ttctatatac tacaaagcat ggacagggca gaagttgccg 2820

aatccccgtg ctgatacggc cagacacggg gatacgctcg gacacgcggg gatacacgta 2880

tcccgccgta tttcctgaat tacgaattaa aaaaagaaaa gaaaagggag gacatggctg 2940

ggacacgtag gggacacgtg aggggctcga ttaatccatc ggtcggagca gcaccctcct 3000

ccgacttcag gcagcgctgg atctggagca ccctcccgcc gccgtgctgc tgctgtgcct 3060

ccactgctgt gcttcgctcc cgccgttgct gctgctgctg cagcggcaag aggagcacgc 3120

tgggaggagg ggggtggcga gaggtgctgc gggggagttg agagatccat cgctgcgagg 3180

aggggcaatg gcaagagagt ggtggcttgg tcaaaagcat tagggcagag agagataggg 3240

cttgagtttt gacggtgcag attgatctgt ggcatgcact agctgggctg gcccaactac 3300

taatatcctc tctgggctta tgttaagaga gttactgctg aatggggcta tcttttgtta 3360

attttttcct ctttacctct atattacatg gtattattta tttatttata tatatatatt 3420

tgacttgttc ccaatgggct gtttttggat aatgccgtat cccgtgtccc catatgcgtg 3480

tcaccgtgtc tgtgtcccgt gctttttagt ctatatacat ctgattcaga aaaacataga 3540

acactttata attcgtaaag gggggagggg gagtagtata ttttgatttt actttccata 3600

aagttggtca ttactgccat gtaaccttag ggtacctttt agtgtaattg ggtttgcagt 3660

tagctgctga aacctttcaa tgacgttcat gagtacttcc tgaaattgga attggaaagt 3720

ggaccattgt ctccttcctc ttcttcagtt cttctttgag tccattgatg gatattgttt 3780

gttgggatac ttaatcaagg tgagtgtgca aatattgcaa aaaggaattg cagcttaatt 3840

gtcaagggat actcaataga taatttggat gcaaaagcct gagcttcgtt aaactggact 3900

acttacctta atacccatgc aaatgtgcac tttcaattat ataaattatt ctctaggaat 3960

atatgattca ttttgttttg tatttttagt ttatgtactt ttttacatat aattgcagga 4020

ttgtactttt gcatggtcca cctggaacag gaaagacttc attgtgtaaa gcactagctc 4080

agaagctttc tattcgtttc aattcaaggt tcttgcttaa tgatattaat gtctattttt 4140

tttaataaat aatgtgttat taattactgt ctttttttca ccaggtattc tatgtgccaa 4200

ctaattgaag tcaatgccca ttcattgttc agcaagtggt tttctgaaag tgggaaactg 4260

gtatgccctt ttgtgcacat attagtaata ttatcatgta tacgttcatg ctgaaatcac 4320

atcatcagct cgtggtaatt gagttacact atgttttttg caccattttg catgatctgg 4380

aaaatttacc attatatgat acattttgtt gtatgtagtt agtttagccc actaaaattg 4440

tccattggag ataaatcttc atttccatct tacattgtac atactataat ttgtcctttc 4500

aggtagccaa acttttccag aagattcagg agatggtgga ggaagaaagc aacctggtat 4560

ttgtattgat cggtatgttt atgacaaaac tggtgaaatg actagatgtt caatctccgt 4620

atgatctgac cagctcaatc catgccttgt tcatcttatc tatgcgttgt taaaaggaat 4680

tctcattcca ttgtgttttt tagtttcaga aatgcctcaa tactgataaa atactgaacc 4740

acattgagct acatgacata tgggcagata agtaagacag caaattattt gtccttttta 4800

tgaatagaga gaacaaagat cataaaatgt tttttaaatc gaaacataca caatttaatt 4860

ccctaggcat catctcctaa ttgccgacta gtgaaacgca ttgtggcact cctatagaat 4920

tttaactata cttcaagatc ttagataatt ttgatttgcg gcatgctgta tatctgaacc 4980

cttaatgtta tctatggcgt ttatgcatta taatacgtga tattgcagat gaagttgaaa 5040

gccttgctgc tgccaggcag gctgccatat ctggctctga gccttcagac tccattaggg 5100

taatgtttac atcagttttc ctttgccata gactacgtcc gtctcaaaat ataatacgtt 5160

tttgtaggct aatagcctac aaaaacgtat tatattttga gacggaggca gtacctttta 5220

tcaggttatg tttacagtct tcttccattc tgtaactttc aggtggtaaa tgccttgcta 5280

acgcaaatgg acaaactgaa ggcttggcca aatgttatca ttttgacaac atcaaatatc 5340

actacagcta tcggtattga tttcccccct tttgagtttt tccttttatg acctgatggc 5400

ccattgtgtc attctgtgtg catggttgcc acatcttttg catggttatc tgtgcgtttg 5460

gaacaacggt gctgtttatg atcaacatct ggacaaattt ggacatttag tatgtgtttt 5520

agttggtcaa gtgaatcacc gcagtgaggg gaagatcaat tgcataataa gaagttcatg 5580

tttgtggggc tgcccatgtt ataacatcac ttttcctact tttccgaagt aactaatgct 5640

acattattga tacatttgaa tttagataat catgttgcca tcgacaatga gatatcaact 5700

gtgctcagta ctgatatctc ttgggctaac aaaattgcct aactgtggac attcattcga 5760

ttttcgatga ctgttaacga gaacagcctg tagctgtcta aaacatcgtg tagacatgta 5820

tggagtccta aaataaataa attctaactc gtatgtagtt tgcagatatt gcatttgttg 5880

acagggcaga tatcaaggca tacgtcggcc cccctactct tcaagcacgc tatgagattt 5940

tgaggtcgtg catagatgaa ctcttccgag taggaattct taaatatcct caggtatttg 6000

ttcctttttg ccaacttggt cgctgtaatg ccaccttttt aaattaaatg aacatcacta 6060

taaggttgct cagaactgct ttgcgcatgg ggcacctttt ctgagcaaaa ctatatttgg 6120

gatctcaaag gatttgtttg tttgaactgc tgtatagaat ttccagcttt cttgcaatct 6180

ttaaaggatt attcctctgc gaatgtaggg tggtgatttg ccttgtattc tgagttattc 6240

aaccctgaag gagaagttgc actgtcctga ggctacagaa ccccatacac ttcatctctc 6300

caaactactg catgaaggtg cagaactgtg tgacgtaagt aatgaagttc acagttacct 6360

tactttcttt aacgctgtag gttaagtgtt cttctgtaat ccagcccaac aggcttatgg 6420

tggtgatttt ttttttacat tctcccacag ggattgagcg gaagatcatt gagaaaactc 6480

cctttcctgg cccacgcttc cgttcccaac ccttcatgct gtgatgctac ttctttcatg 6540

cacacgctga tacagaccgc gcggagggag ctcttggaat cacgcggctg attcctcgtg 6600

gattgcttgg aagagcaagg ggctaacagt tcgatacaag taccatgttg ccgatatgat 6660

gcatagtttg agctcgaaga ctggtgttct gagataaaag caaatccatt ccatctctga 6720

gatataggtg ccgttgctgt tgcattactg ttggttcgct tctaaattgt tacggtagga 6780

tgcacctcac cttgagggca gctgtgactt tttttgcggt gaagttaatg caatacattg 6840

gtcacttgat cagatccttt tctggtaatt ggaacttgaa atggcagcgt ttgtgtgtgt 6900

tacgaagtag atttgttttc acttattacc ggtttcacat gtctctgcaa gatggaaatt 6960

tagaagttgt ctggactgtt tggctcggcg tcagcttttc aggctcacag gaagctgaaa 7020

ctaacatgcg acgcataacc atgtgcattc cttgctaagg ccaactctag cgcgcgacct 7080

catcctgtcc gactgcgctc gtttgaggca aaatagacat atcaaacggc ctagcgtgcg 7140

ggtgcaaacg gacttttgtc cgttttgacc catcctcggc caagtttgag ccgtctttgg 7200

gatgaaatgg acatcacacg gacagaccgg acgtgcgtgt ttgtcctccc ttgacccacc 7260

tgtcggtggc acaagcattc ctttttctat ccctcactct acccccaccc tcacccatcg 7320

agccctccag acgcaccgcc cgccaccctt gtccctcgtc gtcgccaccg ccactaccag 7380

ttcagctcgg acgcgcgctc gccccaaacc ttcgcaggcg ctcggatttg gagcggatcc 7440

ggccggaatt ggggctcgcc aggttgcggg gaggctacgc cgtgccatgg actggccgga 7500

caccg 7505

<210> 32
<211> 1428
<212> DNA
<213> Artificial Sequence

<220>
<223> cDNA of pch2 from Hordeum vulgare

<400> 32
atgagcgccc ccatggagat ctccttcgac ccccaccctt ctcatcccgc cctgcccgcc 60

ttcccctcca gcccacccct ggagcctgca gccgccgcca cccctcccgc ggccccgtcc 120

tcgtcgcagc cgacggcgga ggccgtatct gcgcccaccc cgccagacga caaggtcctc 180

gtgtcagttg aggtgctgct gcacgcctcg agtacggctc gcgccgagga cgtccgagca 240

gcggtggaga ggatgttaga atctcgaagt ttaagctatg ttgatggtcc tgtccagata 300

cctcctgaca attcatttct tgtcgaacat gttaaaagga tccaaatatg tgacactgat 360

gagctgattg agaaccacaa aatcctgttg ttttggcaag tcaaacctga cgttcatgtg 420

ttccggttga atgaagatgg tccaggtgaa gaaccaagtg aagatgacac tctctctagc 480

ttcgatgagt gggctctacc tgccaaagaa tttgatggat tatgggaaag cttactatat 540

gaggtgggtc tcaagcagag gttgctgaga tacgctgcta gtgctttgct atttactgaa 600

agaggtgtag acccatgcct tgtttcatgg aacaggattg tacttttgca tggtccacct 660

ggaacaggaa agacttcatt gtgtaaagca ctagctcaga agctttctat tcgtttcaat 720

tcaaggtatt ctatgtgcca actaattgaa gtcaatgccc attcattgtt cagcaagtgg 780

ttttctgaaa gtgggaaact ggtagccaaa cttttccaga agattcagga gatggtggag 840

gaagaaagca acctggtatt tgtattgatc gatgaagttg aaagccttgc tgctgccagg 900

caggctgcca tatctggctc tgagccttca gactccatta gggtggtaaa tgccttgcta 960

acgcaaatgg acaaactgaa ggcttggcca aatgttatca ttttgacaac atcaaatatc 1020

actacagcta tcgatattgc atttgttgac agggcagata tcaaggcata cgtcggcccc 1080

cctactcttc aagcacgcta tgagattttg aggtcgtgca tagatgaact cttccgagta 1140

ggaattctta aatatcctca gggtggtgat ttgccttgta ttctgagtta ttcaaccctg 1200

aaggagaagt tgcactgtcc tgaggctaca gaaccccata cacttcatct ctccaaacta 1260

ctgcatgaag gtgcagaact gtgtgacgga ttgagcggaa gatcattgag aaaactccct 1320

ttcctggccc acgcttccgt tcccaaccct tcatgctgtg atgctacttc tttcatgcac 1380

acgctgatac agaccgcgcg gagggagctc ttggaatcac gcggctga 1428

<210> 33
<211> 475
<212> PRT
<213> Hordeum vulgare

<400> 33

Met Ser Ala Pro Met Glu Ile Ser Phe Asp Pro His Pro Ser His Pro
1               5                   10                  15

Ala Leu Pro Ala Phe Pro Ser Ser Pro Pro Leu Glu Pro Ala Ala Ala
            20                  25                  30

Ala Thr Pro Pro Ala Ala Pro Ser Ser Ser Gln Pro Thr Ala Glu Ala
        35                  40                  45

Val Ser Ala Pro Thr Pro Pro Asp Asp Lys Val Leu Val Ser Val Glu
    50                  55                  60

Val Leu Leu His Ala Ser Ser Thr Ala Arg Ala Glu Asp Val Arg Ala
65                  70                  75                  80

Ala Val Glu Arg Met Leu Glu Ser Arg Ser Leu Ser Tyr Val Asp Gly
                85                  90                  95

Pro Val Gln Ile Pro Pro Asp Asn Ser Phe Leu Val Glu His Val Lys
            100                 105                 110

Arg Ile Gln Ile Cys Asp Thr Asp Glu Leu Ile Glu Asn His Lys Ile
        115                 120                 125

Leu Leu Phe Trp Gln Val Lys Pro Asp Val His Val Phe Arg Leu Asn
    130                 135                 140

Glu Asp Gly Pro Gly Glu Glu Pro Ser Glu Asp Asp Thr Leu Ser Ser
145                 150                 155                 160

Phe Asp Glu Trp Ala Leu Pro Ala Lys Glu Phe Asp Gly Leu Trp Glu
                165                 170                 175

Ser Leu Leu Tyr Glu Val Gly Leu Lys Gln Arg Leu Leu Arg Tyr Ala
            180                 185                 190

Ala Ser Ala Leu Leu Phe Thr Glu Arg Gly Val Asp Pro Cys Leu Val
        195                 200                 205

Ser Trp Asn Arg Ile Val Leu Leu His Gly Pro Pro Gly Thr Gly Lys
    210                 215                 220

Thr Ser Leu Cys Lys Ala Leu Ala Gln Lys Leu Ser Ile Arg Phe Asn
225                 230                 235                 240

Ser Arg Tyr Ser Met Cys Gln Leu Ile Glu Val Asn Ala His Ser Leu
                245                 250                 255

Phe Ser Lys Trp Phe Ser Glu Ser Gly Lys Leu Val Ala Lys Leu Phe
            260                 265                 270

Gln Lys Ile Gln Glu Met Val Glu Glu Glu Ser Asn Leu Val Phe Val
        275                 280                 285

Leu Ile Asp Glu Val Glu Ser Leu Ala Ala Ala Arg Gln Ala Ala Ile
    290                 295                 300

Ser Gly Ser Glu Pro Ser Asp Ser Ile Arg Val Val Asn Ala Leu Leu
305                 310                 315                 320

Thr Gln Met Asp Lys Leu Lys Ala Trp Pro Asn Val Ile Ile Leu Thr
                325                 330                 335

Thr Ser Asn Ile Thr Thr Ala Ile Asp Ile Ala Phe Val Asp Arg Ala
            340                 345                 350

Asp Ile Lys Ala Tyr Val Gly Pro Pro Thr Leu Gln Ala Arg Tyr Glu
        355                 360                 365

Ile Leu Arg Ser Cys Ile Asp Glu Leu Phe Arg Val Gly Ile Leu Lys
    370                 375                 380

Tyr Pro Gln Gly Gly Asp Leu Pro Cys Ile Leu Ser Tyr Ser Thr Leu
385                 390                 395                 400

Lys Glu Lys Leu His Cys Pro Glu Ala Thr Glu Pro His Thr Leu His
                405                 410                 415

Leu Ser Lys Leu Leu His Glu Gly Ala Glu Leu Cys Asp Gly Leu Ser
            420                 425                 430

Gly Arg Ser Leu Arg Lys Leu Pro Phe Leu Ala His Ala Ser Val Pro
        435                 440                 445

Asn Pro Ser Cys Cys Asp Ala Thr Ser Phe Met His Thr Leu Ile Gln
    450                 455                 460

Thr Ala Arg Arg Glu Leu Leu Glu Ser Arg Gly
465                 470                 475

<210>  34
<211>  6959
<212>  DNA
<213>  Triticum aestivum

<400>  34
gtcaaaaaca tgggtgccca ttgagcccat ccaggtcgct aaacccaccc ggagcttaca      60

gaaaaaatca ttggaagtta aatggaagcc catctcctga ccgcttcttt ggatcacagg     120

attagaaaac aaaggaattc aaaaaacaca tgaatttgac gggattgtag gtgcaaaaca     180

gaggattaca aaatagagaa aaactacaag aatggtcatt tggatggaat acaggaaaaa     240

cacaggaaaa gtgcctcgat cctacgcgaa tcagtgtaaa aaagtggtta tagtggatgt     300

tgaaattcct ataggattga ggtgtaggaa tgcatctata ggaattttgg aggtgtggtt     360

cctttaatcc aaaggacttc attagaaaaa aatccaatgg attggaatcc tccaatattc     420

ctatgccttc aatccaaaga ggccttaagg taccccaagc gcgggaaacc caaatcctct     480

cacagcctcc ttcccccaaa aaaatctcct attcctccac ggctccgcct tcccctcccc     540

tccccaaacc caaaacccta gaaccttctc cattccgctc cctcgcggag cctggccacc     600

atgagcgctc ccatggagat ctccttcgac ccccaccctt ctcatcccgc cccgcccgcc     660

ttcccctcca gcccacccct ggagcccgca gccgccgcca cccctcccgc ggtctcgtcc     720

tcgccgcagc cgacggcgga ggccgtagtc gcgcccaccc cgccagacga caaggtcctc     780

gtctcaggta ggcgccgcga cgcccgcgca tttctggcct cgccctggcg gcgcatgact     840

aaaccgtgct ctgcgaatgc ccgatttggt ttccccggcg ttgattgttt ttttcctttt     900

gcgcttgcag ttgaggtgtt gctgcacgcc acgagtacgg ctcgcgccga ggacgtccga     960

acggcggtcg agaggtcggt gctgttcggg ttacactttg tttcgagcat attttactgt    1020

tatttggatg cttgatgaca cgtggatgaa tgagttgcta aatgcgggtt ggattagatg    1080

tagtttggca ctggaaaagt gaaaatgtgc catgattcta gggaacgtgg tcaacaatca    1140

tcctgtgatt tagcagtgga gtttgctgag tatgttgcag taggaaataa cagacatgtt    1200

tggtaaagat aatcgctgat gatgcagcat atcggatatt gacgtggcaa ctagcaaagt    1260

taggaatact aaattgtttg taataggcaa ttagggtttg cagggtgatt aatattttag    1320

agcaaaatac cagataactt ttaagtttta caacagatta gcaattaggt ggggatttct    1380

gctgtgtaac atggatataa gctgatcatt taaaaaatga ctgttcacca tttgcttgca    1440

caaatgatgg agaaagctgt aaatgaagtt aaaaaatcat atcatctgta gtgttctgtt    1500

cagaaaagat cactggaaac agacataagc caatcttcat cattgcttat tgagaggccg    1560

gataactcac caaatcaatt ccagaaatct tatcattgtt tctttcatat taaagtgcta 1620
atcagatctg aggccatgga attgacagag tttcttgtag acagcttctt ggtttaagct 1680
catagcatat atattgaact tgcacagtta tcttctcttg caggacatta ctgcttgtta 1740
ttgctcatac ttaacattta caagttgttc tcttatcagg atgttagaat ctcgaagttt 1800
aagctatgtc gatggtcctg ttcagatacc tccagacaat tcttttcttc ttgaacatgt 1860
caaaaggatc caaatatgtg acactggtat gatgtttcac ttaggctgga aagtgtgctt 1920
tcttgatctc agttatctat gtacctcttc ctgaatgccc gcttttcatg tagatgagtt 1980
gattgagaac cacaaaatcc tgttgttttg gcaagtcaaa cctgtcgttc atgtattcca 2040
ggtatgcatc taaatgtttt ctgcctaaat cacttaaatc tttgttgcta aatctcttct 2100
agttcctttt aaaacacgga tggtctttgg gtatgtttac atgctattgc cggctatctt 2160
tcttctttga caattgactg ttggaattgt ttcatcttca catcataaga tatctttgta 2220
atgctgctga ggtactggtg ttttgttctt ttcacttaaa atttcacatt tgttttctct 2280
tgaaacaacc ggagtgattt tctaaattag actactgacc tctttccaag agatatatta 2340
cttcatgtca tgttctaagt gaacatttgg ttcaaattca tcatcgcctc aaggctcaag 2400
ctgatctagt tatcttgcag ttgagtgagg atggtccagg tgaagaacca agtgaagacg 2460
acacactctc tagcttcaat gagtgggctc tacctgccaa agaatttgat ggattgtggg 2520
aaaggtttgg tttgatctca ttatccttta ccagttacaa tttctgtgtt ttttattctg 2580
ttgatgatgt cgtatgtgct tgcagcttac tatatgaagt gggtctcaag cagaggttgc 2640
tgagatatgc tgctagtgct ttgctattta ctgaaagagg tgtagaccca tgccttgttt 2700
catggaacag gtaagcatct ttcacatcgt taagcacttg gaggcttcac atgtcatcag 2760
aagtggcata ttttggtttt actttccaca ataaagctgg tcattactgc catggaactt 2820
tagggcacct ttttgtgtag ttgggtttgc agttagcttc tgaaaccttt aaatgacgtt 2880
catgagtact tcctgaaatt gtatctggac agtggaccac tgtctccttc ctcttcttca 2940
gttcttcact tcttccttga gtctattggt ggatattgtt tgttgggata gttaatcaag 3000
atgaatgtgc aaaatactgc aaaaaagaat tgcagcttaa ttgtcaaggg atactcattg 3060
gatgcaaaag cctgaacttc gtgaaactga actacttacc ttttaatacc catgcaaatg 3120
cgtaactaca acaacaacaa caaagccttt agtcccaaac aagttggtag gctagagctg 3180
aaacccatgc aaatgtgcac tttcagttat ataaattatt ctctaggaat atataattta 3240
ttttgttttg tattgttcat gtgctttttt acatacaatt gcaggattgt acttttacat 3300
ggtccacctg gaacaggaaa gacttcatta tgtaaagcac tagctcagaa gctttctatt 3360
cgcttcaatt caaggttctt gcttaatgat attcatgact agtgttttaa taaataatgt 3420
gttattaatt acggtctctt tttcaccagg tattctatgt gccaactaat tgaagtcaat 3480

gcccattcat tgttcagcaa gtggttttct gaaagtggga aactggtatg gctttttgtg 3540

tttatatcaa taatattatc atgtatacgt tcatgctgaa atcacatcat tagctcgtgg 3600

taattgagtt ttgctatgtt tttgcaccat tttgcatgat gtggaaaatt caccatcata 3660

tgatacattt tgtagtatgt agttagttta gcccactaaa attgtccatt ggatatatct 3720

tcatttctgc cttacaatgt acatgctata atttgtcctt tttaggtagc taaacttttc 3780

cagaagattc aggagatggt ggaggaagaa agcaacctgg tatttgtatt gatcggtatg 3840

ttcatgacaa aactggtgaa atgactagat ggtcaatctc cgtatgatct gactagctca 3900

atccatgcct tgttcatctt atctatgtgt tgttaaacgg actcctcatt ccattgcgtt 3960

ttttacttaa agaaatgcct caatactaat gaataatgaa ccccattgag ctacatgaca 4020

tgtgggtgga taagtaagat agcaaatgat tttgtccttt atgtgaatag agataacata 4080

gatcgtaaat gtttttaaaa tcgaaatcaa cacaatttaa ttccctaggc attatctcct 4140

agttgcagac tagcaaaatg cattgtggca ctcctataga attttaacta aacctcattt 4200

tagataaatt tgatttgcag catgctgtat ttgaaccctt aatgttatct atggcgttta 4260

tgcattttaa cacgtgatat tgcagatgaa gttgaaagcc ttgctgctgc cagacaggct 4320

gccatatctg gttctgagcc ttcagactcc attagggtaa agttttcagc agttttcctt 4380

tgccatacac cttttatcag gtaatgttta cactgtcttc tttcactcta atgttcaggt 4440

ggtaaatgcc ttgctaacac aaatggacaa actgaaggct tggccaaatg ttatcatttt 4500

gacgacatca aatatcacca cagctatcgg tattgatttt tcgccttttg agttttttcc 4560

ttttatgacc tgaatggccc atcatgccat tttgtgtgca tagttgccac attttttgca 4620

tgattatctg cgcgtttgga acaacggtgc tgtatatgat caacatctgg acaaatttgg 4680

acatttagta tgtgttttag ttggtcaagt gaatcactgc agtgagggaa agatcaattg 4740

cataataagc atgtttatgt ccgtggagct gtctatgttt ataacatcac ttttcctact 4800

tttctgaagt aactaatgct acattattga tacatttaaa tttagataat cttgttgcca 4860

tcgacaatga gatatcaact gtgctcagta ctgatgtctc ttgggctaat aaaattgcgt 4920

aattgcagac attcattcta ttttcgatgg ctgttaagga gaacagcctg tagccttctt 4980

aaacatcgtg tagacatgca ttgagtccta aaatcaataa attctaactc gtatgtggtt 5040

tgcagatatt gcatttgttg acagggcaga catcaaggca tacgtcggac cccctactct 5100

tcaagcacgc tatgagattt tgagatcatg catagatgaa ctcttccgag taggaattct 5160

tacatatcct caggtatttg ttcctttttg ccaacttggt tgctgtaata ccaccgctgt 5220

atcactgtga aggttgcttg gaactgcttt gcacatgggg cactttttct gagcaaacct 5280

gtatttggga cctcaaagaa tttatttgtt tgaactgctg tatagaattt ccagctttct 5340

tgcaatcttt aaaggattat tcctctccaa atacagggtg gtgatttgcc ttgcattctg 5400

agctattcaa ccctgaaaga gaagttgcac tgtcctgagg ctgcagaacc ccatacactt 5460

catctctcca aattactgca tgaaggcgca gaactgtgcg acgtaagtga tgaagtttac 5520

agttgcctta cctttttatt gctgtagatt aagtgatctg ctgtaatcaa caccaacagg 5580

ctgataatgg tggtgatctt ttttccattc tcccacaggg attgagcgga agatcattga 5640

gaaaacttcc tttcctggca cacgcttctg ttcccaaccc ttcgtgctgt gacgctgctt 5700

ctttcatgca cacgctgata cagaccgcgc gaagagagct cttggaatca cgcggatgat 5760

tcctcgcgta ttgcttggag gagcaaatgg ttaacggttg gatacgagta ccttgccact 5820

gatatgatgc atagtttgag ctaattcgaa gtctgttgtt ctgagataca agcaaatcca 5880

ttccatttct aggtctagag aagtaggaag tccaggatat aggtgccgtt gctgttgcat 5940

tacttttggt tctcctgtaa attgttacgg tgggatgcac tacacctcga gggacgccgc 6000

attactacct acatacatga agtttctctt ttgcggggat ttacctacat gagtgccata 6060

catacttcgg tgacttgatc agatctgttt ctggtcattg gaacttgaaa aggatagcgt 6120

ttgtgtacct tattagcttt gtttctgttg ttctggtttt gcatgttatg tgcaggatgt 6180

aaatcaaagt tggctggact gtttggcttg gcctcagccc ttcaggctta ctctcttcag 6240

tcgcgcgcag aaagctgaaa cgagtgccaa ggtacatgac cgcgcattcc tgtgatggga 6300

atatccaaga gagtttattc ttctctactg gtgccaaggg aggttagtgc agactgcaga 6360

agctagggta aagtctcaac accgcagcta agcttcagtg attccaaaac agcaagagtc 6420

attataacat ccctgattga accgtcaagt cattaaccaa cagttgctcc tatcaattcg 6480

agcatccagc caaagaaaat aaggagagat acaaatcaaa agactcagac cataaactgc 6540

gactcttggc aacttgatgg cgtgcgtaaa gctacaggga gtgaatgtac atgaaggaaa 6600

taagagtcat gacaatgttt atataaaatt tgcaggcgca caggccatct tcaggttaga 6660

accccggatt tcacctgtgc tggtctggtt gggtcataca gctcatacta cgttacagga 6720

taaagagctg gggatgcttg cccctgaaca ctgaacgata acggtgtcga gtagtagatc 6780

aaacttctag tgacaaaatt gaatagtgtg acacaactaa agggcatgga acggctggtg 6840

tgtttcgatt tagttattcc cgagttatac atgtgaaaag accgctcaaa taactacatc 6900

atataattat tttttaaaat ttatataatg attttattaa aaaataatta agtgttgaa 6959

<210> 35
<211> 1649
<212> DNA
<213> Artificial Sequence

<220>
<223> cDNA of pch2 from Triticum aestivum genome A

<400>  35
atgagcgctc ccatggagat ctccttcgac ccccaccctt ctcatcccgc cccgcccgcc      60

ttcccctcca gcccacccct ggagcccgca gccgccgcca cccctcccgc ggtctcgtcc     120

tcgccgcagc cgacggcgga ggccgtagtc gcgcccaccc cgccagacga caaggtcctc     180

gtctcagttg aggtgttgct gcacgccacg agtacggctc gcgccgagga cgtccgaacg     240

gcggtcgaga ggatgttaga atctcgaagt ttaagctatg tcgatggtcc tgttcagata     300

cctccagaca attcttttct tcttgaacat gtcaaaagga tccaaatatg tgacactgat     360

gagttgattg agaaccacaa aatcctgttg ttttggcaag tcaaacctgt cgttcatgta     420

ttccagttga gtgaggatgg tccaggtgaa gaaccaagtg aagacgacac actctctagc     480

ttcaatgagt gggctctacc tgccaaagaa tttgatggat tgtgggaaag cttactatat     540

gaagtgggtc tcaagcagag gttgctgaga tatgctgcta gtgctttgct atttactgaa     600

agaggtgtag acccatgcct tgtttcatgg aacaggattg tacttttaca tggtccacct     660

ggaacaggaa agacttcatt atgtaaagca ctagctcaga agctttctat tcgcttcaat     720

tcaaggtatt ctatgtgcca actaattgaa gtcaatgccc attcattgtt cagcaagtgg     780

ttttctgaaa gtgggaaact ggtagctaaa cttttccaga agattcagga gatggtggag     840

gaagaaagca acctggtatt tgtattgatc gatgaagttg aaagccttgc tgctgccaga     900

caggctgcca tatctggttc tgagccttca gactccatta gggtggtaaa tgccttgcta     960

acacaaatgg acaaactgaa ggcttggcca aatgttatca ttttgacgac atcaaatatc    1020

accacagcta tcgatattgc atttgttgac agggcagaca tcaaggcata cgtcggaccc    1080

cctactcttc aagcacgcta tgagattttg agatcatgca tagatgaact cttccgagta    1140

ggaattctta catatcctca gggtggtgat ttgccttgca ttctgagcta ttcaaccctg    1200

aaagagaagt tgcactgtcc tgaggctgca gaaccccata cacttcatct ctccaaatta    1260

ctgcatgaag gcgcagaact gtgcgacgga ttgagcggaa gatcattgag aaaacttcct    1320

ttcctggcac acgcttctgt tcccaaccct tcgtgctgtg acgctgcttc tttcatgcac    1380

acgctgatac agaccgcgcg aagagagctc ttggaatcac gcggatgatt cctcgcgtat    1440

tgcttggagg agcaaatggt taacggatgt aaatcaaagt tggctggact gtttggcttg    1500

gcctcagccc ttcaggctta ctctcttcag tcgcgcgcag aaagctgaaa cgagtgccaa    1560

ggtacatgac cgcgcattcc tgtgatggga atatccaaga gagtttattc ttctctactg    1620

gtgccaaggg aggttagtgc agactgcag                                      1649

<210>  36
<211>  475
<212>  PRT
<213>  Triticum aestivum

<400>  36

Met Ser Ala Pro Met Glu Ile Ser Phe Asp Pro His Pro Ser His Pro
1               5                   10                  15

Ala Pro Pro Ala Phe Pro Ser Ser Pro Pro Leu Glu Pro Ala Ala Ala
            20                  25                  30

Ala Thr Pro Pro Ala Val Ser Ser Ser Pro Gln Pro Thr Ala Glu Ala
        35                  40                  45

Val Val Ala Pro Thr Pro Pro Asp Asp Lys Val Leu Val Ser Val Glu
    50                  55                  60

Val Leu Leu His Ala Thr Ser Thr Ala Arg Ala Glu Asp Val Arg Thr
65                  70                  75                  80

Ala Val Glu Arg Met Leu Glu Ser Arg Ser Leu Ser Tyr Val Asp Gly
                85                  90                  95

Pro Val Gln Ile Pro Pro Asp Asn Ser Phe Leu Leu Glu His Val Lys
            100                 105                 110

Arg Ile Gln Ile Cys Asp Thr Asp Glu Leu Ile Glu Asn His Lys Ile
        115                 120                 125

Leu Leu Phe Trp Gln Val Lys Pro Val Val His Val Phe Gln Leu Ser
    130                 135                 140

Glu Asp Gly Pro Gly Glu Glu Pro Ser Glu Asp Asp Thr Leu Ser Ser
145                 150                 155                 160

Phe Asn Glu Trp Ala Leu Pro Ala Lys Glu Phe Asp Gly Leu Trp Glu
                165                 170                 175

Ser Leu Leu Tyr Glu Val Gly Leu Lys Gln Arg Leu Leu Arg Tyr Ala
            180                 185                 190

Ala Ser Ala Leu Leu Phe Thr Glu Arg Gly Val Asp Pro Cys Leu Val
        195                 200                 205

Ser Trp Asn Arg Ile Val Leu Leu His Gly Pro Pro Gly Thr Gly Lys
    210                 215                 220

Thr Ser Leu Cys Lys Ala Leu Ala Gln Lys Leu Ser Ile Arg Phe Asn
225                 230                 235                 240

Ser Arg Tyr Ser Met Cys Gln Leu Ile Glu Val Asn Ala His Ser Leu

245                 250                 255

Phe Ser Lys Trp Phe Ser Glu Ser Gly Lys Leu Val Ala Lys Leu Phe
            260                 265                 270

Gln Lys Ile Gln Glu Met Val Glu Glu Glu Ser Asn Leu Val Phe Val
        275                 280                 285

Leu Ile Asp Glu Val Glu Ser Leu Ala Ala Ala Arg Gln Ala Ala Ile
    290                 295                 300

Ser Gly Ser Glu Pro Ser Asp Ser Ile Arg Val Val Asn Ala Leu Leu
305                 310                 315                 320

Thr Gln Met Asp Lys Leu Lys Ala Trp Pro Asn Val Ile Ile Leu Thr
                325                 330                 335

Thr Ser Asn Ile Thr Thr Ala Ile Asp Ile Ala Phe Val Asp Arg Ala
            340                 345                 350

Asp Ile Lys Ala Tyr Val Gly Pro Pro Thr Leu Gln Ala Arg Tyr Glu
        355                 360                 365

Ile Leu Arg Ser Cys Ile Asp Glu Leu Phe Arg Val Gly Ile Leu Thr
    370                 375                 380

Tyr Pro Gln Gly Gly Asp Leu Pro Cys Ile Leu Ser Tyr Ser Thr Leu
385                 390                 395                 400

Lys Glu Lys Leu His Cys Pro Glu Ala Ala Glu Pro His Thr Leu His
                405                 410                 415

Leu Ser Lys Leu Leu His Glu Gly Ala Glu Leu Cys Asp Gly Leu Ser
            420                 425                 430

Gly Arg Ser Leu Arg Lys Leu Pro Phe Leu Ala His Ala Ser Val Pro
        435                 440                 445

Asn Pro Ser Cys Cys Asp Ala Ala Ser Phe Met His Thr Leu Ile Gln
    450                 455                 460

Thr Ala Arg Arg Glu Leu Leu Glu Ser Arg Gly
465                 470                 475

<210>  37
<211>  6957
<212>  DNA
<213>  Triticum aestivum

<400> 37
tcacatttgg aaaaaataat cattttttgc attgatttga aaaatgttca cgcacttaag 60

agaagataaa aatggaagtc aaggagggag gaaaaccagg aagaagaaca aaaaagaaaa 120

aagaagcaga aaatgaaaac caaaaaaaga gaaaggaaag tataaaagaa tgaaagagtt 180

gaatctgaga agatgccgct ccatagcggg gtgggtaata cgtcgaacct cagcgagcga 240

ggtggtgggt tcgatttgca aagctcaatt atttattgcg gatttaaata caccgtaatg 300

gctcggccca gcttgacacg ctgcaggcgc ccgtttgcaa aaatacaggc gcctgcagcg 360

tcaaatagga ttcgccacca catttaccta ccccggaaaa aaaaagacca cacttgtttt 420

ttgagcaact ataggaaaga ccacatatgc atactgctgg gtcgatgagg atttggtcaa 480

aaacatgggt gcccattgag cccatcaaat agaagcccat ctgctgaccc taaaggtacc 540

ccaagcgcgg gaaacccaat tcctctcaac tctcatagtc tctccttccc ccaaaccccc 600

attcctccac ggctccgcct tcccctcccc tcgccaaacc caaaacccta gaaccttctc 660

cattccgctc cctcgcggag cctggccacc atgagcgctc ccatggagat ctccttcgac 720

ccccaccctt ctcatcccgc cctgcccgcc ttcccctcca gcccacccct ggagcctgca 780

gccgccgcca cccctcccgc ggtctcgtcc tcgccgcagc cgtcggcgga ggccgtagtc 840

gcgcccaccc cgccagacga caaggtcctc gtctcaggta ggcgccgcga cgcgcacgca 900

tttctggcct cgccctagcg gcgcgtgatt aaaccgtgct ctgcgaatgc ccgatttggt 960

ttcgccgggg ttgatttttt ccttttgcgc ttgcagttga ggtgctgctg cacgccacga 1020

gtacgtctcg cgccgaggac gtccgagcag cggtcgagag gtcggtgctg ttccagttac 1080

actttgtttc gagcatattt tactgttgtt tgcatgcttg atgacacgtg gatgcatgag 1140

ctgctaattg cgggttggat tagatgtagt ttggcactgg aaaagtgaaa atgtgccatg 1200

attctaggga acgtggtcaa caatcatcct gtgatttagc agtggagttt gctgggcatg 1260

ttttagtagg aaatactaac agacatgttc gttcactaat cgctcatgat gcagcatatt 1320

ggaggtcgac gtggcaacta gcaaagttag gagtactaaa ttgtttgtaa taggcaatta 1380

ggcattgcag ggtgtttagt attttagaga aaaaatacca gataactttt aagttttaca 1440

acagattagc aactaggtgg ggatttctgc tgtgtaacat ggatataagc ccgatcagtt 1500

aaaaaatgac tatgttcacc atttgcttgt acaaatgatg gaaaaagctg taaatgaagt 1560

taaaaaatcc tatcatatat agtgttctgt cagaaaagat cactggaaac acatatataa 1620

gccaatcatt ccaaaaaaat atggtgttca actcttcatc attgcttatt gagaggctgg 1680

ataactcacc aaatcaattc cagaaatctt atcatcgctt ctttcatatt aaagtgctaa 1740

tcagatccga ggccatggaa ttgacagagt ttcttgaaga cagcttcttg gtttaagctc 1800

atagcatata tattgaactt gcacggttat cttctcttgc aggacattaa tgtttgttat 1860

tgctcatact tgacatttac aagtcgttct cttatcagga tgttagaatc tcgaagttta 1920

agctatgttg atggtcctgt tcagatacct ccagacaatt cttttcttct tgaacatgtc 1980

aaaaggatcc aaatatgtga cactggtatg atgtttcact taggctggaa agtgtgcttt 2040

cttgatctca gttatctatg tacatacctc ttcctgaatg ctcgcttttc atgtagatga 2100

gttgattgag aaccacaaaa tcctttgttt ttggcaagtc aaacctgtcg ttcatgtatt 2160

ccaggtatgc atctaaattt tttctgtcta aatcaattaa atctttgttg cttaaatctc 2220

ttctactccc tccgttccta aacatttgtc tttctagaga tttcaacaaa tgactacata 2280

gtacatacgg agcaaaatga gtgaatctac actctaaaat atgtatatat acatccgtat 2340

gtggtagtcc atttgaaatc tctaaaaaga caaatattta ggaacggagg gagtagttcc 2400

ttttaaaaca cagatggtct ttgggtatgt ttacatgcta ttgccggcta tctttcttct 2460

ttgagcaagt gcctgttgga attgtttcat cttcacatca taaaatatct ttgtaatgct 2520

gctgaggtac cggtgttttg ttgttttcac ttaaaatttc acattgtttt ctcttgaaac 2580

aaccggagtg attttctaaa ttagactact gacctctttc caagagatag attactttat 2640

gtcatgttct aagtgaacat ttggttcaaa tttatcatac acctcaaggc tcaaactgat 2700

gtagttatct tgcagttgaa tgaggatggt ccaggtgaag aaccaagtga agatgacaca 2760

ctctctagct tcaatgagtg ggctctacct gccaaagaat ttgatggatt atgggaaagg 2820

tttggtttga tctcattatc ctttaccagt tacaatttct gtgtttttta ttctgttggt 2880

gatgttgtat gtgcttgcag cttactatat gaagtgggtc tcaagcagag gttgctgaga 2940

tatgctgcta gtgctttgct atttactgaa agaggtgtag acccatgcct tgtttcatgg 3000

aacaggtaag catctttcac atcgttaagc acttggaggc ttcacatgtc atcagaagta 3060

gcatattttg gtttttcatt ccacaataaa gttggtcatt actgccatgg aactttaggg 3120

tacctttttg tgtagttgga tttgcagtta gcttctgata catttcaatg acgttcatga 3180

gtacttcctg aaattgtatc tggacagtgg accattgtct ccttcctctt cttcagttct 3240

tccttgagtc tattggtgga tattgttcgt tgggaagtta atcaaggtga atgtgcaaaa 3300

tactgcaaaa aagaattgca gcttaattgt caagggatac taattggatg caaaagcctg 3360

agcttcgtga aactgaacta cttacctttt aatacccatg caaatgtgca acaacaacaa 3420

caacaatgac aaagccttta gtcccaaaca agttggggta ggctagagcg gaaacccatg 3480

caaatgtgca ctttcagtta tataaattat tctctaggaa tataatgatt tattttgttt 3540

tgtattgttt atgtactttt ttacatacaa ttgcaggatt gtacttttgc atggtccacc 3600

tggaacagga aagacttcat tgtgtaaagc actagctcag aagctttcta ttcgcttcaa 3660

ttcaaggttc ttgcttaatg acattcatga ttaatgtttt aataaataat gtgttattaa 3720

ttactctctt tttcaccagg tattctatgt gccaactaat tgaagtcaat gcccattcat 3780

tgttcagcaa gtggttttct gaaagtggga aactggtatg cctttttgtg cttatatcaa 3840

taatattatc atgtatatgt tcatgctaaa atcacatcat cagctcatgg taattgagtt 3900

ttgttatgtt tttgtgtcat tttgcacgat gtggaaaatt caccatcaat gacacgtttt 3960

gcagtatgta gtttgtttag cccactaaaa ttgtccatcg gagatacatc ttcattttcg 4020

tcttacaatg tacatactat aatttgtcct ttttaggtag ccaaactttt ccagaagatt 4080

caggagatgg tggaggaaga aagcaacctg gtatttgtat tgatcggtat gttcatggca 4140

aaactggtga aatgactaga tgttcaatct ccgtatgatc tgactagctc aatccatgcc 4200

ttcatcttat ctatgcattg ttaaactgac ttctcattcc aatgtgttct ttactttcag 4260

aaatgcctca atactaatga ataatgaacc ccattgagct acatgacata tgggattttg 4320

tcctttgtgt gaatagagag aacatagatc ataaaatgtt cttaaaatcg aaaccaacac 4380

aatttaattc cctaggcatt atctcctagt tgcagattag cgaaatgcac tgtggcactc 4440

ctatagaatt ttcactgaac tttttgactt tagataaatt tgattagcag catgctgtat 4500

ttgaactctt aatttatcaa tggcgtttat gcattttaac acgtgatatt gcagatgaag 4560

ttgaaagcct tgctgctgcc agacaggctg ccatatctgg ttctgagcct tcagactcca 4620

ttagggtaaa gttttcatca gttttccttt gccatatact accttttatc aggtaatgtt 4680

tacactgtct tctttcactc tgtaatgttc aggtggtaaa tgccttgcta acacaaatgg 4740

acaaactgaa ggcttggcca aatgttatca ttttgacgac atcaaatatc accacagcta 4800

tcggtattga ttttccgcct tttgagtttt tttcctttta tgacctgaat ggcccatcat 4860

gccattttgt gtgcatagtt gccacatttt ttgcatgatt atctcattat ctgcacgttt 4920

ggaacaacgg tgctgtatat gatcaacatc tggacaaatt tggacattta gtatgtgttt 4980

tagttggtca agtgaatcac cgcaatgagg ggaagatcaa ttgcataata ggcacgttta 5040

tgtttgtgga gctgtctatg tttataacat cacttttcat acttttctga agtaactaat 5100

gctacattat tgatacattt aaatttagat aatcttgtgc catcgacaat gagatatcaa 5160

ctgtgctcag tactgacgtc tcttgggcta ataaaattgc gtaattgcgg acattcattc 5220

tattttttat ggctgttaag tgaacagcct gcagctttct tagacatcgt gtagacatgt 5280

attgattcct aaaatcagta aattctaact cgtatgtggt ttgcagatat tgcatttgtt 5340

gacagggcag acatcaaggc atacgtcgga ccccctactc ttcaagcacg ctatgagatt 5400

ttgaaatcat gcatagatga actcttccga gtaggaattc ttacatatcc tcaggtattt 5460

gttccttttt gccaacttgg ttgctgcaat gctgcctttt ttttcactta agcatcacgt 5520

gaaggttgct tgggactgct ttgctcatgg ggcacgttct ctaagcaaac ctggatttgg 5580

gacctcaaac agtgcgtttg tttgaacttc tgtatagaat ttccagctcg caatctttaa 5640

aggattattc ctctccaaat acagggtggt gatttgccct gcattctgag ctattcaacc 5700

ctgcaagaga agttgcactg tcctgaggct gcagaacccc atacacttca tctctccaaa 5760

ttactgcatg aaggtgcaga actgtgcgac gtaagtgatg aagtttacag ttgccttacc 5820

ttttcatgta gattaagcga tctgctgtaa cccacaccaa catgctgctt atggtggtga 5880

tcttttttcc attctcccac agggattgag cggaagatca ttgagaaaac ttcctttcct 5940

ggcacacgct tccgttccca acccttcgtg ctgtgacgct gcttctttca tgcacacgct 6000

gatacagact gcgcggagag agctcttgga atcacgcggc tgattccctg tggattgcct 6060

ggaggagcaa gtggctaaca gtttgataca agtaccatgt cgccgatacg atgcatagtt 6120

tgagctaatt cgaagtctgg tgttgcgaga tacaagcaaa tccattccat ctctgggtct 6180

agaacagtag gaagtccagg atataggttc cgttgttgtt gcattgcttt tggttctcct 6240

gtaaattgtt acggtaggat gcactacacc tcgagggacg ccgcattatt acctagatac 6300

atttaatttt tcttttgtgg ggatttacct acatgaacgc catacttcgg tgacttgatc 6360

agatctgttt ccggtcattg gaacttgaaa tgaatagcgt ttatctacct tattagcttg 6420

tttctgttat tattgccggt tttgcatgtt atgtgcaaga tgtaaatcaa agttgactgg 6480

actgattggc ttggcctcag ctcaacctct tcagtcgtgc acagaaagct gaaacgagta 6540

gcaaaattat aacatccctg accgtgcatt cctgtgacac aactaaaggg catggaacgg 6600

ctggtgtgtt tcgatttagt tattcccgag ttctacatgt gaaaagaccg ctaatataac 6660

tacatcatat aatgattctt ttgaacttat ataatgattt tatttaataa taataataat 6720

tgttaaatca ccattctagc tctgttcctg ccaagcgtgc aacttagata atgatctagc 6780

agagcatata atataagatg ctttttgaca ctagtgtagt gtcaaaaaaa tgtcttacat 6840

tacgggacag agggagtagt tttctttgaa atgtcatcaa actgctgaac tatttcacat 6900

gtattttgtt tgatgactac catgtttttg attttgaatt atgactgatg aatatta 6957

<210> 38
<211> 1832
<212> DNA
<213> Artificial Sequence

<220>
<223> cDNA of pch2 from Triticum aestivum genome B

<400> 38
attcctccac ggctccgcct tcccctcccc tcgccaaacc caaaacccta gaaccttctc 60

cattccgctc cctcgcggag cctggccacc atgagcgctc ccatggagat ctccttcgac 120

ccccaccctt ctcatcccgc cctgcccgcc ttcccctcca gcccacccct ggagcctgca 180

gccgccgcca cccctcccgc ggtctcgtcc tcgccgcagc cgtcggcgga ggccgtagtc 240

gcgcccaccc cgccagacga caaggtcctc gtctcagttg aggtgctgct gcacgccacg 300

agtacgtctc gcgccgagga cgtccgagca gcggtcgaga ggatgttaga atctcgaagt 360

ttaagctatg ttgatggtcc tgttcagata cctccagaca attcttttct tcttgaacat 420

gtcaaaagga tccaaatatg tgacactgat gagttgattg agaaccacaa aatccttttg 480

ttttggcaag tcaaacctgt cgttcatgta ttccagttga atgaggatgg tccaggtgaa 540

gaaccaagtg aagatgacac actctctagc ttcaatgagt gggctctacc tgccaaagaa 600

tttgatggat tatgggaaag cttactatat gaagtgggtc tcaagcagag gttgctgaga 660

tatgctgcta gtgctttgct atttactgaa agaggtgtag acccatgcct tgtttcatgg 720

aacaggattg tacttttgca tggtccacct ggaacaggaa agacttcatt gtgtaaagca 780

ctagctcaga agctttctat tcgcttcaat tcaaggtatt ctatgtgcca actaattgaa 840

gtcaatgccc attcattgtt cagcaagtgg ttttctgaaa gtgggaaact ggtagccaaa 900

cttttccaga agattcagga gatggtggag gaagaaagca acctggtatt tgtattgatc 960

gatgaagttg aaagccttgc tgctgccaga caggctgcca tatctggttc tgagccttca 1020

gactccatta gggtggtaaa tgccttgcta acacaaatgg acaaactgaa ggcttggcca 1080

aatgttatca ttttgacgac atcaaatatc accacagcta tcgatattgc atttgttgac 1140

agggcagaca tcaaggcata cgtcggaccc cctactcttc aagcacgcta tgagattttg 1200

aaatcatgca tagatgaact cttccgagta ggaattctta catatcctca gggtggtgat 1260

ttgccctgca ttctgagcta ttcaaccctg caagagaagt tgcactgtcc tgaggctgca 1320

gaaccccata cacttcatct ctccaaatta ctgcatgaag gtgcagaact gtgcgacgga 1380

ttgagcggaa gatcattgag aaaacttcct ttcctggcac acgcttccgt tcccaaccct 1440

tcgtgctgtg acgctgcttc tttcatgcac acgctgatac agactgcgcg gagagagctc 1500

ttggaatcac gcggctgatt ccctgtggat tgcctggagg agcaagtggc taacagtttg 1560

atacaagtac catgtcgccg atacgatgca tagtttgagc taattcgaag tctggtgttg 1620

cgagatacaa gcaaatccat tccatctctg ggtctagaac agtaggaagt ccaggatata 1680

ggttccgttg ttgttgcatt gcttttggtt ctcctgtaaa ttgttacggt aggatgcact 1740

acacctcgag ggacgccgca ttattaccta gatacattta atttttcttt tgtggggatt 1800

tacctacatg aacgccatac ttcggtgact tg 1832

<210> 39
<211> 475
<212> PRT
<213> Triticum aestivum

<400> 39

Met Ser Ala Pro Met Glu Ile Ser Phe Asp Pro His Pro Ser His Pro

1               5                   10                  15

Ala Leu Pro Ala Phe Pro Ser Ser Pro Pro Leu Glu Pro Ala Ala Ala
            20                  25                  30

Ala Thr Pro Pro Ala Val Ser Ser Ser Pro Gln Pro Ser Ala Glu Ala
        35                  40                  45

Val Val Ala Pro Thr Pro Pro Asp Asp Lys Val Leu Val Ser Val Glu
    50                  55                  60

Val Leu Leu His Ala Thr Ser Thr Ser Arg Ala Glu Asp Val Arg Ala
65                  70                  75                  80

Ala Val Glu Arg Met Leu Glu Ser Arg Ser Leu Ser Tyr Val Asp Gly
                85                  90                  95

Pro Val Gln Ile Pro Pro Asp Asn Ser Phe Leu Leu Glu His Val Lys
            100                 105                 110

Arg Ile Gln Ile Cys Asp Thr Asp Glu Leu Ile Glu Asn His Lys Ile
        115                 120                 125

Leu Leu Phe Trp Gln Val Lys Pro Val Val His Val Phe Gln Leu Asn
    130                 135                 140

Glu Asp Gly Pro Gly Glu Glu Pro Ser Glu Asp Asp Thr Leu Ser Ser
145                 150                 155                 160

Phe Asn Glu Trp Ala Leu Pro Ala Lys Glu Phe Asp Gly Leu Trp Glu
                165                 170                 175

Ser Leu Leu Tyr Glu Val Gly Leu Lys Gln Arg Leu Leu Arg Tyr Ala
            180                 185                 190

Ala Ser Ala Leu Leu Phe Thr Glu Arg Gly Val Asp Pro Cys Leu Val
        195                 200                 205

Ser Trp Asn Arg Ile Val Leu Leu His Gly Pro Pro Gly Thr Gly Lys
    210                 215                 220

Thr Ser Leu Cys Lys Ala Leu Ala Gln Lys Leu Ser Ile Arg Phe Asn
225                 230                 235                 240

Ser Arg Tyr Ser Met Cys Gln Leu Ile Glu Val Asn Ala His Ser Leu
                245                 250                 255

Phe Ser Lys Trp Phe Ser Glu Ser Gly Lys Leu Val Ala Lys Leu Phe
            260                 265                 270

Gln Lys Ile Gln Glu Met Val Glu Glu Glu Ser Asn Leu Val Phe Val
        275                 280                 285

Leu Ile Asp Glu Val Glu Ser Leu Ala Ala Ala Arg Gln Ala Ala Ile
    290                 295                 300

Ser Gly Ser Glu Pro Ser Asp Ser Ile Arg Val Val Asn Ala Leu Leu
305                 310                 315                 320

Thr Gln Met Asp Lys Leu Lys Ala Trp Pro Asn Val Ile Ile Leu Thr
                325                 330                 335

Thr Ser Asn Ile Thr Thr Ala Ile Asp Ile Ala Phe Val Asp Arg Ala
            340                 345                 350

Asp Ile Lys Ala Tyr Val Gly Pro Pro Thr Leu Gln Ala Arg Tyr Glu
        355                 360                 365

Ile Leu Lys Ser Cys Ile Asp Glu Leu Phe Arg Val Gly Ile Leu Thr
    370                 375                 380

Tyr Pro Gln Gly Gly Asp Leu Pro Cys Ile Leu Ser Tyr Ser Thr Leu
385                 390                 395                 400

Gln Glu Lys Leu His Cys Pro Glu Ala Ala Glu Pro His Thr Leu His
                405                 410                 415

Leu Ser Lys Leu Leu His Glu Gly Ala Glu Leu Cys Asp Gly Leu Ser
            420                 425                 430

Gly Arg Ser Leu Arg Lys Leu Pro Phe Leu Ala His Ala Ser Val Pro
        435                 440                 445

Asn Pro Ser Cys Cys Asp Ala Ala Ser Phe Met His Thr Leu Ile Gln
    450                 455                 460

Thr Ala Arg Arg Glu Leu Leu Glu Ser Arg Gly
465                 470                 475

<210>  40
<211>  6949
<212>  DNA
<213>  Triticum aestivum

<400>  40
tttctttttc ttctgatttt ttcatttctt tttctaggtt ttctgatttt tttctctctc        60

atttttttat caaaaactat taacatagat ctactctact ccctccgctc cataatgtaa 120

gacatttttt gacactaaat gtcttacatt gggacggagg gagtagtttt gaagatctcg 180

aaatgagaaa tccaatagtg aaaatagttt agaagataaa aacattttga ataaatgaat 240

taaaaaagga aaaaacttcc aagttataac aagtgacaca gtaagaacct aagaaggtag 300

ggagtggcct ttgcgagggt taactcttag tgatttttct ctatttaaca cagagagcgt 360

caaatacgag agaccacatt taccccgcaa aaaaaaaaac acatttgttt tttgagcaac 420

tatagggaag accgcatgct gctgggtcga taaggatttg gtcaaaaaca tgggtgccca 480

ttgagcccat ccaggtctct aagcccaccc agagcttaca gaaaatttca ctggaagtca 540

aatagaagcc catctcctga ccctaaaggt accccaagcg cgggaaaccc aaatcctctc 600

acagcctcct tccccaaaat cccccattcc tccacggctc cgccttctcc tcccctcccc 660

aaacccaaaa ccctagaacc ttctccattc cgctccctcg cggagcttgg ccaccatgag 720

cgctcccatg gagatctcct tcgaccccca cccttctcat cccgcgctgc ccgccttccc 780

ctccagccca cccctggagc ccgcagccgc cgccacccct cccgcggtct cgtcctcgcc 840

gcagccgacg gcggaggccg tagtcgcgcc caccccgcca gacgacaagg tcctcgtctc 900

aggtaggcgc cgcgacgcgc acgcatttct ggcctcgccc tagcggcgcg tgattaaacc 960

gtgctctgcg aatgcccgat ttggtttccc cggggtttat tttttccttt tgtgcttgca 1020

gttgaggtgc tgctgcacgc cacgagtacg gcccgcgccg aggacgtccg agtagcggtc 1080

gagaggtcgg tgctgttcgg gttaaacttt gtttcgagct tattttactg ttgttgaatg 1140

cttgatgaca cgtggatgca tgagctgcta aatgcgggtt ggattagatg tagtttggca 1200

ctggaaaagt ggaaatgtgc catgattgta gggaacgtga ttaacaatca tcctgtgatt 1260

tagcagtgga gtttgctgag tatgttgtag taggaaataa cagacatgtt tgtttcataa 1320

tcgctgatgg tgcagcatat tggagattga cgtggcaact agcaaggtta ggagtactaa 1380

attgtttgta ataggcaatc aggcattgca gggtgattgg tattttagag aaaaatacaa 1440

gctaactttt aagttttaca acagattagc cattaggtgg ggatttctgc tgtgtaacat 1500

ggatataagc cgatcattta aaaaatgact gtgttcacca tttgcttgta caaatgatgg 1560

agaaagctgt aaatgaagtt aaaaaaatcc tatcatctgt agtgttctgt tcagaaaaga 1620

tcactggaaa cacagatata agccaatcat tccaaaacaa tatcgcgttc aactgtttat 1680

cattgcttat tgagaggctg gatagctcac caaatcattc cagaaatctt atcatcactt 1740

tctttcatat taaagtgctg atcagatctg aggccatgga attgacagag tttcttgtag 1800

acagcttatt ggtttaagct catagcatat atatcttgca cggttatctt ctctcgcagg 1860

acattaatgt ttgttattgc tcatacttaa catttacaag ttgttctctt atcaggatgt 1920

tagaatctcg aagtttaagc tatgttgatg gtcctgttca gatacctcca gacaattctt 1980
ttcttcttga acatgtcaaa aggatccaaa taagtgacac tggtatgatg tttcacttag 2040
gctggaaagt gtgctttctt gatctcagtt atctatgtac ctcttcctga atgcccgctt 2100
ttcatgtaga tgagttgatt gagaaccaca aaatcctgtt gttttggcaa gtcaaacctg 2160
tcgttcatgt attccaggta tgcatctaaa cgttttctgc ttaaatcact taaatctttg 2220
ttgcctaaat ctcttgtagt tccttttaaa acacagatgg tctttgggta tgtttacatg 2280
ctattgccgg ctatctttct tctttgatca attgcctgtt ggaattgtta catcataaga 2340
tatctttgta atgctgctga ggtactggtg ttttgttgtt ttcacttaaa atttcacatt 2400
tgttttctct tgaaacaacc ggagtgattt tctaaattag actaccgacc tctttccaag 2460
agatagatta cttcatgtca tgttctaagt gaacatttgg ttcaaattta tcatacacct 2520
caaggctcaa gctgatctgg ttatcttgca gttgaatgag gatggtccag gtgaagaacc 2580
aagtgaagat gacacactct ctagcttcaa tgagtgggcg ctacctgcca aagaatttga 2640
tggattatgg gaaaggtttg gtttgatctc attatccttt accagttaca atttctgtgt 2700
tttttattct gctggtgatg tcgtatgtgc ttgcagctta ctatatgaag tgggtctcaa 2760
gcagaggttg ctgagatatg ctgctagtgc tttgctattt actgaaagag gtgtagatcc 2820
atgccttgtt tcatggaaca ggtaagcatc tttcacatca ttaagcactt ggaggcttca 2880
catgtcatca gcagtagcat attttggttt tactttccac aataaagtta gtcattattg 2940
ccatggaact ttagggtacc tttttgtgta gttgggtttg cagttagctt ctgaaacctt 3000
ttaatgacgt tcatgagtac ttcctctgta aagaaatata agagcgttga gatcacttta 3060
cagagggagt acttcctgaa attgtatctg gacagtggac cattgtctgc ttcctcttct 3120
tcagtttttc cttgagtcta ttggtggata ttgtttgttg ggatagttaa tcaaggtgaa 3180
tgtgtagaat actgcaaaaa agtattgcag cttaattgtc aagggatact cattggatgc 3240
aaaagcctga gcttcgtgaa actgaactac ttgccttcta atacccatgc aaatgtgcaa 3300
caaccacaac aacaacaaca tagcctttag tcccaaacaa gttggggtag gctagagctt 3360
gaaacccatg caaatgtgca ctttcagtta tataaattat tctctaggat tatatgattt 3420
attttgtttt gtattgttta tgtacttttt tacataccat tgcaggattg tacttttgca 3480
tggtccacct ggaacaggaa agacttcatt gtgtaaagca ctagctcaga agctttctat 3540
tcgcttcaat tcaaggttct tgcttaatga tatttatgac taatgtttta ctccctccat 3600
cccataatgc aagacgtttt ttgacacgtc ttaaaattat gggatggagg gagtgataaa 3660
ttatgtgtta ttaattactg tctctttttc accaggtact ctatgtgcca actaattgaa 3720
gtcaatgccc attcattgtt cagcaagtgg ttttctgaaa gtggcaaact ggtatgcctt 3780
tttgtgctta tatcaataat attattatgt ataagttcat gctggaatca catcatcagc 3840

tcgtggtaat tgagttttgt tatgtttttg cacccttttg cacgatgtgg acaattcacc 3900

atcatatgat acattttgta gtatgtactt agtttagccc actaaaattg tccattggag 3960

atacatcttc atttccgtct tacaatgtac atactataat ttgacctttt caggtagcca 4020

aacttttcca gaagattcag gagatggttg aggaagaaag caacctggta tttgtattga 4080

tcggtatgtt catgacaaaa ctggtgaaat aactagatgt tcaatctctg tatgatctga 4140

ctagctcaat ccatgccttg ttcatcttat ctatgcgttg ttaaacggac ttctcattcc 4200

attgtgtttt ttactttcag aaatgcctcg gtactaatga ataatgaacc ccattgagct 4260

acatgacata tgggtggata gtaatatagc aaatgatttt gtcctttgtg tgaatagaga 4320

gaacatagat cataaaatgt ttttaaaatt gaaaccaaca caatataatt ctctaggcat 4380

tatctcctag ttgcagacta gcgaaatgca ttatggcact cctatagaat tttaacttaa 4440

cttttagatt ttagataaat ttgatttgca gcatgctgta tttgaaccct taatgttatc 4500

tcatttatgc attttaacac gtgatattgc agatgaagtt gaaagccttg ctgctgccag 4560

acaggctgcc atatctggct ctgagccttc agactccatt agggtatagt tttcatcagt 4620

tttcctttgc catatacctt ttatcaggta atgtttacac tgtcttcttt cactctgtaa 4680

tgttcaggtg gtaaatgcct tgctaacaca aatggacaaa ctgaaggctt ggccaaatgt 4740

tatcattttg acgacatcaa atatcaccac agctatcggt attgattttc cgccttttga 4800

gtttttttcc ttttatgacc tgaatagccc atcatgccat tttgtgtgca tagttgccat 4860

agtttttgca tgattatctg cgcgtttgga acaacggtgc tgtatatgat caacatctgg 4920

acaaatttgg acatttagta tgtgttttag ttggtcaagt gaatcaacac agtgagggga 4980

agatcaattg cataataagc atgtttatgt ttgtggagct gtctatgttt ataacatcac 5040

ttttcctact tttctgaagt aactaatgct acattattga tacatttgaa tttagataat 5100

cttgttgcca tcgacaatga gatatcaact gtgctcagta ctgatgtctc ttgggctaat 5160

aaaattgtgt aattgcagac attcattcta ttttcgatgg ctgttaagga gaacagcctg 5220

tagctttctt aaacatcatg tagacatgta ttgagtccta aaatcaataa attctaactt 5280

gtatgtggtt tgcagatatt gcatttgttg acagggcaga catcaaggca tacgtcggac 5340

cccctactct tcaagcacgc tatgagattt tgagatcatg catagatgaa ctcttccgag 5400

taggaattct tacatatcct caggtatttg ttcctttttg ccaacttggt tgctgtaatg 5460

ctgccttttt tttaattaaa catcactgtg aaggttgttc gggactgctt tgctcatggg 5520

gcactttctc taagcaaacc tggatttggg acctcaaacg atgcgtttat gaacttctgt 5580

atagaatttc tagcttgcaa tctttaaatg attattcctc tccaaataca gggcggtgat 5640

ctgccttgca ttctgagcta ttcaaccctg aaagagaagt tgcacggtcc tgaggctgca 5700

gaaccccata cacttcatct ctccaactta ctgcatgaag gtgcagaact gtgcgacgta 5760

agtgatgaag tttacagttg ccttaccttt tcattgctgt agattaagcg atctgctgta 5820

acccacacca acatgctgct tatggtggtg atgttttttc cattctccca cagggattga 5880

gcggaagatc attgagaaaa cttcctttcc tggcacacgc ttctgttccc aacccttcgt 5940

gctgtgacgc tgcttctttc atgcacacgc tgattcaaac cgcgcggaga gagctcttgg 6000

aatcacgcgg atgattcctc gcgtattgct tggaggagca aatggctaac ggtcggatac 6060

gagtaccttg ccgctgatat gatgcatagt ttgagctaat tcgaagtctg ttgttctgag 6120

atacaagcaa atccattcca tttctaggtc tagagaagta ggaactccag gatataggtg 6180

ccgttgctgt tgcattactt ttggttctcc tgtaaattgt tacggtagga tgcactacac 6240

ctcgagggac gccgcattat tacctacata catgaagttt ctcttgcggg gatttaccta 6300

catgaatgcc atacttcggt gacttgatca gatctgtttc cggtcattgg aacttgaaat 6360

ggatagcgtt gtgtacctta ttagctttgt ttctgttatt gcggttttgc atgatatgtg 6420

caggatgtaa atcaaagttg gctggactgt ttggcctggc ctcagccctt caggctaact 6480

ctcttcagtc ccgctgaaag agtgccaagg tatatgaccg tgcattcctg tgataggaat 6540

agccaagaga gtttattctt ctgtactggt gccaagggag gttagtgcat tgaaccttca 6600

ttaaccaaaa gtttcgccta tcaattcaag catccagcag aaaaaaagag agatacaagt 6660

taattcaaga ctcagaccat aaactgtgac tctcagcaac ttgatggcgt gcgtaggcta 6720

cagggagtga atgtacatga aggaaataag agccatgaca atgtttatat aaaatttgca 6780

ggcgcacagg ccatcttcag gttagaacct tggatttcac ctgtgctggt ctggttggga 6840

catactacgt tgcaggataa agagctggga atgcttgccc ctgaaccggt gcagcaggat 6900

accccgtgct accaaccgaa acgataacgg tgtcgagttg tagttgaaa 6949

<210> 41
<211> 1878
<212> DNA
<213> Artificial Sequence

<220>
<223> cDNA of pch2 from Triticum aestivum genome D

<400> 41
acagcctcct tccccaaaat cccccattcc tccacggctc cgccttctcc tcccctcccc 60

aaacccaaaa ccctagaacc ttctccattc cgctccctcg cggagcttgg ccaccatgag 120

cgctcccatg gagatctcct tcgaccccca cccttctcat cccgcgctgc ccgccttccc 180

ctccagccca cccctggagc ccgcagccgc cgccacccct cccgcggtct cgtcctcgcc 240

gcagccgacg gcggaggccg tagtcgcgcc caccccgcca gacgacaagg tcctcgtctc 300

agttgaggtg ctgctgcacg ccacgagtac ggcccgcgcc gaggacgtcc gagtagcggt 360

cgagaggatg ttagaatctc gaagtttaag ctatgttgat ggtcctgttc agatacctcc 420

agacaattct tttcttcttg aacatgtcaa aaggatccaa ataagtgaca ctgatgagtt 480

gattgagaac cacaaaatcc tgttgttttg gcaagtcaaa cctgtcgttc atgtattcca 540

gttgaatgag gatggtccag gtgaagaacc aagtgaagat gacacactct ctagcttcaa 600

tgagtgggcg ctacctgcca aagaatttga tggattatgg gaaagcttac tatatgaagt 660

gggtctcaag cagaggttgc tgagatatgc tgctagtgct ttgctattta ctgaaagagg 720

tgtagatcca tgccttgttt catggaacag gattgtactt ttgcatggtc cacctggaac 780

aggaaagact tcattgtgta aagcactagc tcagaagctt tctattcgct tcaattcaag 840

gtactctatg tgccaactaa ttgaagtcaa tgcccattca ttgttcagca agtggttttc 900

tgaaagtggc aaactggtag ccaaactttt ccagaagatt caggagatgg ttgaggaaga 960

aagcaacctg gtatttgtat tgatcgatga agttgaaagc cttgctgctg ccagacaggc 1020

tgccatatct ggctctgagc cttcagactc cattagggtg gtaaatgcct tgctaacaca 1080

aatggacaaa ctgaaggctt ggccaaatgt tatcattttg acgacatcaa atatcaccac 1140

agctatcgat attgcatttg ttgacagggc agacatcaag gcatacgtcg gaccccctac 1200

tcttcaagca cgctatgaga ttttgagatc atgcatagat gaactcttcc gagtaggaat 1260

tcttacatat cctcaggcgg gtgatctgcc ttgcattctg agctattcaa ccctgaaaga 1320

gaagttgcac ggtcctgagg ctgcagaacc ccatacactt catctctcca acttactgca 1380

tgaaggtgca gaactgtgcg acggattgag cggaagatca ttgagaaaac ttcctttcct 1440

ggcacacgct tctgttccca acccttcgtg ctgtgacgct gcttctttca tgcacacgct 1500

gattcaaacc gcgcggagag agctcttgga atcacgcgga tgattcctcg cgtattgctt 1560

ggaggagcaa atggctaacg gtcggatacg agtaccttgc cgctgatatg atgcatagtt 1620

tgagctaatt cgaagtctgt tgttctgaga tacaagcaaa tccattccat ttctaggtct 1680

agagaagtag gaactccagg atataggtgc cgttgctgtt gcattacttt tggttctcct 1740

gtaaattgtt acggtaggat gcactacacc tcgagggacg ccgcattatt acctacatac 1800

atgaagtttc tcttgcgggg atttacctac atgaatgcca tacttcggtg acttgatcag 1860

atctgtttcc ggtcattg 1878

<210> 42
<211> 475
<212> PRT
<213> Triticum aestivum

<400> 42

Met Ser Ala Pro Met Glu Ile Ser Phe Asp Pro His Pro Ser His Pro
1               5                   10                  15

Ala Leu Pro Ala Phe Pro Ser Ser Pro Pro Leu Glu Pro Ala Ala Ala
            20                  25                  30

Ala Thr Pro Pro Ala Val Ser Ser Ser Pro Gln Pro Thr Ala Glu Ala
        35                  40                  45

Val Val Ala Pro Thr Pro Pro Asp Asp Lys Val Leu Val Ser Val Glu
    50                  55                  60

Val Leu Leu His Ala Thr Ser Thr Ala Arg Ala Glu Asp Val Arg Val
65                  70                  75                  80

Ala Val Glu Arg Met Leu Glu Ser Arg Ser Leu Ser Tyr Val Asp Gly
                85                  90                  95

Pro Val Gln Ile Pro Pro Asp Asn Ser Phe Leu Leu Glu His Val Lys
            100                 105                 110

Arg Ile Gln Ile Ser Asp Thr Asp Glu Leu Ile Glu Asn His Lys Ile
        115                 120                 125

Leu Leu Phe Trp Gln Val Lys Pro Val Val His Val Phe Gln Leu Asn
    130                 135                 140

Glu Asp Gly Pro Gly Glu Glu Pro Ser Glu Asp Asp Thr Leu Ser Ser
145                 150                 155                 160

Phe Asn Glu Trp Ala Leu Pro Ala Lys Glu Phe Asp Gly Leu Trp Glu
                165                 170                 175

Ser Leu Leu Tyr Glu Val Gly Leu Lys Gln Arg Leu Leu Arg Tyr Ala
            180                 185                 190

Ala Ser Ala Leu Leu Phe Thr Glu Arg Gly Val Asp Pro Cys Leu Val
        195                 200                 205

Ser Trp Asn Arg Ile Val Leu Leu His Gly Pro Pro Gly Thr Gly Lys
    210                 215                 220

Thr Ser Leu Cys Lys Ala Leu Ala Gln Lys Leu Ser Ile Arg Phe Asn
225                 230                 235                 240

Ser Arg Tyr Ser Met Cys Gln Leu Ile Glu Val Asn Ala His Ser Leu
                245                 250                 255

Phe Ser Lys Trp Phe Ser Glu Ser Gly Lys Leu Val Ala Lys Leu Phe

            260                 265                 270

Gln Lys Ile Gln Glu Met Val Glu Glu Glu Ser Asn Leu Val Phe Val
        275                 280                 285

Leu Ile Asp Glu Val Glu Ser Leu Ala Ala Ala Arg Gln Ala Ala Ile
    290                 295                 300

Ser Gly Ser Glu Pro Ser Asp Ser Ile Arg Val Val Asn Ala Leu Leu
305                 310                 315                 320

Thr Gln Met Asp Lys Leu Lys Ala Trp Pro Asn Val Ile Ile Leu Thr
                325                 330                 335

Thr Ser Asn Ile Thr Thr Ala Ile Asp Ile Ala Phe Val Asp Arg Ala
            340                 345                 350

Asp Ile Lys Ala Tyr Val Gly Pro Pro Thr Leu Gln Ala Arg Tyr Glu
        355                 360                 365

Ile Leu Arg Ser Cys Ile Asp Glu Leu Phe Arg Val Gly Ile Leu Thr
    370                 375                 380

Tyr Pro Gln Gly Gly Asp Leu Pro Cys Ile Leu Ser Tyr Ser Thr Leu
385                 390                 395                 400

Lys Glu Lys Leu His Gly Pro Glu Ala Ala Glu Pro His Thr Leu His
                405                 410                 415

Leu Ser Asn Leu Leu His Glu Gly Ala Glu Leu Cys Asp Gly Leu Ser
            420                 425                 430

Gly Arg Ser Leu Arg Lys Leu Pro Phe Leu Ala His Ala Ser Val Pro
        435                 440                 445

Asn Pro Ser Cys Cys Asp Ala Ala Ser Phe Met His Thr Leu Ile Gln
    450                 455                 460

Thr Ala Arg Arg Glu Leu Leu Glu Ser Arg Gly
465                 470                 475